# EUROPEAN PATENT SPECIFICATION

(11) **EP 4 034 547 B1**
(45) Date of publication and mention of the grant of the patent: **22.05.2024**
(21) Application number: 20781119.1
(22) Date of filing: 24.09.2020
(51) Int. Cl.: C07K 9/00, A61P 31/04, A61K 38/00

(54) **ANTIBIOTIC COMPOUNDS**
ANTIBIOTISCHE VERBINDUNGEN
COMPOSÉS ANTIBIOTIQUES

(30) Priority: 24.09.2019 NL 2023883
(43) Date of publication of application: 03.08.2022
(73) Proprietor: Universiteit Leiden, 2311 EZ Leiden (NL)
(72) Inventor: MARTIN, Nathaniel I., 2311 EZ Leiden (NL); VAN GROESEN, Emma, 2311 EZ Leiden (NL); TEHRANI, Kamaleddin H.M.E., 2311 EZ Leiden (NL); WADE, Nicola, 2311 EZ Leiden (NL)
(74) Representative: HGF
(86) International application number: PCT/NL2020/050587
(87) International publication number: WO 2021/060980

(56) References cited:
- WO-A1-00/39156
- WO-A2-01/81373
- CHUN-MAN HUANG ET AL: "Teicoplanin Reprogrammed with the N-Acyl-Glucosamine Pharmacophore at the Penultimate Residue of Aglycone Acquires Broad-Spectrum Antimicrobial Activities Effectively Killing Gram-Positive and -Negative Pathogens", ACS INFECTIOUS DISEASES, vol. 5, no. 3, 1 January 2019 (2019-01-01) , pages 430-442, XP055695938, US ISSN: 2373-8227, DOI: 10.1021/acsinfecdis.8b00317
- DONGLIANG GUAN ET AL: "Extra Sugar on Vancomycin: New Analogues for Combating Multidrug-Resistant Staphylococcus aureus and Vancomycin-Resistant Enterococci", JOURNAL OF MEDICINAL CHEMISTRY, vol. 61, no. 1, 2 January 2018 (2018-01-02), pages 286-304, XP055564634, US ISSN: 0022-2623, DOI: 10.1021/acs.jmedchem.7b01345

## Description

This invention relates to a novel class of lipidated guanidino derivatives of glycopeptide antibiotics. This invention also provides formulations and compositions comprising such compounds. The compounds, formulations and compositions may be used as a medicament, such as in the treatment of bacterial infection.

### BACKGROUND

In pursuing antibiotics with activity against Gram-positive bacteria, the bacterial cell wall, often referred to as the peptidoglycan layer, presents an attractive target. Critical to peptidoglycan biosynthesis is lipid II, the penultimate bacterial cell wall building block. A number of natural product antibiotics operate by specifically binding to lipid II (Grein, F., et al. (2019) Docking on Lipid II-A Widespread Mechanism for Potent Bactericidal Activities of Antibiotic Peptides, J Mol Biol.; Oppedijk, S. F., et al. (2016) Hit 'em where it hurts: The growing and structurally diverse family of peptides that target lipid-II, Biochim Biophys Acta 1858, 947-957). Prominent among these is the clinically used glycopeptide vancomycin which binds tightly to the terminal d-Ala-d-Ala motif of the lipid II pentapeptide.

Increasingly, resistance to vancomycin is encountered as many bacterial strains have emerged that can employ a lipid II variant containing a d-Ala-d-Lac terminated pentapeptide. This d-Lac for d-Ala mutation lowers the affinity of vancomycin for lipid II and in doing so greatly reduces it antibacterial effect (Healy, V. L., et al. (2000) Vancomycin resistance in enterococci: reprogramming of the D-ala-D-Ala ligases in bacterial peptidoglycan biosynthesis, Chem Biol 7, R109-119; Blaskovich, M. A. T., et al. (2018) Developments in Glycopeptide Antibiotics, ACS Infect Dis 4, 715-735; Willyard, C. (2017) The drug-resistant bacteria that pose the greatest health threats, Nature 543, 15). The development of new glycopeptide antibiotics with enhanced antibacterial activity thus continues to be of great importance. WO 00/39156 describes a wide of possible substitutions to the glycosidic amine of Vancomycin. WO 01/81373 discloses that the glycosidic amine in Vancomycin derivatives may be replaced by guanidine. CHUN-MAN HUANG ET AL: "Teicoplanin Reprogrammed with the N-Acyl-Glucosamine Pharmacophore at the Penultimate Residue of Aglycone Acquires Broad-Spectrum Antimicrobial Activities Effectively Killing Gram-Positive and -Negative Pathogens", ACS INFECTIOUS DISEASES, vol. 5, no. 3, 1 January 2019, pages 430-442, describe a Teicoplanin derivative in which the free amine group is converted to a guanidine group. DONGLIANG GUAN ET AL: "Extra Sugar on Vancomycin: New Analogues for Combating Multidrug-Resistant Staphylococcus aureus and Vancomycin-Resistant Enterococci", JOURNAL OF MEDICINAL CHEMISTRY, vol. 61, no. 1, 2 January 2018, pages 286-304, describe Vancomycin derivatives with various modifications to the glycosidic amine.

An object of the invention is to provide compounds that are useful in the treatment of bacterial infections, in particular for the treatment of infections that are resistant to existing antibiotics, such as vancomycin.

### BRIEF SUMMARY OF THE DISCLOSURE

The invention provides compounds that are useful in the treatment of bacterial infection. For example, the compounds may be useful in the treatment of an infection by Gram-positive bacteria. Compounds of the invention are lipidated glycopeptides, where the lipid moiety is attached to the glycan moiety via a linker and a guanidino moiety.

The invention provides in a first aspect a compound of formula I: 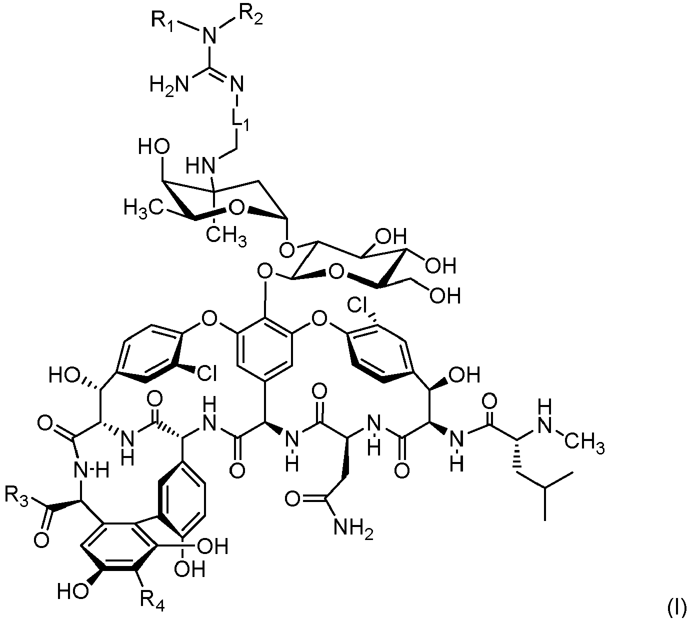 wherein:
R₁ is a lipid;
R₂ is selected from -H or a lipid;
R_{3,} R₄ and L are defined in the appended claim 1,
or a pharmaceutically acceptable salt, stereoisomer, or solvate thereof.

A second aspect of the invention provides a formulation of the invention comprising compound of the invention and optionally a pharmaceutically acceptable carrier. The formulation may be a parenteral formulation or an oral formulation. The formulation may be a parenteral formulation, such as a formulation for intravenous injection.

A third aspect provides a compound or formulation of the invention for use as a medicament.

A fourth aspect provides a compound or formulation of the invention for use in the treatment of a bacterial infection. The bacterial infection may be an infection by Gram-positive bacteria. The Gram-positive bacteria may be from at least one of the following families *Staphylococcus* (e.g. *S*. *aureus, S. epidermidis, S. saprophyticus*)*, Streptococcus* (e.g. *Strep. pyogenes, Strep. agalactiae, Strep. viridans, Strep. pneumonia*)*, Enterococus* (e.g. *E. faecalis*)*, Bacillus, Clostridia, Listeria* and *Corynebacterium.* The bacteria (e.g. Gram-positive bacteria) may be resistant to treatment with at least one other antibiotic (e.g. methicillin, vancomycin). The bacterial infection may be an infection by vancomycin-resistant *Enterococcus* (VRE), methicillin-resistant *S*. *aureus* (MRSA), or vancomycin-resistant *S*. *aureus* (VRSA). The bacterial infection may be selected from skin and structure infections, lower respiratory tract infections, bacteremia, sepsis, septicemia, infective endocarditis, peritonitis, enterocolitis mastitis, *Clostridium difficile* infection-associated diarrhoea and colitis.

### BRIEF DESCRIPTION OF THE DRAWINGS

Embodiments of the invention are further described hereinafter with reference to the accompanying drawings, in which:
Figure 1 illustrates the results obtained when selected compounds were assessed using a hemolysis assay.
Figure 2 illustrates the results obtained with a UDP-MurNAc-pentapeptide accumulation assay for selected compounds. All of the compounds resulted in accumulation of UDP-MurNAc-pentapeptide, demonstrating that the compounds interfere with peptidoglycan cell wall biosynthesis in Gram-positive bacteria.
Figure 3 illustrates the results obtained when selected compounds were assessed using a resistance development serial passage assay. A) Daily fold-increase of MIC compared to initial MIC against MRSA USA 300 grown in sub-lethal concentrations of daptomycin, compound **5** or compound **16** over 30 days. B) . Daily fold-increase of MIC compared to initial MIC against VRE E155 grown in sub-lethal concentrations of daptomycin, compound **5** or compound **16** over 30 days.
Figure 4 illustrates the results obtained when selected compounds were assessed using a time kill assay. A) Bactericidal activity of vancomycin, telavancin, daptomycin (left), compound **5** and compound **16** (right) against VRE E155 as measured by agar plate dilution colony count of samples at different time intervals. B) Bactericidal activity of vancomycin, telavancin, daptomycin (left) and compound 5 (right) against MRSA USA300 as measured by agar plate dilution colony count of samples at different time intervals.
Figure 5 illustrates the pharmacokinetic profile of blood concentrations of a selected compound over a time period of 8 hours.
Figure 6 illustrates the results obtained in an efficacy study for selected compounds against MRSA USA300 strain NRS384.

### DETAILED DESCRIPTION

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

### DEFINITIONS

The following explanations of terms and methods are provided to better describe the present disclosure and to guide those of ordinary skill in the art in the practice of the present disclosure. The references to methods of treatment in the subsequent paragraphs of this description are to be interpreted as references to the compounds, pharmaceutical compositions and medicaments of the present invention for use in a method for treatment of the human (or animal) body by therapy.

The invention concerns amongst other things the treatment of a disease. The term "treatment", and the therapies encompassed by this invention, include the following and combinations thereof: (1) hindering, e.g. delaying initiation and/or progression of, an event, state, disorder or condition, for example arresting, reducing or delaying the development of the event, state, disorder or condition, or a relapse thereof in case of maintenance treatment or secondary prophylaxis, or of at least one clinical or subclinical symptom thereof; (2) preventing or delaying the appearance of clinical symptoms of an event, state, disorder or condition developing in an animal (e.g. human) that may be afflicted with or predisposed to the state, disorder or condition but does not yet experience or display clinical or subclinical symptoms of the state, disorder or condition; and/or (3) relieving and/or curing an event, state, disorder or condition (*e*.*g*., causing regression of the event, state, disorder or condition or at least one of its clinical or subclinical symptoms, curing a patient or putting a patient into remission). The benefit to a patient to be treated may be either statistically significant or at least perceptible to the patient or to the physician. It will be understood that a medicament will not necessarily produce a clinical effect in each patient to whom it is administered; thus, in any individual patient or even in a particular patient population, a treatment may fail or be successful only in part, and the meanings of the terms "treatment" and "prophylaxis" and of cognate terms are to be understood accordingly. The compositions and methods described herein are of use for therapy and/or prophylaxis of the mentioned conditions.

The term "prophylaxis" includes reference to treatment therapies for the purpose of preserving health or inhibiting or delaying the initiation and/or progression of an event, state, disorder or condition, for example for the purpose of reducing the chance of an event, state, disorder or condition occurring. The outcome of the prophylaxis may be, for example, preservation of health or delaying the initiation and/or progression of an event, state, disorder or condition. It will be recalled that, in any individual patient or even in a particular patient population, a treatment may fail, and this paragraph is to be understood accordingly.

The term "antibiotic" refers to a compound that inhibits the growth of or destroys microorganisms, such as bacteria (e.g. Gram-positive bacteria, or Gram-negative bacteria). An "antibacterial" is an antibiotic that is active against bacteria. Compounds of the invention are antibacterial, in particular with activity against Gram-positive bacteria. Gram-positive bacteria include *Staphylococcus* (e.g. *S*. *aureus, S. epidermidis, S. saprophyticus*)*, Streptococcus* (e.g. *Strep. pyogenes, Strep. agalactiae, Strep. viridans, Strep. pneumonia*)*, Enterococus, Bacillus, Clostridia, Listeria* and *Corynebacterium.*

The term "alkyl" as used herein includes reference to a straight or branched chain alkyl moiety having up to 20 (e.g. 1, 2, 3, 4, 5, 6, 7, 8, 9, 10, 11, 12, 13, 14, 15, 16, 17, 18, 19 or 20) carbon atoms. The term includes reference to, for example, methyl, ethyl, propyl (n-propyl or isopropyl), butyl (n-butyl, sec-butyl or tert-butyl), pentyl, hexyl and the like. In particular, alkyl may be a "C₁-C₁₀ alkyl", i.e. an alkyl having 1, 2, 3, 4, 5, 6, 7, 8, 9 or 10 carbon atoms; "C₁-C₆ alkyl", i.e. an alkyl having 1, 2, 3, 4, 5 or 6 carbon atoms; "C₁-C₄ alkyl", i.e. an alkyl having 1, 2, 3 or 4 carbon atoms; a "C₁-C₆ alkyl", i.e. an alkyl having 1, 2, 3, 4, 5 or 6 carbon atoms; or a "C₁-C₃ alkyl", i.e. an alkyl having 1, 2 or 3 carbon atoms. The term "lower alkyl" includes reference to alkyl groups having 1, 2, 3 or 4 carbon atoms.

The term "alkenyl" as used herein includes reference to a straight or branched chain alkenyl moiety having up to 20 (e.g. 2, 3, 4, 5, 6, 7, 8, 9, 10, 11, 12, 13, 14, 15, 16, 17, 18, 19 or 20) carbon atoms. The term includes reference to, for example, ethenyl, propenyl, butenyl, pentenyl, hexenyl and the like. In particular, alkenyl may be a "C₂-C₁₀ alkenyl", i.e. an alkenyl having 2, 3, 4, 5, 6, 7, 8, 9 or 10 carbon atoms; "C₂-C₆ alkyl", i.e. an alkenyl having 2, 3, 4, 5 or 6 carbon atoms; "C₂-C₄ alkyl", i.e. an alkenyl having 1, 2, 3 or 4 carbon atoms; The term "lower alkenyl" includes reference to alkyl groups having 2, 3 or 4 carbon atoms. The alkenyl may be monounsaturated (i.e. comprise a single carbon carbon double bond) or polyunsaturated (i.e. comprise a two or more carbon carbon double bonds, e.g. 2, 3 or 4 carbon carbon double bonds). For example, an alkenyl may be an alkadienyl, alkatrienyl, etc..

The term "alkylene" by itself or as part of another substituent means a divalent radical derived from an alkyl, as exemplified, but not limited, by -CH2CH2CH2CH2-. Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.

The term "cycloalkyl" as used herein includes reference to an alicyclic moiety having 3, 4, 5 or 6 carbon atoms. The group may be a bridged or polycyclic ring system. More often cycloalkyl groups are monocyclic. This term includes reference to groups such as cyclopropyl, cyclobutyl, cyclopentyl, cyclohexyl and the like.

The term "heteroalkyl," by itself or in combination with another term, means, unless otherwise stated, a stable straight or branched chain, or cyclic hydrocarbon radical, or combinations thereof, consisting of at least one carbon atoms and at least one heteroatom selected from the group consisting of O, N, P, Si and S, and wherein the nitrogen and sulfur atoms may optionally be oxidized and the nitrogen heteroatom may optionally be quaternized. The heteroatom(s) O, N, P, S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, - CH=CH-O-CH₃, -Si(CH₃)₃, -CH₂-CH=N-OCH₃, -CH=CH-N(CH₃)-CH₃, O-CH₃, -O-CH₂-CH₃, and -CN. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (*e*.*g*., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula - C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR', and/or-SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The term "heterocycloalkyl" as used herein includes reference to a saturated heterocyclic moiety having 3, 4, 5, 6 or 7 ring carbon atoms and 1, 2, 3, 4 or 5 ring heteroatoms selected from nitrogen, oxygen, phosphorus and sulphur. For example, a heterocycloalkyl may comprise 3, 4, or 5 ring carbon atoms and 1 or 2 ring heteroatoms selected from nitrogen and oxygen. The group may be a polycyclic ring system but more often is monocyclic. This term includes reference to groups such as azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolizidinyl and the like.

The terms "halo" or "halogen" as used herein includes reference to F, C!, Br or I, for example F, Cl or Br. In a particular class of embodiments, halogen is F or CI, of which F is more common.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "haloalkyl" refers to an alkyl group where one or more hydrogen atoms are substituted by a corresponding number of halogens. For example, the term "halo(C₁-C₄)alkyl" is mean to include, but not be limited to, trifluoromethyl, 2,2,2-trifluoroethyl, 4-chlorobutyl, 3-bromopropyl, and the like.

The term "alkoxy" as used herein include reference to -O-alkyl, wherein alkyl is straight or branched chain and comprises 1, 2, 3, 4, 5 or 6 carbon atoms. In one class of embodiments, alkoxy has 1, 2, 3 or 4 carbon atoms, e.g. 1, 2 or 3 carbon atoms. This term includes reference to, for example, methoxy, ethoxy, propoxy, isopropoxy, butoxy, tert-butoxy, pentoxy, hexoxy and the like. The term "lower alkoxy" includes reference to alkoxy groups having 1, 2, 3 or 4 carbon atoms.

The term "haloalkoxy" as used herein refers to an alkoxy group where one or more hydrogen atoms are substituted by a corresponding number of halogens.

The term "aryl" means, unless otherwise stated, a polyunsaturated, aromatic, hydrocarbon substituent which can be a single ring or multiple rings (preferably from 1 to 3 rings) which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. "Arylene" and "heteroarylene" refers to a divalent radical derived from an aryl and heteroaryl, respectively.

The term "lipid" with reference to a substituent as used herein represents a moiety that is typically hydrophobic. A lipid may comprise substituted or unsubstituted alkyl, alkenyl, cycloalkyl, bridged cycloalkyl, (alkyl)cycloalkyl, (alkyl) bridged cycloalkyl, (alkyl)cycloalkenyl, and/or alkylaryl groups. For example, a lipid may comprise substituted or unsubstituted alkyl, alkenyl, (alkyl)cycloalkyl, (alkyl)cycloalkenyl, and/or alkylaryl groups. Exemplary substituents include -OH, =O, -CN, -halo, -NH₂, -NH(C₁-C₆ alkyl), -N(C₁-C₄ alkyl)₂, -phenyl, -phenyl-halo; for example -OH, =O, -CN, -halo, -NH₂, -NH(C₁-C₆ alkyl), - N(C₁-C₄ alkyl)₂. The backbone of the substituted or unsubstituted lipid may also be interrupted by a disulfide linkage (-S-S-), thioether linkage (-S-), ether linkage -O- or ester (-C(O)O-).

Each of the above terms (*e*.*g*., "alkyl," "cycloalkyl," "heteroalkyl," "aryl" and "heteroaryl"), unless otherwise noted, are meant to include both substituted and unsubstituted forms of the indicated radical. Where a substituent is R-substituted (e.g. an R^{x}-substituted alkyl, where "x" is an integer), the substituent may be substituted with one or more R groups as allowed by chemical valency rules where each R group is optionally different (e.g. an R^{x}-substituted alkyl may include multiple R^{x} groups wherein each R^{x} group is optionally different). Certain examples of substituents for each type of radical are provided below.

The term "substituted" as used herein in reference to a moiety means that one or more, especially up to 5, more especially 1, 2 or 3, of the hydrogen atoms in said moiety are replaced independently of each other by the corresponding number of the described substituents. Unless otherwise specified, exemplary substituents include -OH, -CN, -NH₂, - NH(C₁-C₆ alkyl), -N(C₁-C₄ alkyl)₂, =O, -halo, -C₁-C₆ alkyl, -C₂-C₆ alkenyl, -C₁-C₆ haloalkyl, - C₁-C₆ haloalkoxy and-C₂-C₆ haloalkenyl, -C₁-C₆ alkylcarboxylic acid (e.g. -CH₃COOH or - COOH). Where the substituent is a -C₁-C₆ alkyl or -C₁-C₆ haloalkyl, the C₁-C₆ chain is optionally interrupted by an ether linkage (-O-) or an ester linkage (-C(O)O-). Exemplary substituents for a substituted alkyl may include -OH, -CN, -NH₂, =O, -halo, -CO₂H, -C₁-C₆ haloalkyl, -C₁-C₆ haloalkoxy and-C₂-C₆haloalkenyl, -C₁-C₆ alkylcarboxylic acid (e.g. - CH₃COOH or -COOH). For example, exemplary substituents for an alkyl may include -OH, -CN, -NH₂, =O, -halo.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

Where steric issues determine placement of substituents on a group, the isomer having the lowest conformational energy may be preferred.

Where a compound, moiety, process or product is described as "optionally" having a feature, the disclosure includes such a compound, moiety, process or product having that feature and also such a compound, moiety, process or product not having that feature. Thus, when a moiety is described as "optionally substituted", the disclosure comprises the unsubstituted moiety and the substituted moiety.

Where two or more moieties are described as being "independently" or "each independently" selected from a list of atoms or groups, this means that the moieties may be the same or different. The identity of each moiety is therefore independent of the identities of the one or more other moieties.

The term "pharmaceutically acceptable" as used herein includes reference to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio. This term includes acceptability for both human and veterinary purposes.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like (*see*, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

Certain compounds of the present invention possess asymmetric carbon atoms (optical centres) or double bonds; the racemates, diastereomers, tautomers, geometric isomers and individual isomers are encompassed within the scope of the present invention. The compounds of the present invention do not include those which are known in the art to be too unstable to synthesize and/or isolate.

The symbol 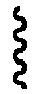 denotes a point of attachment of a moiety to the remainder of a compound.

The term "prodrug" as used herein represents compounds which are transformed *in vivo* to the parent compound or other active compound, for example, by hydrolysis in blood. An example of such a prodrug is a pharmaceutically acceptable ester of a carboxylic acid. A thorough discussion is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987; H Bundgaard, ed, Design of Prodrugs, Elsevier, 1985; and Judkins, et al. Synthetic Communications, 26(23), 4351-4367 (1996); and The organic chemistry of drug design and drug action by Richard B Silverman in particular pages 497 to 546. Compounds may represent prodrugs (e.g. comprising a latent MBL inhibitor), where hydrolysis of a β-lactam results in release and activation of the latent MBL inhibitor.

The term "pharmaceutical formulation" as used herein includes reference to a formulation comprising at least one active compound and optionally one or more additional pharmaceutically acceptable ingredients, for example a pharmaceutically acceptable carrier. Where a pharmaceutical formulation comprises two or more active compounds, or comprises at least one active compound and one or more additional pharmaceutically acceptable ingredients, the pharmaceutical formulation is also a pharmaceutical composition. Unless the context indicates otherwise, all references to a "formulation" herein are references to a pharmaceutical formulation.

The term "product" or "product of the invention" as used herein includes reference to any product containing a compound of the present invention. In particular, the term product relates to compositions and formulations containing a compound of the present invention, such as a pharmaceutical composition, for example.

The term "therapeutically effective amount" as used herein refers to an amount of a drug, or pharmaceutical agent that, within the scope of sound pharmacological judgment, is calculated to (or will) provide a desired therapeutic response in a mammal (animal or human). The therapeutic response may for example serve to cure, delay the progression of or prevent a disease, disorder or condition.

The following abbreviations are used herein:
- Alloc: allyloxycarbonyl
- ATCC: American Type Culture Collection
- BBO: Broadband Observe
- CFU: colony forming units
- CLSI: Clinical & Laboratory Standards Institute
- d: doublet
- dd: doublet of doublets
- dt: doublet of triplets
- DCM: dichloromethane
- DIPEA: di-isopropylethylamine
- DMF: dimethylformamide
- DMSO: dimethylsulfoxide
- EDC: 1-ethyl-3-(3-dimethylaminopropyl)carbodiimide
- EtOAc: ethyl acetate
- EtOH: ethanol
- eq: equivalent
- FBS: fetal bovine serum
- Gal: galactose
- GaINAc: N-acetylgalactosamine
- Glc: glucose
- GIcNAc: N-acetylglucosamine
- h: sextet
- HEPA: high-efficiency particulate air
- HPLC: high-performance liquid chromatography
- HR-MS: high-resolution mass spectrometry
- LC: liquid chromatography
- m: multiplet
- Man: mannose
- ManNAc: N-acetylmannoseamine
- MeCN: acetonitrile
- MeOH: methanol
- MIC: minimum inhibitory concentration
- MRSA: methicillin resistant *Staphylococcus aureus*
- MSSA: methicillin sensitive *Staphylococcus aureus*
- MTT: 3-(4,5-dimethylthiazol-2-yl)-2,5-diphenyltetrazolium bromide
- MurNAc: N-Acetylmuramic acid
- m/z: mass-to-charge ratio
- NEt₃: triethylamine
- NLD: The Netherlands
- NMR: nuclear magnetic resonance
- p80: polysorbate 80
- PBS: phosphate buffered saline
- PE: petroleum ether
- PFGE: pulsed field gel electrophoresis
- PK: pharmacokinetic
- pp: pentapeptide
- ppm: parts per million
- rpm: rounds per minute
- RT: room temperature
- RP-HPLC: reverse phase high-performance liquid chromatography
- s: singlet
- SD: standard deviation
- t: triplet
- THF: tetrahydrofuran
- TLC: thin layer chromatography
- TMS: trimethylsilyl
- TSB: tryptic soy broth
- UDP: uridine diphosphate
- UV: ultraviolet
- VISA: vancomycin intermediate *Staphylococcus aureus*
- VRE: vancomycin resistant *Enterococci*
- VRSA: vancomycin resistant *Staphylococcus aureus*
- VSE: vancomycin sensitive *Enterococci*

### COMPOUNDS

In one aspect, the invention provides compounds of formula I as previously described or a pharmaceutically acceptable salt, stereoisomer or solvate thereof. The compounds of formula I represent lipidated glycopeptides, where the lipid moiety/moieties (R₁ and optionally R₂) is attached to the glycan moiety via a linker (-L₁-) and a guanidino moiety. The glycopeptide portion of the compound represents a substituted vancomycin, with optional additional substitutions R₃ and R₄.

R₁ is a lipid. R₁ may be selected from substituted or unsubstituted -C₄-C₂₀ cycloalkyl (e.g. bridged cycloalkyl), substituted or unsubstituted -C₁-C₄ alkyl-C₄-C₂₀ cycloalkyl (e.g. bridged cycloalkyl), substituted or unsubstituted -C₄-C₂₀ alkyl, substituted or unsubstituted -C₄-C₂₀ alkenyl, substituted or unsubstituted -C₁-C₄ alkylaryl, -C₁-C₄ alkyl-[O(CH₂)ₘ]ₙ-O(CH₂)ₘ₋₁CH₃, where m is selected from 2 and 3 and n is selected from 0 to 20, - C₂-C₁₂ alkyl-S-S-C₁-C₁₂ alkyl. R₁ may be selected from substituted or unsubstituted -C₄-C₂₀ alkyl, substituted or unsubstituted -C₄-C₂₀ alkenyl, substituted or unsubstituted -C₁-C₄ alkylaryl, -C₁-C₄ alkyl-[O(CH₂)ₘ]ₙ-O(CH₂)ₘ₋₁CH₃, where m is selected from 2 and 3 and n is selected from 0 to 20, -C₂-C₁₂ alkyl-S-S-C₁-C₁₂ alkyl. R₁ may be selected from substituted or unsubstituted -C₄-C₁₆ cycloalkyl (e.g. bridged cycloalkyl), substituted or unsubstituted -C₁-C₄ alkyl-C₄-C₁₆ cycloalkyl (e.g. bridged cycloalkyl), substituted or unsubstituted -C₄-C₁₆ alkyl, substituted or unsubstituted -C₄-C₁₆ alkenyl, substituted or unsubstituted -C₁-C₄ alkylaryl, - C₁-C₄ alkyl-[O(CH₂)ₘ]ₙ-O(CH₂)ₘ₋₁CH₃, where m is selected from 2 and 3 and n is selected from 0 to 14, -C₂-C₈ alkyl-S-S-C₂-C₈ alkyl. R₁ may be selected from substituted or unsubstituted -C₄-C₁₆ alkyl, substituted or unsubstituted -C₄-C₁₆ alkenyl, substituted or unsubstituted -C₁-C₄ alkylaryl, -C₁-C₄ alkyl-[O(CH₂)ₘ]ₙ-O(CH₂)ₘ₋₁CH₃, where m is selected from 2 and 3 and n is selected from 0 to 14, -C₂-C₈ alkyl-S-S-C₂-C₈ alkyl. R₁ may be selected from -C₄-C₁₆ cycloalkyl (e.g. bridged cycloalkyl), -C₁-C₄ alkyl-C₄-C₁₆ cycloalkyl (e.g. bridged cycloalkyl), -C₄-C₁₆ alkyl, -C₆-C₁₈ alkenyl and substituted (e.g. halo substituted) or unsubstituted -C₁-C₄ alkylbisphenyl. R₁ may be selected from -C₄-C₁₆ alkyl, -C₆-C₁₈ alkenyl and substituted (e.g. halo substituted) or unsubstituted -C₁-C₄ alkylbisphenyl. R₁ may be selected from -C₆-C₁₄ cycloalkyl (e.g. bridged cycloalkyl), -C₁-C₄ alkyl-C₆-C₁₄ cycloalkyl (e.g. bridged cycloalkyl), -C₆-C₁₄ alkyl, -C₆-C₁₆ alkenyl and substituted or unsubstituted -C₁-C₄ alkylbisphenyl. R₁ may be selected from -C₆-C₁₄ alkyl, -C₆-C₁₆ alkenyl and substituted or unsubstituted -C₁-C₄ alkylbisphenyl. R₁ may be selected from -C₆-C₁₄ cycloalkyl (e.g. bridged cycloalkyl), -CH₂-C₆-C₁₄ cycloalkyl (e.g. bridged cycloalkyl), -C₆-C₁₄ alkyl, -geranyl, - farnesyl and -chlorobisphenyl. R₁ may be selected from -C₆-C₁₄ alkyl, -C₁-C₄ adamantyl, - adamantyl, -geranyl, -farnesyl and -chlorobisphenyl. R₁ may be selected from -C₆-C₁₄ alkyl, -geranyl, -farnesyl and -chlorobisphenyl.

R₁ may be substituted or unsubstituted -C₄-C₂₀ alkyl, for example substituted or unsubstituted -C₄-C₁₆ alkyl. R₁ may be unsubstituted -C₄-C₂₀ alkyl, for example unsubstituted -C₄-C₁₆ alkyl. R₁ may be substituted or unsubstituted -C₄-C₂₀ alkenyl, for example substituted or unsubstituted -C₆-C₁₈ alkenyl. R₁ may be unsubstituted -C₄-C₂₀ alkenyl, for example unsubstituted -C₆-C₁₈ alkenyl. R₁ may be substituted or unsubstituted - C₁-C₄ alkylaryl. R₁ may be unsubstituted -C₁-C₄ alkylaryl, wherein the aryl substituent may be a biaryl. R₁ may be substituted -C₁-C₄ alkylaryl, wherein the aryl substituent may be a halo-subsituted biaryl, e.g. chlorobisphenyl. R₁ may be substituted CH₂-alkylarl, wherein the aryl substituent may be a halo-subsituted biaryl, e.g. chlorobisphenyl. R₁ may be -C₁-C₄ alkyl-[O(CH₂)ₘ]ₙ-O(CH₂)ₘ₋₁CH₃, where m is selected from 2 and 3 and n is selected from 0 to 20. m may be 2. m may be 3. n may be selected from 2 to 16, e.g. n may be selected from 4 to 12. m may be 2 and n may be selected from 2 to 16. m may be 3 and n may be selected from 2 to 16. R₁ may be -C₂-C₁₂ alkyl-S-S-C₁-C₁₂ alkyl, for example -C₂-C₁₀ alkylS-S-C₂-C₁₀ alkyl; e.g. -C₂-C₈ alkyl-S-S-C₂-C₈ alkyl. R₁ may be substituted or unsubstituted - C₄-C₂₀ cycloalkyl, for example substituted or unsubstituted -C₄-C₁₆ cycloalkyl or substituted or unsubstituted -C₆-C₁₄ cycloalkyl (e.g. bridged cycloalkyl). R₁ may be unsubstituted -C₄-C₂₀ cycloalkyl, for example unsubstituted -C₄-C₁₆ alkyl or unsubstituted -C₆-C₁₄ cycloalkyl. R₁ may be an unsubstituted bridged -C₆-C₁₄ cycloalkyl. R₁ may be an unsubstituted bridged C₁₀ cycloalkyl, e.g. adamantyl. R₁ may be substituted or unsubstituted -C₁-C₄ alkyl-C₄-C₂₀ cycloalkyl, for example substituted or unsubstituted -C₁-C₄ alkyl-C₄-C₁₆ cycloalkyl or substituted or unsubstituted -C₁-C₄ alkyl-C₆-C₁₄ cycloalkyl. R₁ may be unsubstituted -C₁-C₄ alkyl-C₄-C₂₀ cycloalkyl, for example unsubstituted -C₁-C₄ alkyl-C₄-C₁₆ cycloalkyl or unsubstituted -C₁-C₄ alkyl-C₆-C₁₄ cycloalkyl. R₁ may be unsubstituted -C₁-C₄ alkyl-C₆-C₁₄ cycloalkyl, wherein the cycloalkyl is bridged. R₁ may an unsubstituted -C₁-C₄ alkyl-C₁₀ cycloalkyl, wherein the cycloalkyl is bridged, e.g. -C₁-C₄ alkyl-adamantyl. R₁ may be unsubsituted -CH₂ -C₁₀ cycloalkyl, wherein the cycloalkyl is bridged, e.g. -CH₂-adamantyl.

When R₁ is a substituted moiety, it may be substituted by at least one -OH, =O, - CN, -halo, -NH₂, -NH(C₁-C₆ alkyl), -N(C₁-C₄ alkyl)₂; for example it may be substituted by at least one -OH, =O, -CN, -halo. When R₁ is a substituted or unsubstituted alkyl, the carbon backbone of the alkyl may be interrupted by at least one disulfide linkage (-S-S-), thioether linkage (-S-), ether linkage -O- or ester linkage (-C(O)O-).

R₂ is selected from -H or a lipid. R₂ may be -H. R₂ may be a lipid. R₂ may be selected from -H, substituted or unsubstituted -C₄-C₂₀ alkyl, substituted or unsubstituted - C₄-C₂₀ alkenyl, substituted or unsubstituted -C₁-C₄ alkylaryl, -C₁-C₄ alkyl-[O(CH₂)ₚ]_{q}-O(CH₂)ₚ₋₁CH₃, where p is selected from 2 and 3 and q is selected from 0 to 20, -C₂-C₁₂ alkyl-S-S-C₁-C₁₂ alkyl. R₂ may be selected from -H, substituted or unsubstituted -C₄-C₁₆ alkyl, substituted or unsubstituted -C₄-C₁₆ alkenyl, substituted or unsubstituted -C₁-C₄ alkylaryl, -C₁-C₄ alkyl-[O(CH₂)ₚ]_{q}-O(CH₂)ₚ₋₁CH₃, where p is selected from 2 and 3 and q is selected from 0 to 14, - C₂-C₈ alkyl-S-S-C₂-C₈ alkyl. R₂ may be selected from -H, -C₄-C₁₂ alkyl, -C₄-C₁₂ alkenyl, substituted or unsubstituted -C₁-C₄ alkylaryl, and -C₁-C₄ alkyl-[O(CH₂)ₚ]_{q}-O(CH₂)ₚ₋₁CH₃, where p is selected from 2 and 3 and q is selected from 0 to 20. R₂ may be selected from - H, -C₄-C₁₂ alkyl, and -C₄-C₁₂ alkenyl. R₂ may be selected from -H, -C₄-C₁₆ alkyl, -C₆-C₁₈ alkenyl and substituted (e.g. halo substituted) or unsubstituted -C₁-C₄ alkylbisphenyl. R₂ may be selected from -H, -C₆-C₁₄ alkyl, -C₆-C₁₆ alkenyl and substituted or unsubstituted -C₁-C₄ alkylbisphenyl. R₂ may be selected from -C₆-C₁₄ alkyl, -geranyl, -farnesyl and - chlorobisphenyl.

Where R₂ is a lipid, R₂ may be selected from substituted or unsubstituted -C₄-C₂₀ cycloalkyl (e.g. bridged cycloalkyl), substituted or unsubstituted -C₁-C₄ alkyl-C₄-C₂₀ cycloalkyl (e.g. bridged cycloalkyl), substituted or unsubstituted -C₄-C₂₀ alkyl, substituted or unsubstituted -C₄-C₂₀ alkenyl, substituted or unsubstituted -C₁-C₄ alkylaryl, -C₁-C₄ alkyl-[O(CH₂)ₚ]_{q}-O(CH₂)ₚ₋₁CH₃, where p is selected from 2 and 3 and q is selected from 0 to 20, - C₂-C₁₂ alkyl-S-S-C₁-C₁₂ alkyl. R₂ may be selected from substituted or unsubstituted -C₄-C₂₀ alkyl, substituted or unsubstituted -C₄-C₂₀ alkenyl, substituted or unsubstituted -C₁-C₄ alkyl-[O(CH₂)ₚ]_{q}-O(CH₂)ₚ₋₁CH₃, where p is selected from 2 and 3 and q is selected from 0 to 20, - C₂-C₁₂ alkyl-S-S-C₁-C₁₂ alkyl. R₂ may be selected from substituted or unsubstituted -C₄-C₁₆ cycloalkyl (e.g. bridged cycloalkyl), substituted or unsubstituted -C₁-C₄ alkyl-C₄-C₁₆ cycloalkyl (e.g. bridged cycloalkyl), substituted or unsubstituted -C₄-C₁₆ alkyl, substituted or unsubstituted -C₄-C₁₆ alkenyl, substituted or unsubstituted -C₁-C₄ alkylaryl-C₁-C₄ alkyl-[O(CH₂)ₚ]_{q}-O(CH₂)ₚ₋₁CH₃, where p is selected from 2 and 3 and q is selected from 0 to 14, - C₂-C₈ alkyl-S-S-C₂-C₈ alkyl. R₂ may be selected from substituted or unsubstituted -C₄-C₁₆ alkyl, substituted or unsubstituted -C₄-C₁₆ alkenyl, substituted or unsubstituted -C₁-C₄ alkyl-[O(CH₂)ₚ]_{q}-O(CH₂)ₚ₋₁CH₃, where p is selected from 2 and 3 and q is selected from 0 to 14, - C₂-C₈ alkyl-S-S-C₂-C₈ alkyl. R₂ may be selected from -C₄-C₁₆ cycloalkyl (e.g. bridged cycloalkyl), -C₁-C₄ alkyl-C₄-C₁₆ cycloalkyl (e.g. bridged cycloalkyl), -C₄-C₁₆ alkyl, -C₆-C₁₈ alkenyl and substituted (e.g. halo substituted) or unsubstituted -C₁-C₄ alkylbisphenyl. R₂ may be selected from -C₄-C₁₆ alkyl, -C₆-C₁₈ alkenyl and substituted (e.g. halo substituted) or unsubstituted -C₁-C₄ alkylbisphenyl. R₂ may be selected from -C₆-C₁₄ cycloalkyl (e.g. bridged cycloalkyl), -C₁-C₄ alkyl-C₆-C₁₄ cycloalkyl (e.g. bridged cycloalkyl), -C₆-C₁₄ alkyl, -C₆-C₁₆ alkenyl and substituted or unsubstituted -C₁-C₄ alkylbisphenyl. R₂ may be selected from -C₆-C₁₄ alkyl, -C₆-C₁₆ alkenyl and substituted or unsubstituted -C₁-C₄ alkylbisphenyl. R₂ may be selected from -C₆-C₁₄ cycloalkyl (e.g. bridged cycloalkyl, such as adamantyl), -CH₂-C₆-C₁₄ cycloalkyl (e.g. bridged cycloalkyl, such as adamantyl), -C₆-C₁₄ alkyl, -geranyl, -farnesyl and -chlorobisphenyl.

R₂ may be selected from -H, -C₆-C₁₄ alkyl, -C₁-C₄ adamantyl, -adamantyl, -geranyl, -farnesyl and -chlorobisphenyl. R₁ may be selected from -H, -C₆-C₁₄ alkyl, -geranyl, - farnesyl and -chlorobisphenyl.

R₂ may be -H. R₂ may be substituted or unsubstituted -C₄-C₂₀ alkyl, for example substituted or unsubstituted -C₄-C₁₆ alkyl. R₂ may be substituted or unsubstituted -C₂-C₁₄ alkyl, for example substituted or unsubstituted -C₄-C₁₂ alkyl, e.g. -C₄-C₁₀ alkyl. R₂ may be unsubstituted -C₄-C₂₀ alkyl, for example unsubstituted -C₄-C₁₆ alkyl. R₂ may be substituted or unsubstituted -C₄-C₂₀ alkenyl, for example substituted or unsubstituted -C₆-C₁₈ alkenyl. R₂ may be unsubstituted -C₄-C₂₀ alkenyl, for example unsubstituted -C₆-C₁₈ alkenyl. R₂ may be substituted or unsubstituted -C₁-C₄ alkylaryl. R₂ may be -C₁-C₄ alkyl-[O(CH₂)ₚ]_{q}-O(CH₂)ₚ₋₁CH₃, where p is selected from 2 and 3 and q is selected from 0 to 20. p may be 2. p may be 3. q may be selected from 2 to 16, e.g. q may be selected from 4 to 12. p may be 2 and q may be selected from 2 to 16. p may be 3 and q may be selected from 2 to 16. R₂ may be -C₂-C₁₂ alkyl-S-S-C₁-C₁₂ alkyl, for example -C₂-C₁₀ alkyl-S-S-C₂-C₁₀ alkyl; e.g. -C₂-C₈ alkyl-S-S-C₂-C₈ alkyl.

When R₂ is a substituted moiety, it may be substituted by at least one -OH, =O, - CN, -halo, -NH₂, -NH(C₁-C₆ alkyl), -N(C₁-C₄ alkyl)₂; for example it may be substituted by at least one -OH, =O, -CN, -halo. When R₂ is a substituted or unsubstituted alkyl, the carbon backbone of the alkyl may be interrupted by at least one disulfide linkage (-S-S-), thioether linkage (-S-), ether linkage -O- or ester linkage(-C(O)O-).

Each of R₁ and R₂ may be independently selected from substituted or unsubstituted -C₂-C₁₂ alkyl, substituted or unsubstituted -C₂-C₁₂ alkenyl, -C₁-C₄ alkyl-[O(CH₂)ₘ]ₙ-O(CH₂)ₘ₋₁CH₃, where m is selected from 2 and 3 and n is selected from 0 to 20, and -C₂-C₁₂ alkyl-S-S-C₁-C₁₂ alkyl. Each of R₁ and R₂ may be independently selected from - C₂-C₁₂ alkyl, -C₂-C₁₂ alkenyl, -C₁-C₄ alkyl-[O(CH₂)ₘ]ₙ-O(CH₂)ₘ₋₁CH₃, where m is selected from 2 and 3 and n is selected from 0 to 20, and -C₂-C₁₂ alkyl-S-S-C₁-C₁₂ alkyl. Each of R₁ and R₂ may be independently selected from -C₂-C₁₀ alkyl, -C₂-C₁₀ alkenyl, -C₁-C₄ alkyl-[O(CH₂)ₘ]ₙ-O(CH₂)ₘ₋₁CH₃, where m is selected from 2 and 3 and n is selected from 2 to 16, and -C₂-C₁₀ alkyl-S-S-C₂-C₁₀ alkyl. Each of R₁ and R₂ may be independently selected from - C₂-C₁₀ alkyl, -C₂-C₁₀ alkenyl and -C₁-C₄ alkyl-[O(CH₂)ₘ]ₙ-O(CH₂)ₘ₋₁CH₃, where m is selected from 2 and 3 and n is selected from 2 to 16. Each of R₁ and R₂ may be independently selected from substituted or unsubstituted -C₂-C₁₂ alkyl and substituted or unsubstituted -C₂-C₁₂ alkenyl. Each of R₁ and R₂ may be independently selected from -C₂-C₁₂ alkyl and -C₂-C₁₂ alkenyl. Each of R₁ and R₂ may be independently selected from substituted or unsubstituted -C₂-C₁₀ alkyl and substituted or unsubstituted -C₂-C₁₀ alkenyl. Each of R₁ and R₂ may be independently selected from -C₂-C₁₀ alkyl and -C₂-C₁₀ alkenyl. Each of R₁ and R₂ may be independently selected from substituted or unsubstituted -C₄-C₁₀ alkyl and substituted or unsubstituted -C₄-C₁₀ alkenyl. Each of R₁ and R₂ may be independently selected from -C₄-C₁₀ alkyl and -C₄-C₁₀ alkenyl. Each of R₁ and R₂ may be independently selected from substituted or unsubstituted -C₄-C₈ alkyl and substituted or unsubstituted -C₄-C₈ alkenyl. Each of R₁ and R₂ may be independently selected from -C₄-C₈ alkyl and -C₄-C₈ alkenyl.

Each of R₁ and R₂ may be substituted or unsubstituted -C₂-C₁₂ alkyl. Each of R₁ and R₂ may be -C₂-C₁₂ alkyl. Each of R₁ and R₂ may be substituted or unsubstituted -C₂-C₁₀ alkyl. Each of R₁ and R₂ may be -C₂-C₁₀ alkyl. Each of R₁ and R₂ may be substituted or unsubstituted -C₄-C₁₀ alkyl. Each of R₁ and R₂ may be -C₄-C₁₀ alkyl. Each of R₁ and R₂ may be substituted or unsubstituted -C₄-C₈ alkyl. Each of R₁ and R₂ may be -C₄-C₈ alkyl. Each of R₁ and R₂ may be substituted or unsubstituted -C₂-C₁₂ alkenyl. Each of R₁ and R₂ may be -C₂-C₁₂ alkenyl. Each of R₁ and R₂ may be substituted or unsubstituted -C₂-C₁₀ alkenyl. Each of R₁ and R₂ may be -C₂-C₁₀ alkenyl. Each of R₁ and R₂ may be substituted or unsubstituted -C₄-C₁₀ alkenyl. Each of R₁ and R₂ may be -C₄-C₁₀ alkenyl. Each of R₁ and R₂ may be substituted or unsubstituted -C₄-C₈ alkenyl. Each of R₁ and R₂ may be -C₄-C₈ alkenyl. Each of R₁ and R₂ may be -C₁-C₄ alkyl-[O(CH₂)ₘ]ₙ-O(CH₂)ₘ₋₁CH₃, where m is selected from 2 and 3 and n is selected from 0 to 20. m may be 2. m may be 3. n may be selected from 2 to 16, e.g. n may be selected from 4 to 12. m may be 2 and n may be selected from 2 to 16. m may be 3 and n may be selected from 2 to 16. Each of R₁ and R₂ may be -C₂-C₁₂ alkyl-S-S-C₁-C₁₂ alkyl, for example -C₂-C₁₀ alkyl-S-S-C₂-C₁₀ alkyl; e.g. -C₂-C₈ alkyl-S-S-C₂-C₈ alkyl.

R₁ and R₂ may be the same, e.g. R₁ and R₂ may each be the same substituted or unsubstituted -C₂-C₁₂ alkyl. R₁ and R₂ may be different, e.g. R₂ may be -H and R₁ may be another substituent as specified elsewhere herein.

R₁ may be selected from

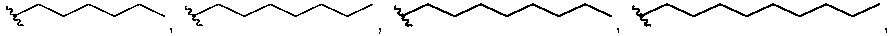

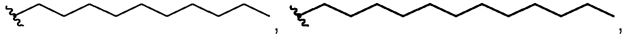

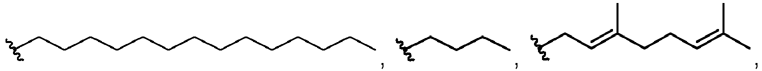

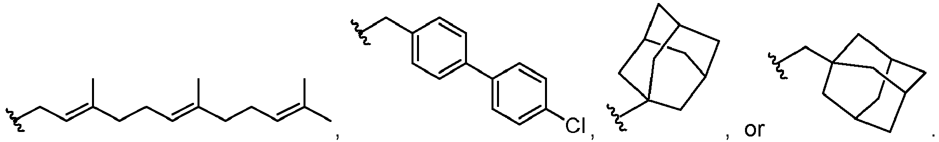

R₂ may be selected from H, 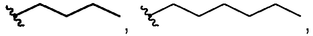 or

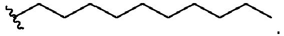

R₁ may be selected from 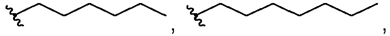 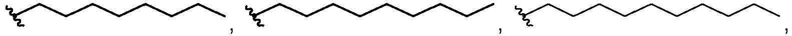 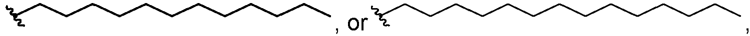 and R₂ may be H. R₁ may be 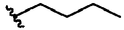 and R₂ may be 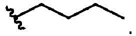 R₁ may be 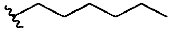 and R₂ may be 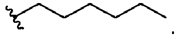 R₁ may be 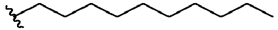 and R₂ may be

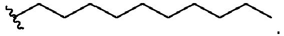

R₃ is selected from -OH, substituted or unsubstituted -C₁-C₂₀ alkyl, substituted or unsubstituted -C₂-C₂₀ alkenyl, substituted or unsubstituted -C₁-C₄ alkylaryl and carbohydrate. The substituted -C₁-C₂₀ alkyl may be, for example, -NHCH₂CH₂CH₂N(CH₃)₂, or 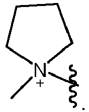 The carbohydrate is selected from -(NHCH₂CH₂)ₓ-Glc, -(NHCH₂CH₂)ₓ-Gal, - (NHCH₂CH₂)ₓ-Man, -(NHCH₂CH₂)ₓ-GlcNAc, -(NHCH₂CH₂)ₓ-MurNAc, -(NHCH₂CH₂)ₓ-ManNAc, -(NHCH₂CH₂)ₓ-GalNAc, -(NHCH₂CH₂)ₓ-cellbiose and -(NHCH₂CH₂)ₓ-maltose, where x is 0 or 1. R₃ may be selected from -OH, -C₁-C₂₀ alkyl, -C₂-C₂₀ alkenyl, -C₁-C₄ alkylaryl and carbohydrate. R₃ may be selected from -OH, substituted or unsubstituted -C₁-C₁₀ alkyl, substituted or unsubstituted -C₂-C₁₀ alkenyl, substituted or unsubstituted -C₁-C₄ alkylaryl and carbohydrate. R₃ may be selected from -OH, -C₁-C₁₀ alkyl, -C₂-C₁₀ alkenyl, - C₁-C₄ alkylaryl and carbohydrate. R₃ may be selected from -OH, -NHCH₂CH₂CH₂N(CH₃)₂, 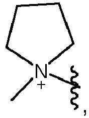 -(NHCH₂CH₂)ₓ-Glc, -(NHCH₂CH₂)ₓ-Gal, -(NHCH₂CH₂)ₓ-Man, -(NHCH₂CH₂)ₓ-GlcNAc, -(NHCH₂CH₂)ₓ-MurNAc, -(NHCH₂CH₂)ₓ-ManNAc, -(NHCH₂CH₂)ₓ-GalNAc, - (NHCH₂CH₂)ₓ-cellbiose and -(NHCH₂CH₂)ₓ-maltose, where x is 0 or 1. x may be 0. x may be 1.

R₃ may be -OH. R₃ may be substituted or unsubstituted -C₁-C₂₀ alkyl; for example substituted or unsubstituted C₁-C₁₀ alkyl, e.g. substituted or unsubstituted C₁-C₆ alkyl. R₃ may be -C₁-C₂₀ alkyl; for example C₁-C₁₀ alkyl, e.g. C₁-C₆ alkyl. R₃ may be substituted or unsubstituted -C₂-C₂₀ alkenyl; for example substituted or unsubstituted C₂-C₁₀ alkenyl, e.g. substituted or unsubstituted C₂-C₆ alkenyl. R₃ may be -C₂-C₂₀ alkenyl; for example C₂-C₁₀ alkenyl, e.g. C₂-C₆ alkenyl. R₃ may be substituted or unsubstituted -C₁-C₄ alkylaryl, for example -C₁-C₄ alkylaryl. R₃ may be carbohydrate (e.g. comprising a glycan selected from Glc, Gal, Man, GlcNAc, MurNAc, ManNAc, GaINAc, cellbiose and maltose), optionally comprising a linker such as -NHCH₂CH₂-. R₃ may be -NHCH₂CH₂CH₂N(CH₃)₂. R₃ may be 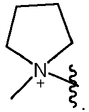 R₃ may be -(NHCH₂CH₂)ₓ-Glc. R₃ may be -(NHCH₂CH₂)ₓ-Gal. R₃ may be - (NHCH₂CH₂)ₓ-Man. R₃ may be -(NHCH₂CH₂)ₓ-GlcNAc. R₃ may be -(NHCH₂CH₂)ₓ-MurNAc. R₃ may be -(NHCH₂CH₂)ₓ-ManNAc. R₃ may be -(NHCH₂CH₂)ₓ-GalNAc. R₃ may be - (NHCH₂CH₂)ₓ-cellbiose. R₃ may be -(NHCH₂CH₂)ₓ-maltose. x may be 0. x may be 1.

R₄ may be selected from -H, substituted or unsubstituted -C₁-C₂₀ alkyl, substituted or unsubstituted -C₂-C₂₀ alkenyl, substituted or unsubstituted -C₁-C₄ alkylaryl and carbohydrate. The substituted -C₁-C₂₀ alkyl may be substituted with one or more nitrogen, phosphorous or oxygen; for example, the substituted -C₁-C₂₀ alkyl may be - CH₂NHCH₂P(O)(OH)₂, -CH₂N(CH₂PO₃H₂)₂, -CH₂NHCH₂CH₂CH₂N(CH₃)₂, - CH₂NHCH₂CH₂COOH, -CH₂N(CH₃)CH₂(CH(OH))₄CH₂OH, - CH₂NHCH(COOH)CH₂COOH, - CH₂NH(CH₂CH₂OH)₂. The carbohydrate is selected from -(NHCH₂CH₂)_{y}-Glc, - (NHCH₂CH₂)_{y}-Gal, -(NHCH₂CH₂)_{y}-Man, -(NHCH₂CH₂)_{y}-GlcNAc, -(NHCH₂CH₂)_{y}-MurNAc, - (NHCH₂CH₂)_{y}-ManNac, -(NHCH₂CH₂)_{y}-GalNAc, -(NHCH₂CH₂)_{y}-cellbiose and - (NHCH₂CH₂)_{y}-maltose, where y is 0 or 1. R₄ may be selected from -H, -C₁-C₂₀ alkyl, -C₂-C₂₀ alkenyl, -C₁-C₄ alkylaryl and carbohydrate. R₄ may be selected from -H, substituted or unsubstituted -C₁-C₁₀ alkyl, substituted or unsubstituted -C₂-C₁₀ alkenyl, substituted or unsubstituted -C₁-C₄ alkylaryl and carbohydrate. R₄ is selected from -H, - CH₂NHCH₂P(O)(OH)₂, -CH₂N(CH₂PO₃H₂)₂, -CH₂NHCH₂CH₂CH₂N(CH₃)₂, - CH₂NHCH₂CH₂COOH, -CH₂N(CH₃)CH₂(CH(OH))₄CH₂OH, - CH₂NHCH(COOH)CH₂COOH, - CH₂NH(CH₂CH₂OH)₂, -(NHCH₂CH₂)_{y}-Glc, -(NHCH₂CH₂)_{y}-Gal, -(NHCH₂CH₂)_{y}-Man, - (NHCH₂CH₂)_{y}-GlcNAc, -(NHCH₂CH₂)_{y}-MurNAc, -(NHCH₂CH₂)_{y}-ManNac, -(NHCH₂CH₂)_{y}-GaINAc, -(NHCH₂CH₂)_{y}-cellbiose and -(NHCH₂CH₂)_{y}-maltose, where y is 0 or 1. y may be 0. y may be 1.

R₄ may be -H. R₄ may be substituted or unsubstituted -C₁-C₂₀ alkyl; for example substituted or unsubstituted C₁-C₁₀ alkyl, e.g. substituted or unsubstituted C₁-C₆ alkyl. R₄ may be -C₁-C₂₀ alkyl; for example C₁-C₁₀ alkyl, e.g. C₁-C₆ alkyl. R₄ may be substituted or unsubstituted -C₂-C₂₀ alkenyl; for example substituted or unsubstituted C₂-C₁₀ alkenyl, e.g. substituted or unsubstituted C₂-C₆ alkenyl. R₄ may be -C₂-C₂₀ alkenyl; for example C₂-C₁₀ alkenyl, e.g. C₂-C₆ alkenyl. R₄ may be substituted or unsubstituted -C₁-C₄ alkylaryl, for example -C₁-C₄ alkylaryl. R₄ may be carbohydrate (e.g. comprising a glycan selected from Glc, Gal, Man, GlcNAc, MurNAc, ManNAc, GaINAc, cellbiose and maltose), optionally comprising a linker such as -NHCH₂CH₂-. R₄ may be -CH₂NHCH₂P(O)(OH)₂. R₄ may be - CH₂N(CH₂PO₃H₂)₂. R₄ may be -CH₂NHCH₂CH₂CH₂N(CH₃)₂. R₄ may be - CH₂NHCH₂CH₂COOH. R₄ may be -CH₂N(CH₃)CH₂(CH(OH))₄CH₂OH. R₄ may be - CH₂NHCH(COOH)CH₂COOH. R₄ may be -CH₂NH(CH₂CH₂OH)₂. R₄ may be - (NHCH₂CH₂)_{y}-Glc. R₄ may be -(NHCH₂CH₂)_{y}-Gal. R₄ may be -(NHCH₂CH₂)_{y}-Man. R₄ may be -(NHCH₂CH₂)_{y}-GlcNAc. R₄ may be -(NHCH₂CH₂)_{y}-MurNAc. R₄ may be -(NHCH₂CH₂)_{y}-ManNac. R₄ may be -(NHCH₂CH₂)_{y}-GalNAc. R₄ may be -(NHCH₂CH₂)_{y}-cellbiose. R₄ may be -(NHCH₂CH₂)_{y}-maltose. y may be 0. y may be 1.

L₁ is a linker selected from -C₁-C₂₀ alkylene-, -C₂-C₂₀ alkenylene-, -(C₁-C₄ alkyl)arylene-, -C₂-C₄ alkyl-[O(CH₂)ᵣ]ₛ-O(CH₂)ᵣ-, -C₂-C₁₂ alkyl-S-S-C₁-C₁₂ alkyl-, -C(O)C₁-C₂₀ alkylene-, -C(O)C₂-C₂₀ alkenylene-, -C(O)(C₁-C₄ alkyl)arylene-, -C(O)C₁-C₄ alkyl-[O(CH₂)ᵣ]ₛ-O(CH₂)ᵣ-, -C(O)NHC₁-C₂₀ alkylene-, -C(O)NHC₂-C₂₀ alkenylene-, --C(O)NH(C₁-C₄ alkyl)arylene-, -C(O)NHC₁-C₄ alkyl-[O(CH₂)ᵣ]ₛ-O(CH₂)ᵣ-, -C(S)NHC₁-C₂₀ alkylene-, - C(S)NHC₂-C₂₀ alkenylene-, -C(S)NH(C₁-C₄ alkyl)arylene- and -C(S)NHC₁-C₄ alkyl-[O(CH₂)ᵣ]ₛ-O(CH₂)ᵣ-, where r is selected from 2 and 3 and s is selected from 0 to 20. L₁ may be selected from -C₂-C₁₂ alkylene-, -C₂-C₁₂ alkenylene-, -(C₁-C₄ alkyl)arylene-, -C₂-C₄ alkyl-[O(CH₂)ᵣ]ₛ-O(CH₂)ᵣ-, -C₂-C₁₀ alkyl-S-S-C₁-C₁₀ alkyl-, -C(O)C₁-C₁₂ alkylene-, -C(O)C₂-C₁₂ alkenylene-, -C(O)(C₁-C₄ alkyl)arylene-, -C(O)C₁-C₄ alkyl-[O(CH₂)ᵣ]ₛ-O(CH₂)ᵣ-, -C(O)NHC₁-C₁₂ alkylene-, -C(O)NHC₂-C₁₂ alkenylene-, --C(O)NH(C₁-C₄ alkyl)arylene-, -C(O)NHC₁-C₄ alkyl-[O(CH₂)ᵣ]ₛ-O(CH₂)ᵣ-, -C(S)NHC₁-C₁₂ alkylene-, -C(S)NHC₂-C₁₂ alkenylene-, - C(S)NH(C₁-C₄ alkyl)arylene- and -C(S)NHC₁-C₄ alkyl-[O(CH₂)ᵣ]ₛ-O(CH₂)ᵣ-, where r is selected from 2 and 3 and s is selected from 0 to 20. L₁ may be selected from -C₂-C₁₀ alkylene-, -C₂-C₁₀ alkenylene-, -(C₁-C₄ alkyl)arylene-, -C₂-C₄ alkyl-[O(CH₂)ᵣ]ₛ-O(CH₂)ᵣ-, -C₂-Cs alkyl-S-S-C₁-C₈ alkyl-, -C(O)C₁-C₁₀ alkylene-, -C(O)C₂-C₁₀ alkenylene-, -C(O)(C₁-C₄ alkyl)arylene-, -C(O)C₁-C₄ alkyl-[O(CH₂)ᵣ]ₛ-O(CH₂)ᵣ-, -C(O)NHC₁-C₁₀ alkylene-, -C(O)NHC₂-C₁₀ alkenylene-, --C(O)NH(C₁-C₄ alkyl)arylene-, -C(O)NHC₁-C₄ alkyl-[O(CH₂)ᵣ]ₛ-O(CH₂)ᵣ-, - C(S)NHC₂-C₁₀ alkylene-, -C(S)NHC₂-C₁₀ alkenylene-, -C(S)NH(C₁-C₄ alkyl)arylene- and - C(S)NHC₁-C₄ alkyl-[O(CH₂)ᵣ]ₛ-O(CH₂)ᵣ-, where r is selected from 2 and 3 (e.g. 2) and s is selected from 0 to 20 (e.g. 2 to 16).

L₁ may be selected from -C₂-C₂₀ alkylene-, -C₂-C₂₀ alkenylene- and -(C₁-C₄ alkyl)arylene-. L₁ may be selected from -C₂-C₁₂ alkylene-, -C₂-C₁₂ alkenylene- and -(C₁-C₄ alkyl)arylene-. L₁ may be selected from -C₂-C₁₀ alkylene-, -C₂-C₁₂ alkenylene- and -(C₁-C₄ alkyl)arylene-. L₁ may be selected from -C₂-C₂₀ alkylene- and -(C₁-C₄ alkyl)arylene-. L₁ may be selected from -C₂-C₁₂ alkylene- and -(C₁-C₄ alkyl)arylene-. L₁ may be selected from -C₂-C₁₀ alkylene- (e.g. -C₂-C₈ alkylene-) and -(C₁-C₄ alkyl)arylene-.

L₁ may be -(C₁-C₄ alkyl)arylene-; for example -CH₂Ph-. L₁ may be -C₂-C₂₀ alkylene-; for example -C₂-C₁₂ alkylene-, e.g. a -C₂-C₁₀ alkylene- (such as a -C₂-C₈ alkylene-). L₁ may be -C₂-C₂₀ alkenylene-; for example -C₂-C₁₂ alkenylene-, e.g. a -C₂-C₁₀ alkenylene-. L₁ may be -C₂-C₄ alkyl-[O(CH₂)ᵣ]ₛ-O(CH₂)ᵣ-. L₁ may be -C₂-C₁₂ alkyl-S-S-C₂-C₁₂ alkyl-, for example -C₂-C₁₀ alkyl-S-S-C₂-C₁₀ alkyl-; e.g. -C₂-C₈ alkyl-S-S-C₂-C₈ alkyl-. L₁ may be -C(O)C₂-C₂₀ alkenylene-; for example -C(O)C₁-C₁₂ alkylene-, e.g. -C(O)C₁-C₁₀ alkylene-. L₁ may be -C(O)(C₁-C₄ alkyl)arylene-. L₁ may be -C(O)C₁-C₄ alkyl-[O(CH₂)ᵣ]ₛ-O(CH₂)ᵣ-. L₁ may be -C(O)NHC₁-C₂₀ alkylene-; for example -C(O)NHC₁-C₁₂ alkylene-, e.g. - C(O)NHC₁-C₁₀ alkylene-. L₁ may be -C(O)NHC₂-C₂₀ alkenylene-; for example -C(O)NHC₂-C₁₂ alkenylene-, e.g. -C(O)NHC₂-C₁₀ alkenylene-. L₁ may be -C(O)NH(C₁-C₄ alkyl)arylene-. L₁ may be -C(O)NHC₁-C₄ alkyl-[O(CH₂)ᵣ]ₛ-O(CH₂)ᵣ-. L₁ may be -C(S)NHC₁-C₂₀ alkylene-; for example -C(S)NHC₁-C₁₂ alkylene-, e.g. -C(S)NHC₁-C₁₀ alkylene-. L₁ may be -C(S)NHC₂-C₂₀ alkenylene-; for example -C(S)NHC₂-C₁₂ alkenylene-, e.g. -C(S)NHC₂-C₁₀ alkenylene-. L₁ may be -C(S)NH(C₁-C₄ alkyl)arylene-. L₁ may be -C(S)NHC₁-C₄ alkyl-[O(CH₂)ᵣ]ₛ-O(CH₂)ᵣ-.

r may be 2. r may be 3. s may be selected from 2 to 16, e.g. s may be selected from 4 to 12. r may be 2 and s may be selected from 2 to 16. r may be 3 and s may be selected from 2 to 16.

The compound may be selected from: 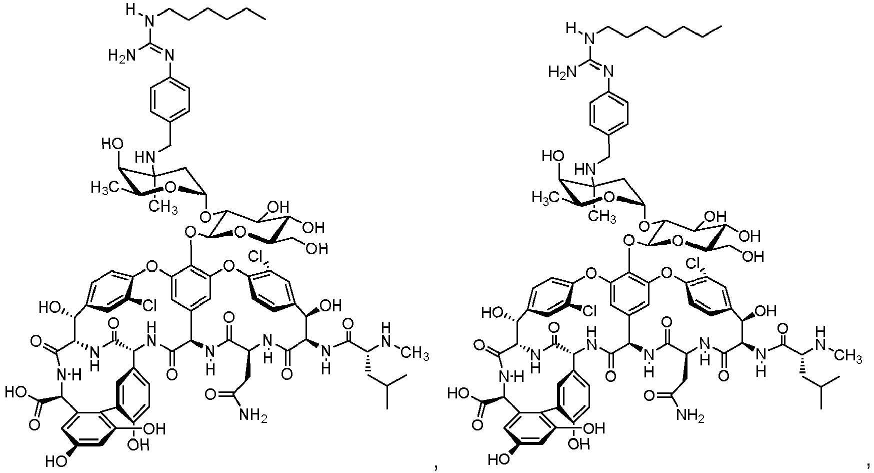 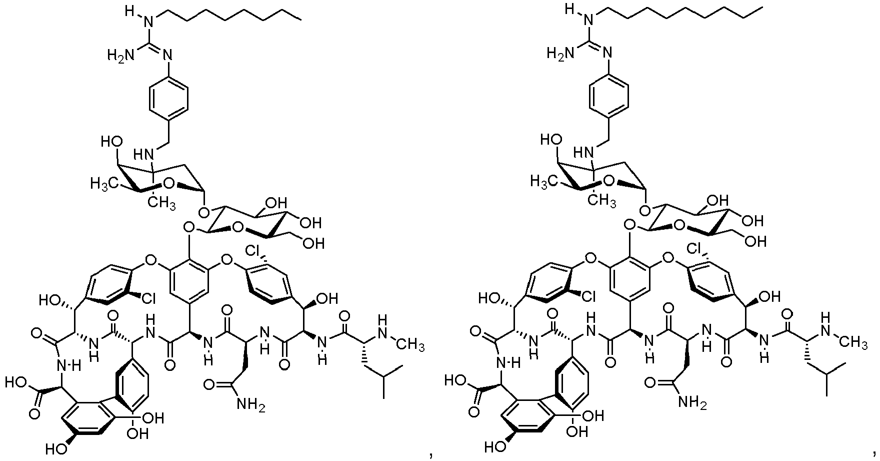 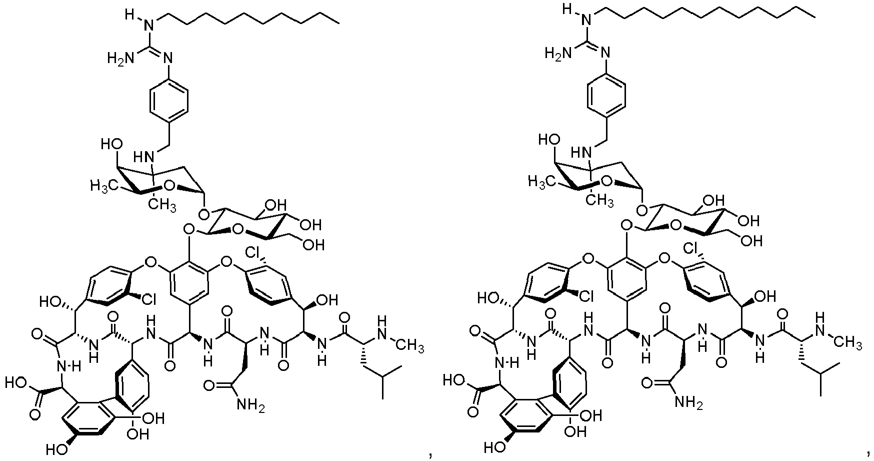 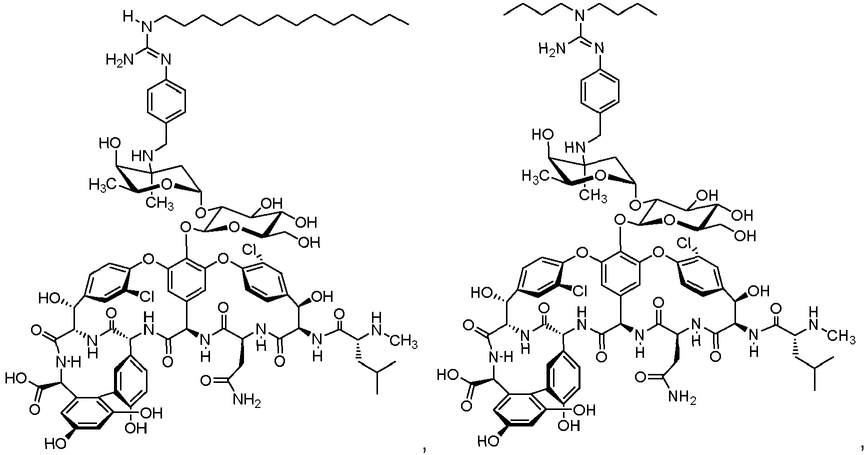 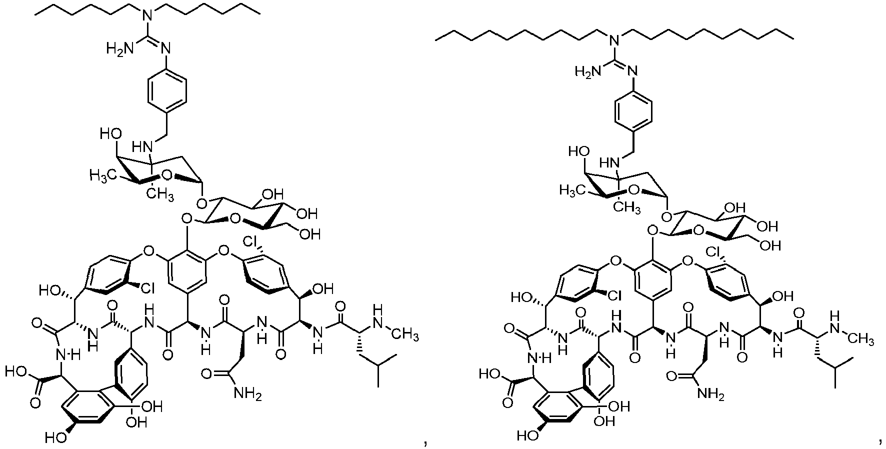 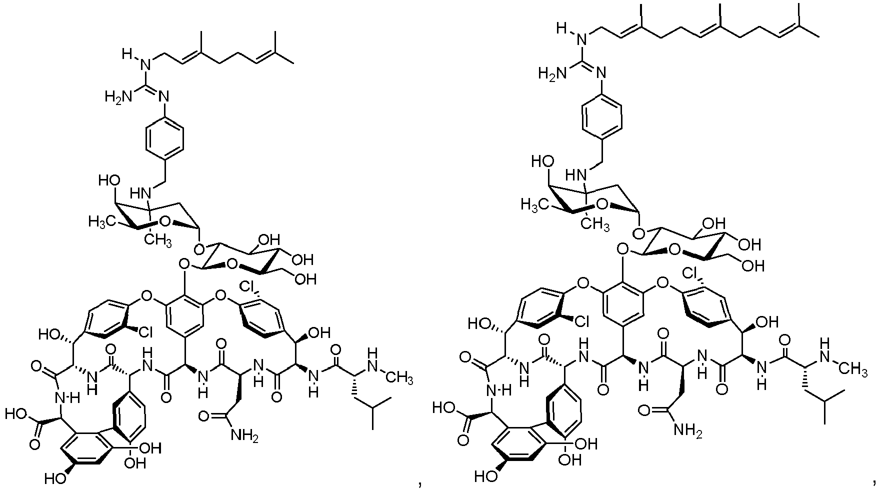 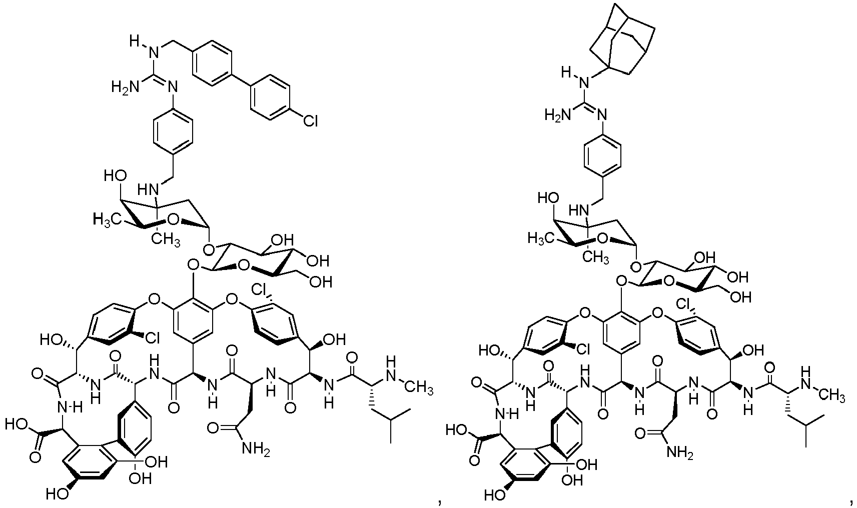 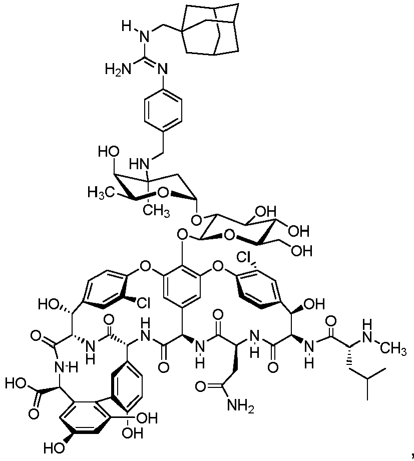 or a pharmaceutically acceptable salt or solvate thereof.

Exemplary compounds may be made in accordance with the methods of synthesis illustrated in the examples and in accordance with reaction schemes A and B. In addition, as the skilled person will appreciate, these methods and the methods illustrated in reaction schemes A and B (see below) may be readily adapted to provide other compounds of the present invention and disclosure.

### FORMULATIONS AND ADMINISTRATION

According to a further aspect of the invention there is provided a pharmaceutical formulation or composition including a compound of the invention, optionally in admixture with at least one pharmaceutically acceptable adjuvant, diluent or carrier.

The formulation or composition may be a parenteral formulation or an oral formulation. The formulation may be a parenteral formulation, for example a formulation for intravenous injection. The formulation may be an oral formulation.

Compounds, formulations or compositions of the invention may be administered orally, topically, intravenously, subcutaneously, buccally, rectally, dermally, nasally, tracheally, bronchially, by any other parenteral route, as an oral or nasal spray or via inhalation. The compounds may be administered in the form of pharmaceutical preparations comprising the compound either as a free compound or, for example, a pharmaceutically acceptable non-toxic organic or inorganic acid or base addition salt, in a pharmaceutically acceptable dosage form. Depending upon the disorder and patient to be treated and the route of administration, the compositions may be administered at varying doses.

Typically, therefore, the pharmaceutical compounds of the invention may be administered parenterally ("parenterally" as used herein, refers to modes of administration which include intravenous, intramuscular, intraperitoneal, intrasternal, subcutaneous and intraarticular injection and infusion) or orally to a host to obtain an antibacterial effect. For example, the pharmaceutical compounds of the invention may be administered by intravenous injection or infusion. In the case of larger animals, such as humans, the compounds may be administered alone or as compositions in combination with pharmaceutically acceptable diluents, excipients or carriers.

Actual dosage levels of active ingredients in the pharmaceutical formulations and pharmaceutical compositions of this invention may be varied so as to obtain an amount of the active compound(s) that is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved. Suitable doses are generally in the range of from 0.01 - 100 mg/kg/day, for example in the range of 0.1 to 50 mg/kg/day.

Pharmaceutical formulations or compositions of this invention for parenteral (e.g. intravenous) injection may comprise pharmaceutically acceptable sterile aqueous or non-aqueous solutions, dispersions, suspensions or emulsions as well as sterile powders for reconstitution into sterile injectable solutions or dispersions just prior to use. Examples of suitable aqueous and non-aqueous carriers, diluents, solvents or vehicles include water, ethanol, polyols (such as glycerol, propylene glycol, polyethylene glycol and the like), and suitable mixtures thereof, vegetable oils (such as olive oil) and injectable organic esters, such as ethyl oleate. Proper fluidity can be maintained, for example, by the use of coating materials, such as lecithin, by the maintenance of the required particle size in the case of dispersions and by the use of surfactants. Formulations or compositions for parenteral injection may represent preferred formulations or compositions of the invention.

These compositions may also contain adjuvants such as preservative, wetting agents, emulsifying agents and dispersing agents. Inhibition of the action of microorganisms may be ensured by the inclusion of various antibacterial and antifungal agents, for example, paraben, chlorobutanol or phenol sorbic acid. It may also be desirable to include isotonic agents, such as sugars or sodium chloride, for example. Prolonged absorption of the injectable pharmaceutical form may be brought about by the inclusion of agents (for example, aluminium monostearate and gelatine) which delay absorption.

Solid dosage forms for oral administration include capsules, tablets, pills, powders and granules. In such solid dosage forms, the active compound is typically mixed with at least one inert, pharmaceutically acceptable excipient or carrier, such as sodium citrate or dicalcium phosphate and/or one or more: a) fillers or extenders, such as starches, lactose, sucrose, glucose, mannitol and silicic acid; b) binders, such as carboxymethylcellulose, alginates, gelatin, polyvinylpyrrolidone, sucrose and acacia; c) humectants, such as glycerol; d) disintegrating agents, such as agar-agar, calcium carbonate, potato or tapioca starch, alginic acid, certain silicates and sodium carbonate; e) solution retarding agents, such as paraffin; f) absorption accelerators, such as quaternary ammonium compounds; g) wetting agents, such as cetyl alcohol and glycerol monostearate; h) absorbents, such as kaolin and bentonite clay and i) lubricants, such as talc, calcium stearate, magnesium stearate, solid polyethylene glycols, sodium lauryl sulfate and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycol, for example.

Oral formulations may contain a dissolution aid. Examples of dissolution aids include nonionic surface active agents, such as sucrose fatty acid esters, glycerol fatty acid esters, sorbitan fatty acid esters (e.g. sorbitan trioleate), polyethylene glycol, polyoxyethylene hydrogenated castor oil, polyoxyethylene sorbitan fatty acid esters, polyoxyethylene alkyl ethers, methoxypolyoxyethylene alkyl ethers, polyoxyethylene alkylphenyl ethers, polyethylene glycol fatty acid esters, polyoxyethylene alkylamines, polyoxyethylene alkyl thioethers, polyoxyethylene polyoxypropylene copolymers, polyoxyethylene glycerol fatty acid esters, pentaerythritol fatty acid esters, propylene glycol monofatty acid esters, polyoxyethylene propylene glycol monofatty acid esters, polyoxyethylene sorbitol fatty acid esters, fatty acid alkylolamides, and alkyamine oxides; bile acid and salts thereof (e.g. chenodeoxycholic acid, cholic acid, deoxycholic acid, dehydrocholic acid and salts thereof, and glycine or taurine conjugate thereof); ionic surface active agents, such as sodium laurylsulfate, fatty acid soaps, alkylsufonates, alkylphosphates, ether phosphates, fatty acid salts of basic amino acids; triethanolamine soap, and alkyl quaternary ammonium salts; and amphoteric surface active agents, such as betaines and aminocarboxylic acid salts.

The solid dosage forms of tablets, dragees, capsules, pills, and granules can be prepared with coatings and shells such as enteric coatings and other coatings well known in the pharmaceutical formulating art. They may optionally contain opacifying agents and may also be of a composition such that they release the active ingredient(s) only, or preferentially, in a certain part of the intestinal tract, and/or in delayed fashion. Examples of embedding compositions include polymeric substances and waxes.

The active compounds may also be in microencapsulated form, if appropriate, with one or more of the above-mentioned excipients.

The active compounds may be in finely divided form, for example it may be micronized.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers such as ethyl alcohol, isopropyl alcohol, ethyl carbonate, ethyl acetate, benzyl alcohol, benzyl benzoate, propylene glycol, 1 ,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof. Besides inert diluents, the oral compositions may also include adjuvants such as wetting agents, emulsifying and suspending agents, sweetening, flavouring and perfuming agents. Suspensions, in addition to the active compounds, may contain suspending agents such as ethoxylated isostearyl alcohols, polyoxyethylene sorbitol and sorbitan esters, microcrystalline cellulose, aluminium metahydroxide, bentonite, agar-agar, and traganacanth and mixtures thereof.

Compositions for rectal or vaginal administration may be in the form of suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, sprays, creams, foams, gels, ointments and inhalants. The active compound is mixed under sterile conditions with a pharmaceutically acceptable carrier and any needed preservatives, buffers or propellants which may be required. Ophthalmic formulations, eye ointments, powders and solutions are also contemplated as being within the scope of this invention.

Liquid (e.g. aqueous) formulations and compositions, whether intended for parenteral or oral use, may comprise additional compound(s) to help prevent precipitation of the active compound. Compounds of the invention are glycopeptide derivatives. Precipitation of such compounds in aqueous solution may be avoided or minimised by including a monosaccharide in the solution. For example, aqueous formulations or compositions may comprise glucose. In particular, a parenteral (e.g. intravenous injection) formulation or composition may comprise a compound of the invention, water for injection and glucose.

Insofar as they do not interfere with the activity of the compounds, the formulations or compositions according to the present subject matter may contain other active agents intended, in particular, for use in treating a bacterial infection.

The formulations according to the present subject matter may also contain inactive components. Suitable inactive components are well known in the art and are described in standard textbooks, such as Goodman and Gillman's: The Pharmacological Bases of Therapeutics, 13thEd., Brunton et al., Eds. McGraw-Hill Education (2017), and Remington's Pharmaceutical Sciences, 17th Ed., Mack Publishing Co., Easton, Pa. (1990).

The formulations may be used in combination with an additional pharmaceutical dosage form to enhance their effectiveness in treating any of the disorders described herein. In this regard, the present formulations may be administered as part of a regimen additionally including any other pharmaceutical and/or pharmaceutical dosage form known in the art as effective for the treatment of any of these disorders.

### USES

The compounds of the invention represent novel guanidino containing derivatives of the glycopeptide vancomycin.

Vancomycin is an antibiotic that is active against Gram-positive bacteria, as it interferes with the synthesis of the peptidoglycan layer of Gram-positive bacteria according to the following mechanism. Critical to peptidoglycan biosynthesis is lipid II, the penultimate bacterial cell wall building block. Vancomycin binds tightly to the terminal d-Ala-d-Ala motif of the lipid II pentapeptide.

Resistance to vancomycin is increasingly encountered as many bacterial strains have emerged that can employ a lipid II variant containing a d-Ala-d-Lac terminated pentapeptide. This d-Lac for d-Ala mutation lowers the affinity of vancomycin for lipid II and in doing so greatly reduces it antibacterial effect (Healy, V. L., et al. (2000) Vancomycin resistance in enterococci: reprogramming of the D-ala-D-Ala ligases in bacterial peptidoglycan biosynthesis, Chem Biol 7, R109-119; Blaskovich, M. A. T., et al. (2018) Developments in Glycopeptide Antibiotics, ACS Infect Dis 4, 715-735; Willyard, C. (2017) The drug-resistant bacteria that pose the greatest health threats, Nature 543, 15). We have noted that this mode of resistance is partially overcome in some related lipoglycopeptides wherein the attachment of hydrophobic groups to the vancosamine unit enhances activity. This is typified by the clinically-used drug telavancin (Blaskovich, M. A. T., et al. (2018) Developments in Glycopeptide Antibiotics, ACS Infect Dis 4, 715-735; Willyard, C. (2017) The drug-resistant bacteria that pose the greatest health threats, Nature 543, 15; Corey, G. R., et al. (2009) Telavancin, Nat Rev Drug Discov 8, 929-930). While telavancin is more potent than vancomycin it is also not without its drawbacks. Compared with vancomycin telavancin is significantly less soluble in aqueous media and also carries severe health risks. The FDA recently applied a black box warning to telavancin due to a suspected impact on heart rate (prolonged QT interval) and an increased mortality compared with vancomycin in patients with renal impairment (Barriere, S. L. (2014) The ATTAIN trials: efficacy and safety of telavancin compared with vancomycin for the treatment of hospital-acquired and ventilator-associated bacterial pneumonia, Future Microbiol 9, 281-289). Telavancin is therefore considered a drug of last resort and used only in cases where other treatments are not effective.

Compounds provided herein represent antibiotics, in particular antibiotics useful for the treatment of conditions related to infection by Gram-positive bacteria. The compounds of the invention may provide similar or better antibiotic activity, for example as measured by MIC, compared to known antibiotics such as vancomycin, telavancin, teicoplanin, oritavancin and/or dalbavancin. The compounds of the invention may also provide good safety profiles.

Where the compounds are used for the treatment of a bacterial infection, the bacterial infection may be caused by Gram-negative or Gram-positive bacteria. For example, the bacterial infection may be caused by bacteria from one or more (e.g. at least one) of the following families: *Clostridium, Pseudomonas, Escherichia, Klebsiella, Enterococcus, Enterobacter, Serratia, Stenotrophomonas, Aeromonas, Morganella, Yersinia, Salmonella, Proteus, Pasteurella, Haemophilus, Citrobacter, Burkholderia, Brucella, Moraxella, Mycobacterium, Streptococcus or Staphylococcus. Particular examples include Clostridium, Pseudomonas, Escherichia, Klebsiella, Enterococcus, Enterobacter, Streptococcus* and *Staphylococcus.* The bacterial infection may, for example, be caused by one or more bacteria selected from *Moraxella catarrhalis, Brucella abortus, Burkholderia cepacia, Citrobacter species, Escherichia coli, Haemophilus Pneumonia, Klebsiella Pneumonia, Pasteurella multocida, Proteus mirabilis, Salmonella typhimurium, Clostridium difficile, Yersinia enterocolitica Mycobacterium tuberculosis, Staphylococcus aureus, group B streptococci, Streptococcus Pneumonia,* and *Streptococcus pyogenes.*

The compounds of the invention are particularly useful for the treatment of bacterial infection caused by Gram-positive bacteria. For example, the bacterial infection may be caused by bacteria from one or more (e.g. at least one of) the following families: *Staphylococcus* (e.g. *S*. *aureus, S. epidermidis, S. saprophyticus*)*, Streptococcus* (e.g. *Strep. pyogenes, Strep. agalactiae, Strep. viridans, Strep. pneumonia*)*, Enterococus* (e.g. *E*. *faecalis*)*, Bacillus, Clostridia, Listeria* and *Corynebacterium.* The bacterial infection may be caused by bacteria from one or more (e.g. at least one of) the following families: *Staphylococcus* (e.g. *S*. *aureus, S. epidermidis, S. saprophyticus*)*, Streptococcus* (e.g. *Strep. pyogenes, Strep. agalactiae, Strep. viridans, Strep. pneumonia*)*, Enterococus.* The bacteria may be resistant to treatment with methicillin and/ or vancomycin, for example the bacteria may comprise a strain of methicillin and/or vancomycin resistant *Staphylococcus* (e.g. *S*. *aureus*)*, Streptococcus* or *Enterococus* (e.g. *E. faecium, E. faecalis*)*.* The bacterial infection may comprise an infection by vancomycin-resistant *Enterococcus* (VRE), methicillin-resistant *S*. *aureus* (MRSA), or vancomycin-resistant *S*. *aureus* (VRSA). The bacterial infection may comprise an infection by *S*. *aureus* or *E. faecium.* The bacterial infection may comprise an infection by one or more of *S*. *aureus* ATCC29213, *S*. *aureus* USA300, *E. faecium* E155, *E. faecium* E7314, *E. faecium* E980, *E. faecalis* E1246, or *E*. *faecalis* E7406.

Exemplary bacterial (e.g. Gram-positive) infections that may be treated by compounds of the invention include skin and structure infections, lower respiratory tract infections, bacteremia, sepsis, septicemia, infective endocarditis, peritonitis (e.g. associated with continuous ambulatory peritoneal dialysis), enterocolitis (e.g. staphyloccocal), mastitis, *Clostridium difficile* infection-associated diarrhoea and colitis. The infection that may be treated may be selected from skin and structure infections and bacteremia. Exemplary skin and structure infections include cellulitis/erysipelas, major cutaneous abscesses, and wound infections. Such skin and structure infections may be due to infection by S. *aureus* (including methicillin-susceptible and methicillin-resistant strains), *Strep. pyogenes*, *Strep. agalactiae*, *Strep. dysgalactiae*, *Strep. anginosus* (including *S*. *anginosus, S. intermedius, S. constellates*) or *E. faecalis.* Exemplary lower respiratory tract infections include pneumonia, community-acquired pneumonia (CAP), nosocomial pneumonia, and pleural empyema.

### Synthesis of Compounds

Compounds of the invention can be made according to reaction schemes A and B. Scheme A illustrates a general scheme that may be used, while scheme B outlines methods that can be used with exemplary compounds. As the skilled person will appreciate, scheme B can be readily adapted to provide additional compounds, where substituents R₁ and R₂ are as defined for compounds of the invention and disclosure. 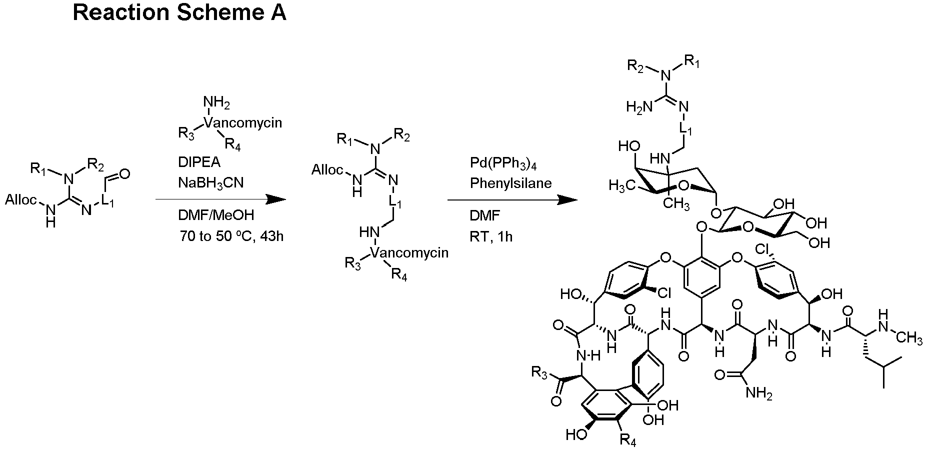 R₁, R₂, R₃, R₄ and L₁ as defined for compounds of the invention and disclosure.

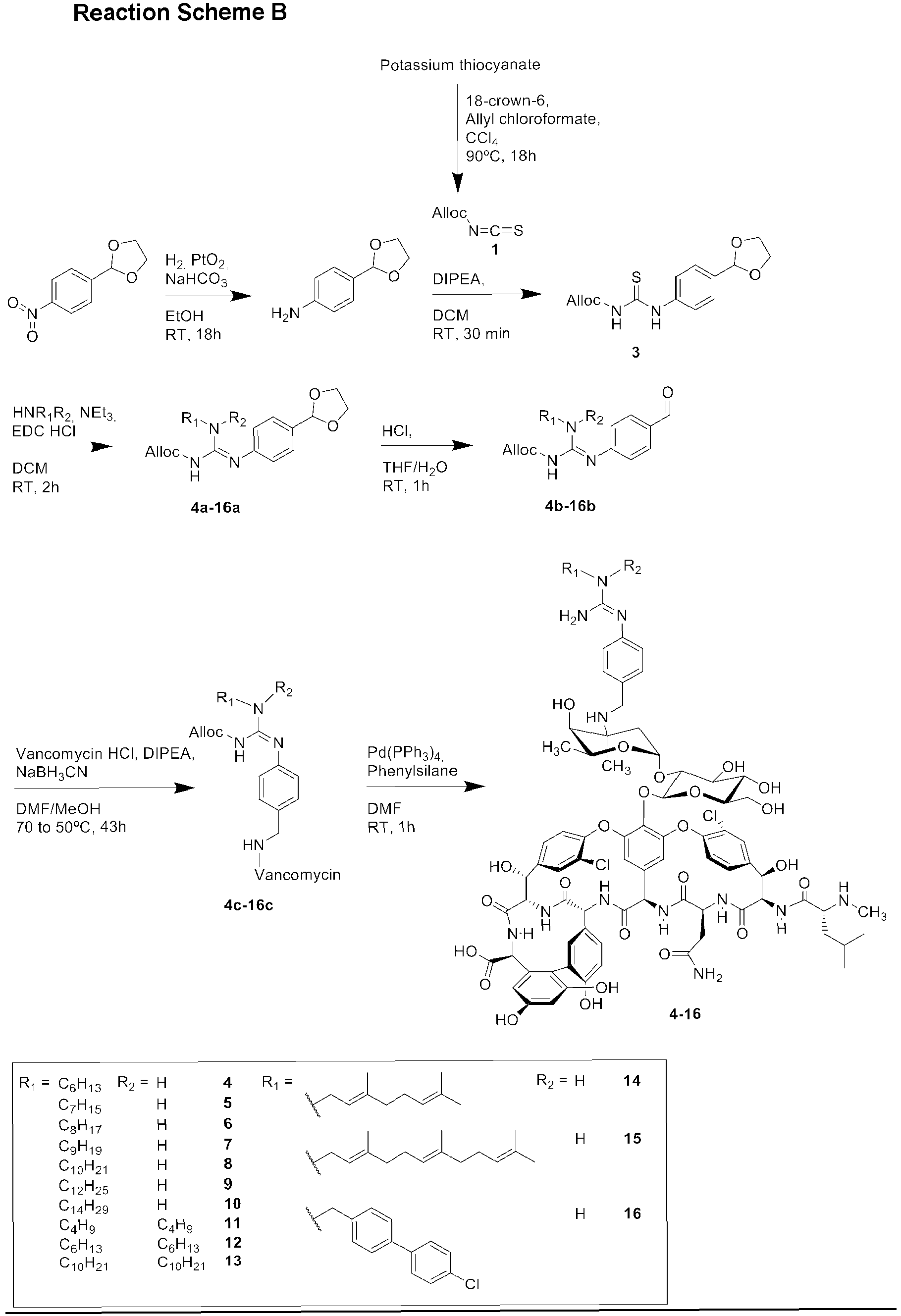

### ASSAYS

Compounds of the invention can be assessed for biological activity using any suitable assay that would be known to the person skilled in the art. Exemplary assays that are useful for the assessment of compounds of the invention are provided in the following paragraphs.

### Minimum inhibitory concentration

The antibacterial activity of the compounds was tested against a panel of bacteria, including Gram-positive bacteria (*S*. *aureus* ATCC29213, *S*. *aureus* USA300, *S*. *aureus* LIM-2, NR-45881, *S*. *aureus* HIP13419, NR-46413, *E. faecium* E155, *E. faecium* E7314, *E*. *faecium* E980, *E. faecalis* E1246, *E. faecalis* E7406 and *S*. *pneumonia* 153). The antibacterial activity of the compounds was further tested against *S*. *aureus* NY-155, NR-46236, *S*. *aureus* HIP12864, NR-46074, *S*. *aureus* 880 (BR-VRSA), NR-49120, *E. faecalis* E1246, *E. faecalis* E7604, as well as some Gram-negative bacteria (*Escherichia coli* and *Klebsiella pneumoniae).* Minimum inhibitory concentrations (MICs) in µg/mL were performed according to the CLSI guidelines. From glycerol stocks, bacterial strains were cultured on blood agar plates and incubated at 37°C for 18h. A single colony was transferred to tryptic soy broth (TSB) with 0.002% polysorbate 80 (p80). For the *Enterococcus*, VISA and VRSA strains, the cultures were grown at 37°C until the optical density of the suspension reached a level equivalent to the 0.5 McFarland standard. The bacterial suspensions were diluted in TSB with 0.002% p80 to reach a bacterial cell density of 10⁶ CFU/mL. In case of the other (vancomycin-sensitive) *S. aureus* strains, the direct colony suspension method was used. For these strains, after transfer of the colonies to TSB with 0.002% p80, they were immediately diluted to 10⁶ CFU/mL, instead of incubated prior to this dilution. In the case of *S*. *pneumonia,* direct colony suspension was used by immediately suspending multiple colonies from fresh blood agar plates in TSB + 0.002% p80 to an OD₆₀₀ of 0.5 and subsequent dilution to 10⁶ CFU mL⁻¹ in TSB + 0.002% p80 + 5% lysed horse blood. Antibiotic dilutions for this strains were also made in TSB + 0.002% p80 + 5% lysed horse blood. Both agar and microplates containing *S*. *pneumonia* were incubated at 37°C with 5% CO₂ for 24h with constant shaking (600 rpm). In the case of VRSA strains, 6 µg mL⁻¹ vancomycin was supplemented to the media. The cultures were grown to exponential phase (OD₆₀₀ = 0.5) at 37°C. The bacterial suspensions were diluted in TSB with 0.002% p80 (for VRSA no vancomycin was supplemented to the media form here on) to reach a bacterial cell density of 10⁶ CFU mL ⁻¹. In polypropylene 96-well microtiter plates, test compounds were added in biological triplicates and serial diluted 2-fold by transfer and mixing from one well to the next to achieve a final volume of 50 µL per well. An equal volume of bacterial suspension (10⁶ CFU mL⁻¹) was added to the wells. The plates were sealed with breathable membranes and incubated at 37°C for 24h with constant shaking (600 rpm). In polypropylene microtiter plates, 100 µL of the test compounds was added to the first row in biological triplicates, and 50 µL of media was added to the other wells. The compounds were subjected to serial dilution by transfer and mixing of 50 µL from one well to the next. Subsequently, 50 µL of bacterial suspension (10⁶ CFU/mL) was added to the wells. The plates were sealed with breathable membranes and incubated at 37°C for 24h with constant shaking (600 rpm). Positive growth control consisted of wells with bacterial suspension and without antibiotic. Negative growth control consisted of solely media without bacterial suspension or antibiotic. The MICs were determined from the median of a minimum of triplicates. MIC in the presence of serum followed the same protocol but using TSB + 0.002% p80 + 50% serum as growth media.

### Hemolysis

Whole defibrinated sheep blood (4 mL) was centrifuged for 15 minutes at 4°C at 400 g. The top layer was discarded and the bottom layer was washed with phosphate buffered saline (PBS) and centrifuged for 15 minutes at 4°C at 400 g. Washing cycles were repeated three times. Polypropylene microtiter plates were prepared by adding 150 µL of compounds in PBS with 0.002% p80 (256 µg/mL, containing ≤1% DMSO) to the first row in biological triplicates. PBS with 0.002% p80 (75 µL) was added to all other wells. The compounds were serially diluted by transfer and mixing of 75 µL from one well to the next well. The packed blood cells were diluted 25x in PBS with 0.002% p80 and 75 µL of this solution was added to all wells. The final concentrations of test compound in the plate ranged from 2-128 µg/mL. Plates were incubated for 18h at 37°C with continuous shaking (500 rpm). After incubation, plates were centrifuged for 5 min at 800 g and 25 µL of supernatant was transferred to a UV-star flat bottom polystyrene plate. 100 µL of MQ-H₂O was added to all wells of the UV-star plate and absorption was measured at 415 nm. Positive control wells (100% hemolysis) consisted of 0.1% Triton X-100 with blood cells. Negative control wells (no hemolysis) consisted of 1% DMSO with blood cells. In a separate experiment, the optimal wavelength for the hemolysis assay was determined by doing a full scan. In another separate experiment, linear detection range was determined, based on which the 25 times blood cell dilution was chosen in the main experiment.

### UDP-MurNAc-pentapeptide accumulation

An overnight culture of bacterial suspension (S. aureus ATCC29213, E. faecium E155, E. faecium E7314 and E. faecium E980) was diluted 100-fold in TSB supplemented with 0.002% p80 and cultured at 37°C until the optical density of the suspension reached a level equivalent to the 0.5 McFarland standard. Chloramphenicol was added at a final concentration of 130 µg/mL and the culture was incubated for an additional 15 min at 37°C. Next, the culture was split in 5 mL cultures and test antibiotics (vancomycin, 5, 6, 7, 14, 16) were added at a final concentration of 5 µM). Vancomycin (5 µM) was used as positive control and untreated sample was used as negative control. The cultures were incubated at 37°C for 1h, after which they were centrifuged to pellet the bacteria. The supernatant was removed and the pellets were resuspended in 1 mL MQ-H2O. The samples were boiled at 100°C for 15 minutes and subsequently centrifuged at 12000 rpm for 30 minutes. The supernatant of the samples was lyophilized and redissolved in 250 µL buffer A (50 mM ammonium bicarbonate, 5 mM NEt3, pH 8.3). Samples were analyzed by analytical RP-HPLC using a 0-25% buffer B (MeOH) gradient over 25 minutes.

### Lipid II antagonization assay

Lipid II in chloroform was added in the appropriate amount (5-fold molar excess compared to test antibiotics) to a 96-well plate and the chloroform was allowed to evaporate. Test antibiotics (vancomycin, telavancin, 5, 6, 7, 14, 16) were dissolved at a high stock concentration of 12.8 mg/mL in DMSO, after which they were diluted using TSB supplemented with 0.002% p80 to a concentration of 16xMIC (containing no more that 1% DMSO max). Of these dilutions, 50 µL was mixed with the 5-fold molar excess of pure lipid II in triplicates in the plate as well as added to the plate in triplicates without lipid II present. S. aureus ATCC29123 colonies were suspended in TSB with 0.002% p80 by direct colony suspension to an optical density that reached a level equivalent to the 0.5 McFarlan standard. The bacterial suspension was diluted in TSB with 0.002% p80 to reach 106 CFU/mL and 50 µL was added to the test compounds in the microplate to achieve a final concentration of 8xMIC for the test compounds. The samples were incubated at 37 °C for 24h with constant shaking (600 rpm) and inspected for visible bacterial growth. Positive growth control consisted of wells with bacterial suspension and without antibiotic or lipid II. Negative growth control consisted of solely media without bacterial suspension, antibiotic or lipid II.

### Resistance development serial passage assay

From glycerol stocks, bacterial strains were cultured on blood agar plates and incubated overnight at 37°C. A single colony was grown to exponential phase (OD₆₀₀ = 0.5) in TSB + 0.002% p80 and diluted 1:100 in fresh media. In polypropylene 96-well microtiter plates, antibiotics were added in biological triplicates and serial diluted 2-fold by transfer and mixing from one well to the next to achieve a final volume of 50 µL per well. An equal volume of bacterial suspension was added to the wells and plates were incubated overnight at 37°C. Bacterial cultures corresponding to 0.25xMIC were diluted 100-fold in fresh media and added (50 µL per well) to a newly prepared antibiotic dilution series (50 µL per well) followed by overnight incubation at 37°C. This procedure was repeated for 30 days and the MIC was recorded daily. Cultures containing daptomycin were supplemented with 50 mg L⁻¹ CaCl₂ and 10 mg L⁻¹ MgSO₄. The experiment was performed in biological replicates and for each replicate the MIC was determined from the median of a minimum of triplicates.

### Time Kill Assay

From glycerol stocks, bacterial strains were cultured on blood agar plates and incubated overnight at 37°C. Subsequently, a single colony was cultured in TSB + 0.002% p80 overnight at 37°C. The culture was diluted 100-fold in fresh media and grown until early exponential phase (OD₆₀₀ = 0.2-0.4) followed by dilution in media to OD₆₀₀ = 0.0025. The culture was split in separate culture tubes containing 2 mL. Antibiotics were added to the cultures (at concentration indicated in the experiments results) and incubated at 37°C for a total of 24 hours. At indicated time points (t=0, t=1, t=2, t=4, t=8 and t=24 h) 100 µL of each culture was transferred to eppendorfs and centrifuged for 5 min (10,000 rpm). Supernatant was removed and the cell pellets were resuspended in an equal volume of 0.9% NaCl in water (filter-sterilized). Samples were serial diluted with a 10-fold factor in filter-sterilized 0.9% NaCl in water. Of these serial dilutions the 100-fold, 1,000-fold, 10,000-fold and 100,000-fold dilution were plated out on blood agar plates (20 µL) in duplicates, subsequently allowed to evaporate and incubated at 37°C for 24 h. The colonies were counted and used to calculate the CFU mL⁻¹ remaining in the original culture by taking the dilution factors into account. Experiment was performed in biological duplicates.

### Cytotoxicity assay for HepG2

Mammalian cytotoxicity assays were performed by Cyprotex. Cytotoxicity was assessed using the MTT assay. Briefly, HepG2 human hepatocellular carcinoma cells (100 µL per well) were plated on black-walled clear-bottomed polystyrene 96-well tissue culture treated plates 24h prior to dosing the cells. The cells were dosed with test compound (dissolved in 0.5% DMSO in growth media containing 10% FBS) at a range of concentrations (0.04 µM, 0.1 µM, 0.4 µM, 1 µM, 4 µM, 10 µM, 40 µM, 100 µM). After 23 h period the cells were loaded with the MTT dye (yellow; 3-(4,5-dimethyl-2-thiazolyl)-2,5-diphenyl-2H-tetrazolium bromide) followed by an additional 1h incubation (resulting in a total incubation time with antibiotics of 24 h). Subsequently, the plates are dried and solubilized in DMSO followed by scanning at 570 nm using a microplate absorbance reader. Cell viability is determined by conversion of the soluble MTT to insoluble formazan (purple) by mitochondrial hydrogenase in live cells. Production of formazan indicates loss of mitochondrial function and cell loss. Carbonyl cyanide 3-chlorophenylhydrazone is used as no response control and chlorpromazine is used as control known to be cytotoxic. Solvent control wells (0.5% DMSO in growth media containing 10% FBS) are then used to determine significance limits for wells that have a greater than expected fraction of low or high responders. The minimum effective concentration is determined from the lowest concentration whose mean value exceeds the significance level, provided either a clear dose-response relationship is observed, or at least two consecutive concentration points are above the significance level. AC50 values are also determined provided a clear dose-response relationship is observed. Experiment was performed in triplicates.

### In vivo studies

Mouse studies were performed using pathogen-free CD1 mice (ICR), a well characterized outbred murine strain (supplied by Charles River, Margate UK). All mice were male and weighed 11-15 gram upon arrival.and were allowed to acclimatize for at least 7 days prior to study initiataion. Mice were individually housed in sterilized ventilated cages supplied with HEPA filtered sterile air and aspen chip bedding (changed a minimum of once a week). Food and water was available ad libitum. The room temperature was 22°C ± 1°C, with a 60% relative humidity and a maximum background noise of 56 dB. Mice were exposed to a 12h light/dark cycle.

### Tolerability

Tolerability of compound 5 was assessed at 100 mg kg⁻¹ subcutaneous injection (in 10% DMSO in water for injection) in naive mice (n=2) that were not immunosuppressed or infected.

### PK

Test article **5** was administered at 3 mg kg⁻¹ subcutaneously in naive mice (n=3) that were not immunosuppressed or infected. Whole blood was collected from the tail vein at 5 min, 15 min, 30 min, 1 h, 2 h, 4 h and 8 h. The last sample was taken as a terminal cardiac puncture.

### Efficacy

Mice were made neutropenic by a subcutaneous injection of 150 mg kg⁻¹ and 100 mg kg⁻¹ cyclophosphamide 4 days and 1 day before infection respectively. Mice were infected in both thighs with MRSA USA300 strain NRS384 (1.47 x 10⁶ CFU mL⁻¹, 7.33 × 10⁴ CFU per thigh) intramuscularly under temporary anesthesia. Mice were treated with vehicle (10% DMSO in water for injection) every 6 hours, 25 mg kg⁻¹ vancomycin (in water for injection) every 12 hours, 3 mg kg⁻¹ or 10 mg kg⁻¹ compound **5** (in 10% DMSO in water for injection) every 6 hours, starting from 1 hour after infection. Pretreatment group was euthanized one hour after infection and the other treatment groups were euthanized 23 hours after infection. Weighted thighs were homogenized and cultured on MSA agar at 37°C for 18-24h followed by colony counting. Each group consisted of n=6 mice with both thighs infected (n = 12 thighs per treatment group) and each thigh was treated as a separate sample. Data analysis was done with StatsDirect software v. 3.2.8 using non-parametric statistical models (Kruskal-Wallis using Conover-Inman to make all pairwise comparisons between groups).

### EXAMPLES

Thin layer chromatography (TLC) was performed on SiliaPlate TLC plates (SiliCycle, glass-backed, silica, 250 µm). Visualization was done using UV light, ninhydrin stain, permanganate stain or cerium ammonium molybdate stain. Silica gel column chromatography was performed using SiliaFlash^{®} P60 silica gel (SiliCycle). The final compounds were purified by preparative reverse phase high performance liquid chromatography (RP-HPLC) using a Reprosil Gold 120 C18 10 µm column (Length: 250 mm, ID: 25 mm. Lot No: 8768. part No: r10.9g.s2525. Serial No: 18020211570. Dr Maisch GmbH) with BESTA pumps, FLASH 10 DAD UV detector and SCPA PrepCon 5 software. Analytical HPLC to asses compound purity was performed using a Phemomenex Jupiter su C18 300A column (250x4, 60mm, 5micron) on a Shimadzu LC-2030 Plus instrument. All spectra displayed to show purity were recorded at 214 nm. Buffers used for preparative and analytical HPLC were 50 mM ammonium acetate as buffer A and 95% MeCN + 5% H₂O as buffer B in all cases, except for in the uridine diphosphate N-acetylmuramic acid pentapeptide (UDP-MurNAc-pp) accumulation assay. In that assay, analysis was done using the Phemomenex Jupiter su C18 300A column (250x4, 60mm, 5micron) on a Shimadzu LC-2030 Plus instrument. The buffers were 50 mM ammonium bicarbonate, 5 mM NEt₃, pH 8.3 as buffer A and MeOH as buffer B, and the displayed data were recorded at 254 nm. Samples were lyophilized on a VirTis BenchTop Pro with Omnitronics. Nuclear magnetic resonance (NMR) spectra were obtained from a Bruker DPX-300, super conducting magnet with a field strength of 7.0 Tesla, equipped with 5 mm BBO, Broadband Observe probe head, high resolution with Z- Gradient, and a 5 mm ¹⁹F / ¹H dual high resolution probe. High resolution mass spectroscopy (HR-MS) analyses were performed on a Thermo Scientific Dionex UltiMate 3000 HPLC system with a Phenomenex Kinetex C18 column (2.1 × 150 mm, 2.6 µm) at 35 °C and equipped with a diode array detector. The following solvent system, at a flow rate of 0.3 mL/min, was used: solvent A, 0.1 % formic acid in water; solvent B, 0.1% formic acid in acetonitrile. Gradient elution was as follows: 95:5 (A/B) for 1 min, 95:5 to 5:95 (A/B) over 9 min, 5:95 to 2:98 (A/B) over 1 min, 2:98 (A/B) for 1 min, then reversion back to 95:5 (A/B) over 2 min, 95:5 (A/B) for 1 min. This system was connected to a Bruker micrOTOF-Q II mass spectrometer (electrospray ionization) calibrated internally with sodium formate. Tecan Spark was used for absorbance measurements.

Bacterial strains used were Staphylococcus aureus ATCC 29213 (MSSA, Rosenbach strain), Staphylococcus aureus USA300 (MRSA, hospitalized patient, Texas, USA. PFGE type: USA300. SCCmec type Iva), Enterococcus faecium E155 (VRE, hospitalized patient, Chicago, USA, VanA gene, MIC vancomycin 1024, year 1995), Enterococcus faecium E980 (VSE, human community isolate (commensal), no VanA of VanB gene. year 1998), Enterococcus faecium E7314 (VRE, hospitalized patient, NLD, VanB gene), Enterococcus faecalis E1246 (VRE, VanA gene, clinical isolate), and Enterococcus faecalis E7406 (VRE, VanB gene, clinical isolate).

### Example 1: Synthesis of compound 1: O-allyl carbonisothiocyanatidate

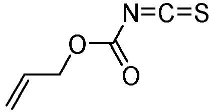

Compound 1 was synthesized according to a published literature procedure (Martin, N. I., Woodward, J. J. & Marletta, M. A. NG-Hydroxyguanidines from Primary Amines. Org. Lett. 8, 4035-4038 (2006)). In short, to a solution of potassium thiocyanate (9 g, 92 mmol, 1.3 eq) in CCl4 (200 mL), 18-crown-6 (924 mg, 3.5 mmol, 0.05 eq) and allyl chloroformate (7.4 mL, 70 mmol, 1 eq) were added and the reaction mixture was refluxed at 90 °C for 18h. After incubation, the reaction mixture was diluted with PE (200 mL) and left stirring on ice for 1h. Subsequently, the mixture was filtered through celite and washed with DCM. The filtrate was concentrated under reduced pressure, redissolved in DCM to an estimated concentration of 0.5M and stored at 4 °C until further use. 1 was used crude in the next reaction.

### Example 2: Synthesis of compound 2: 4-(1,3-dioxolan-2-yl)aniline

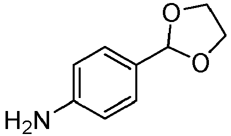

Compound 2 was synthesized following a modified literature procedure (Hughes, A. et al. Diamide compounds having muscarinic receptor antagonist and β2 andrenergic receptor agonist activity (2010)). To a solution of 2-(4-Nitrophenyl)-1,3-dioxolane (9.8 g, 50 mmol, 1 eq) and NaHCO3 (4.3 g, 50 mmol, 1 eq) in EtOH (350 mL) under nitrogen atmosphere, platinum(iv) oxide monohydrate (1.2 g, 5 mmol, 0.1 eq) was added. The reaction mixture was sparged with hydrogen for 15 min and then stirred under hydrogen atmosphere for 18h at RT. Next, the solution was filtered through celite and washed with MeOH. The filtrate was concentrated under reduced pressure to give 2, which was used crude and immediately in the next step.

### Example 3: Synthesis of compound 3

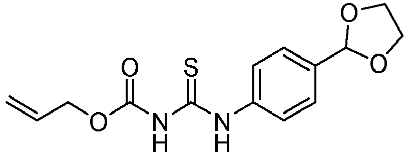

To a crude solution of 2 (50 mmol, 1 eq) and DIPEA (8.7 mL, 50 mmol, 1 eq) in DCM (50 mL), 1 was added at RT until TLC (in DCM with 5% EtOAc) confirmed complete conversion of 2 to 3. Crude product was concentrated under reduced pressure and purified by silica gel column chromatography (DCM with increasing gradient up to 5% EtOAc). Yield over 2 steps; 77%. 1H-NMR (300 MHz, CDCl3) δ/ppm: 11.47 (s, 1H), 8.42 (s, 1H), 7.67 (d, J = 8.5 Hz, 2H), 7.51 (d, J = 8.5, 2H), 6.00 - 5.85 (m, 1H), 5.83 (s, 1H), 5.39 (dd, J = 17.2, 1.4 Hz, 1H), 5.33 (dd, J = 10.4, 1.2 Hz, 1H), 4.70 (dt, J = 5.8, 1.3 Hz, 2H), 4.18 - 3.98 (m, 4H). 13C-NMR (75 MHz, CDCl3) δ/ppm: 177.75, 152.65, 138.30, 136.59, 130.84, 127.15, 124.10, 119.86, 103.25, 67.37, 65.40. HR-MS: m/z 309.0913 (observed), 309.0909 (calculated for [M+H+]).

### Example 4: Synthesis of trans,trans-farnesyl bromide: (E)-1-bromo-3,7-dimethylocta-2,6-diene

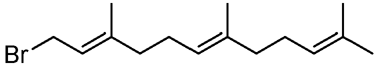

(E)-1-bromo-3,7-dimethylocta-2,6-diene was synthesized according to a modified literature procedure (Zahn, T. J. et al. Evaluation of Isoprenoid Conformation in Solution and in the Active Site of Protein-Farnesyl Transferase Using Carbon-13 Labeling in Conjunction with Solution- and Solid-State NMR. J. Am. Chem. Soc. 122, 7153-7164 (2000); Xie, H., Shao, Y., Becker, J. M., Naider, F. & Gibbs, R. A. Synthesis and Biological Evaluation of the Geometric Farnesylated Analogues of the a-Factor Mating Peptide of Saccharomyces cerevisiae. J. Org. Chem. 65, 8552-8563 (2000)). In short, to a solution of trans,trans-farnesol (4.5 mL, 18 mmol, 1 eq) in DCM under argon atmosphere, triphenylphosphene (4 mL,18 mmol, 1 eq) and tetrabromomethane (7.4 g, 22.5 mmol, 1.25 eq) were added. The reaction was stirred at RT for 4h. After the solvent was removed under reduced pressure, hexane (15 mL) was added to the residue to precipitate byproduct. The mixture was centrifuged (4500 rpm, 5 min) and the supernatant was concentrated under reduced pressure. Three additional precipitation-centrifugation-evaporation cycles were performed after which crude trans,trans-farnesyl bromide was obtained and directly used in the next step.

### Example 5: Synthesis of trans-geranyl amine and trans,trans-farnesyl amine: (E)-3,7-dimethylocta-2,6-dien-1-amine and (2E,6E)-3,7,11-trimethyldodeca-2,6,10-trien-1-amine

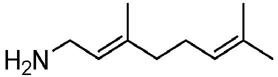

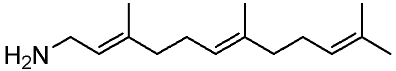

(E)-3,7-dimethylocta-2,6-dien-1-amine and (2E,6E)-3,7,11-trimethyldodeca-2,6,10-trien-1-amine were synthesized following a modified literature procedure (Koopmans, T. et al. Semisynthetic Lipopeptides Derived from Nisin Display Antibacterial Activity and Lipid II Binding on Par with That of the Parent Compound. J. Am. Chem. Soc. 137, 9382-9389 (2015); Coppola, G. M. & Prashad, M. A Convenient Preparation of Farnesylamine. Synth. Commun. 23, 535-541 (1993)). In short, to lithium bis(trimethylsilyl)amide (10 mL, 1M in THF) under argon atmosphere, pure trans-geranyl bromide or crude trans,trans-farnesyl bromide (1 eq) was added respectively. The reaction was stirred at RT for 18h and subsequently quenched with saturated ammonium chloride solution. The mixture was extracted twice with methyl t-butyl ether and the organic phases were combined and dried over Na2SO4. After filtration, the solvent was removed under reduced pressure to yield the 2xTMS amine. This product was dissolved in 40 mL of MeOH and 5 mL of DCM, and stirred at RT for 18h. Solvent was removed under reduced pressure to yield crude trans-geranyl amine or trans,trans-farnesyl amine, which was immediately used in the next step.

### Example 6: Synthesis of chlorobisphenylamine: 4'-chloro-[1,1'-biphenyl]-4-yl)methanamine

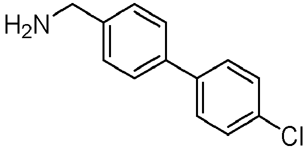

4'-chloro-[1,1'-biphenyl]-4-yl)methanamine was made according to a published literature procedure (Lee, H. et al. (Biphenyl-4-yl)methylammonium Chlorides: Potent Anticonvulsants That Modulate Na+ Currents. J. Med. Chem. 56, 5931-5939 (2013)). To a solution of 4-bromobenzylamine (4.32 g, 23 mmol, 1 eq) in MeCN (250 mL) under argon, 4-chlorophenylboronicacid (4 g, 26 mmol, 1.1 eq), Pd(PPh3)4 (1.36 g, 1.16 mmol, 0.05 mmol) and 2M aqueous K2CO3 (58 mL) were added. After sparging the solution with argon for 30 min, the reaction was stirred under reflux at 90 °C for 16h. The solvent was removed under reduced pressure. The resulting residue was dissolved in EtOAc (100 mL) and washed with water (2x 100 mL) and brine (2x 100 mL). The organic layer was dried with Na2SO4, filtered and concentrated under reduced pressure. The residue was redissolved in EtOAc, and concentrated aqueous HCl (2 mL) was added which resulted in precipitation. To the EtOAc, H2O was added and the layers were separated. The pH of the aqueous layer was adjusted with 4N NaOH and extracted with DCM (2x). The DCM layers were combined, dried over Na2SO4, filtered and concentrated under reduced pressure. To the resulting residue, which was redissolved in DCM, 4N HCl in dioxane was added to cause precipitation. The precipitate was filtered and washed with hexanes to yield 4'-chloro-[1,1'-biphenyl]-4-yl)methanamine as final product, which was used crude in the next reaction step.

### Example 7: Synthesis of 4a

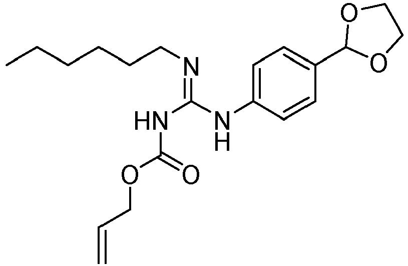

To a solution of 3 (1.80 g, 5.8 mmol, 1 eq) in DCM, hexylamine (1.5 mL, 11.7 mmol, 2 eq) and NEt3 (1.6 mL, 11.7 mmol, 2 eq) were added. Subsequently EDC HCl (2.2 g, 11.7 mmol, 2 eq) was added and the reaction mixture was stirred at RT. After 2h, the reaction was complete and the solution was concentrated under reduced pressure. The product was purified by silica gel column chromatography (DCM + 5% EtOAc). Yield; 100%. 1H-NMR (300 MHz, CDCl3) δ/ppm: 10.70 (s, 1H), 7.53 (d, J = 8.0 Hz, 2H), 7.22 (d, J = 6.8 Hz, 2H), 6.13 - 5.91 (m, 1H), 5.78 (s, 1H), 5.34 (d, J = 17.3 Hz, 1H), 5.21 (d, J = 10.4 Hz, 1H), 4.62 (d, J = 5.6 Hz, 2H), 4.21 - 3.99 (m, 4H), 3.43 - 3.29 (m, 2H), 1.57-1.38 (m, 2H), 1.36-1.16 (m, 6H), 0.94 - 0.78 (m, 3H). 13C-NMR (75 MHz, CDCl3) δ/ppm: 164.21, 158.64, 137.02, 136.61, 133.67, 128.33, 117.42, 103.14, 66.02, 65.48, 41.37, 31.45, 29.40, 26.51, 22.54, 14.02. HR-MS: m/z 376.2242 (observed), 376.2236 (calculated for [M+H+])

### Example 8: Synthesis of 5a-16a

Compounds **5a-16a** were synthesized and purified according to the above described protocol for **4a** using the corresponding lipid amines, with the exception of **16a** which after reaction resulted in a product mixture of **16a** and **16b. 16a** and **16b** were used combined and crude in the next step. The results for these compounds are summarized in Table 1.

**Table 1: Compounds 5a-16a**

| | **Structure** | **Yield** | **HR-MS** | **NMR** |
|---|---|---|---|---|
| **5a** | 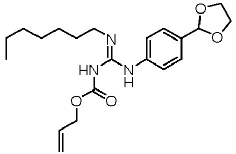 | quantitative | *m*/*z* 390.2402 (observed), 390.2393 (calculated for [M+H⁺]) | ¹H-NMR (300 MHz, CDCl₃) δ/ppm: 10.70 (s, 1H), 7.53 (d, J = 8.0 Hz, 2H), 7.23 (d, J = 7.7 Hz, 2H), 6.14 - 5.93 (m, 1H), 5.79 (s, 1H), 5.34 (dd, J = 17.2, 1.3 Hz, 1H), 5.21 (dd, J = 10.4, 1.2 Hz, 1H), 4.62 (d, J = 5.7 Hz, 2H), 4.22 - 4.00 (m, 4H), 3.42 - 3.29 (m, 2H), 1.57 1.38 (m, 2H), 1.34 - 1.18 (m, 8H), 0.92 - 0.80 (m, 3H).¹³C-NMR (75 MHz, CDCl₃) δ/ppm: 164.27, 158.69, 137.10, 136.57, 133.77, 128.40, 117.45, 103.18, 66.06, 65.54, 41.43, 31.77, 29.50, 29.00, 26.85, 22.65, 14.15. |
| **6a** | 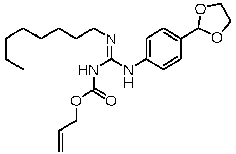 | quantitative | *m*/*z* | ¹H-NMR (300 MHz, CDCl₃) δ/ppm: 10.70 (s, 1H), 7.53 (d, J = 8.0 Hz, 2H), 7.22 (d, J = 7.4 Hz, 2H), 6.12 - 5.93 (m, 1H), 5.79 (s, 1H), 5.34 (dd, J = 17.2, 1.5 Hz, 1H), 5.21 (dd, J = 10.4, 1.4 Hz, 1H), 4.62 (d, J = 5.7 Hz, 2H), 4.22 - 3.99 (m, 4H), 3.43 - 3.28 (m, 2H), 1.59 - 1.37 (m, 2H), 1.34 - 1.15 (m, 10H), 0.93 - 0.81 (m, 3H).¹³C-NMR (75 MHz, CDCl₃) δ/ppm: 164.23, 158.67, 137.09, 136.55, 133.74, 128.37, 125.51, 117.43, 103.15, 66.04, 65.51, 41.41, 31.82, 29.47, 29.27, 29.21, 26.87, 22.69, 14.16. |
| | | | 404.2547 (observed), 404.2549 (calculated for [M+H⁺]) | |
| **7a** | 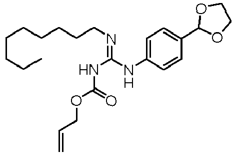 | quantitative | *m*/*z* 418.2716 (observed), 418.2706 (calculated for [M+H⁺]) | ¹H-NMR (300 MHz, CDCl₃) δ/ppm: 10.71 (s, 1H), 7.53 (d, J = 7.8 Hz, 2H), 7.22 (d, J = 7.2 Hz, 2H), 6.15 - 5.93 (m, 1H), 5.78 (s, 1H), 5.34 (d, J = 17.2 Hz, 1H), 5.21 (d, J = 10.4 Hz, 1H), 4.61 (d, J = 5.5 Hz, 2H), 4.22 - 3.97 (m, 4H), 3.44 - 3.26 (m, 2H), 1.57 - 1.38 (m, 2H), 1.38-1.13 (m, 12H), 0.96 - 0.79 (m, 3H). ¹³C-NMR (75 MHz, CDCl₃) δ/ppm: 164.14, 158.58, 137.01, 136.45, 133.67, 128.26, 125.38, 117.32, 103.06, 65.93, 65.41, 41.31, 31.81, 29.41, 29.38, 29.23, 29.18, 26.78, 22.63, 14.10. |
| **8a** | 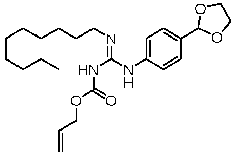 | quantitative | *m*/*z* 432.2871 (observed), 432.2862 (calculated for [M+H⁺]) | ¹H-NMR (300 MHz, CDCl₃) δ/ppm: 10.70 (s, 1H), 7.53 (d, J = 7.6 Hz, 2H), 7.22 (d, J = 7.1 Hz, 2H), 6.11 - 5.92 (m, 1H), 5.79 (s, 1H), 5.34 (d, J = 17.2 Hz, 1H), 5.21 (d, J = 10.4 Hz, 1H), 4.62 (d, J = 5.3 Hz, 2H), 4.22 - 3.99 (m, 4H), 3.43 - 3.28 (m, 2H), 1.56 - 1.38 (m, 2H), 1.36-1.14 (m, 14H), 0.92 - 0.82 (m, 3H). ¹³C-NMR (75 MHz, CDCl₃) δ/ppm: 164.23, 158.66, 137.09, 136.54, 133.74, 128.37, 117.43, 103.15, 66.03, 65.51, 41.41, 31.94, 29.56, 29.48, 29.35, 29.32, 26.88, 22.74, 14.19. |
| **9a** | 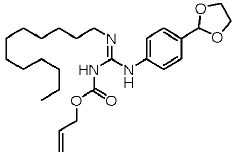 | quantitative | *m*/*z* 460.3184 (observed), 460.3175 (calculated for [M+H⁺]) | ¹H-NMR (300 MHz, CDCl₃) δ/ppm: 10.70 (s, 1H), 7.53 (d, J = 7.8 Hz, 2H), 7.22 (d, J = 7.4 Hz, 2H), 6.17 - 5.91 (m, 1H), 5.78 (s, 1H), 5.34 (d, J = 17.2 Hz, 1H), 5.21 (d, J = 10.4 Hz, 1H), 4.62 (d, J = 5.5 Hz, 2H), 4.21 - 3.99 (m, 4H), 3.41 - 3.29 (m, 2H), 1.57 - 1.37 (m, 2H), 1.37 - 1.13 (m, 18H), 0.94 - 0.79 (m, 3H). ¹³C-NMR (75 MHz, CDCl₃) δ/ppm: 164.22, 158.64, 137.08, 136.56, 133.72, 128.34, 125.48, 117.39, 103.12, 66.00, 65.48, 41.38, 31.95, 29.67, 29.60, 29.53, 29.45, 29.39, 29.30, 26.85, 22.73, 14.17. |
| **10a** | 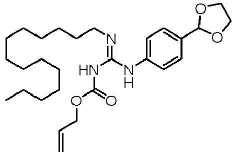 | quantitative | *m*/*z* 488.3491 (observed), 488.3488 (calculated for [M+H⁺]) | ¹H-NMR (300 MHz, CDCl₃) δ/ppm: 10.71 (s, 1H), 7.53 (d, J = 7.9 Hz, 2H), 7.22 (d, J = 7.1 Hz, 2H), 6.11 - 5.93 (m, 1H), 5.78 (s, 1H), 5.34 (dd, J = 17.2, 1.2 Hz, 1H), 5.21 (dd, J = 10.4, 1.0 Hz, 1H), 4.61 (d, J = 5.6 Hz, 2H), 4.22 - 3.98 (m, 4H), 3.45 - 3.26 (m, 2H), 1.59 - 1.38 (m, 2H), 1.38 - 1.13 (m, 22H), 0.99 - 0.78 (m, 3H). ¹³C-NMR (75 MHz, CDCl₃) δ/ppm: 164.16, 158.61, 137.06, 136.50, 133.69, 128.30, 125.45, 117.37, 103.11, 65.98, 65.44, 41.36, 31.94, 29.70, 29.66, 29.58, 29.51, 29.43, 29.38, 29.28, 26.83, 22.71, 14.15. |
| **11a** | 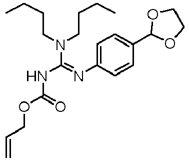 | 81% | *m*/*z* 404.2564 (observed), 404.2549 (calculated for [M+H⁺]) | ¹H-NMR (300 MHz, CDCl₃) δ/ppm: δ 10.31 (s, 1H), 7.42 (d, J = 8.5 Hz, 2H), 7.01 (d, J = 8.4 Hz, 2H), 6.10 - 5.89 (m, 1H), 5.75 (s, 1H), 5.32 (dd, J = 17.2, 1.5 Hz, 1H), 5.19 (dd, J = 10.4, 1.3 Hz, 1H), 4.58 (dt, J = 5.8, 1.3 Hz, 2H), 4.21 - 3.97 (m, 4H), 3.26 (t, J = 7.4 Hz, 4H), 1.61 - 1.39 (m, 4H), 1.24 (h, J = 7.4 Hz, 4H), 0.87 (t, J = 7.3 Hz, 6H). ¹³C-NMR (75 MHz, CDCl₃) δ/ppm: 163.73, 160.90, 141.21, 133.74, 133.61, 127.87, 121.12, 117.56, 103.46, 66.25, 65.41, 48.06, 29.76, 20.12, 13.90. |
| **12a** | 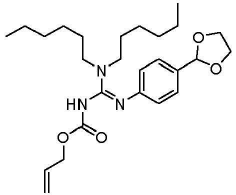 | 52% | *m*/*z* 460.3200 (observed), 460.3175 (calculated for [M+H⁺]) | ¹H-NMR (300 MHz, CDCl₃) δ/ppm: 10.31 (s, 1H), 7.42 (d, J = 8.4 Hz, 2H), 7.01 (d, J = 8.3 Hz, 2H), 6.09 - 5.92 (m, 1H), 5.76 (s, 1H), 5.32 (dd, J = 17.2, 1.5 Hz, 1H), 5.20 (dd, J = 10.4, 1.4 Hz, 1H), 4.58 (dt, J = 5.8, 1.3 Hz, 2H), 4.21 - 3.97 (m, 4H), 3.35 - 3.16 (t, J = 7.4 Hz, 4H), 1.59 - 1.41 (m, 4H), 1.37 - 1.11 (m, 12H), 0.91 - 0.83 (m, 6H).¹³C-NMR (75 MHz, CDCl₃) δ/ppm: 160.93, 141.23, 133.79, 133.67, 127.89, 121.16, 117.57, 103.50, 66.28, 65.45, 48.37, 31.62, 27.64, 26.61, 22.66, 14.13. |
| **13a** | 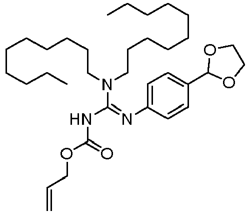 | quantitative | *m*/*z* 572.4429 (observed), 572.4427 (calculated for [M+H⁺]) | ¹H-NMR (300 MHz, CDCl₃) δ/ppm: 10.29 (s, 1H), 7.41 (d, J = 8.4 Hz, 2H), 7.01 (d, J = 8.2 Hz, 2H), 6.11 - 5.89 (m, 1H), 5.74 (s, 1H), 5.30 (dd, J = 17.2, 1.4 Hz, 1H), 5.17 (dd, J = 10.4, 1.3 Hz, 1H), 4.57 (d, J = 5.7 Hz, 2H), 4.18 - 3.92 (m, 4H), 3.26 (t, J = 7.2 Hz, 4H), 1.61 - 1.41 (m, 4H), 1.39 - 1.12 (m, 28H), 0.95 - 0.80 (m, 6H).¹³C-NMR (75 MHz, CDCl₃) δ/ppm: 163.40, 160.56, 140.91, 133.56, 133.40, 127.58, 120.81, 117.17, 103.17, 65.93, 65.11, 48.09, 31.73, 29.36, 29.14, 27.39, 26.64, 22.53, 13.97. |
| **14a** | 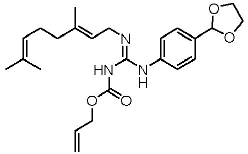 | quantitative | *m*/*z* 428.2556 (observed), 428.2549 (calculated for [M+H⁺]) | ¹H-NMR (300 MHz, CDCl₃) δ/ppm: 10.72 (s, 1H), 7.52 (d, J = 8.2 Hz, 2H), 7.21 (d, J = 8.0 Hz, 2H), 6.11 - 5.92 (m, 1H), 5.78 (s, 1H), 5.34 (dd, J = 17.2, 1.5 Hz, 1H), 5.21 (dd, J = 10.4, 1.4 Hz, 1H), 5.15 (t, J = 5.8 Hz, 1H), 5.09 - 4.98 (m, 1H), 4.62 (d, J = 5.8 Hz, 2H), 4.20 - 4.00 (m, 4H), 4.02 - 3.91 (m, 2H), 2.12-1.91 (m, 4H), 1.65 (s, 3H), 1.63 (s, 3H), 1.58 (s, 3H). ¹³C-NMR (75 MHz, CDCl₃) δ/ppm: 140.53, 133.52, 131.79, 128.26, 125.27, 123.74, 119.41, 117.49, 103.13, 66.02, 65.45, 39.45, 26.27, 25.70, 17.72, 16.40. |
| **15a** | 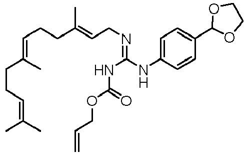 | 75% | *m*/*z* 496.3178 (observed), 496.3175 (calculated for [M+H⁺]) | ¹H-NMR (300 MHz, CDCl₃) δ/ppm: 10.71 (s, 1H), 7.52 (d, J = 8.1 Hz, 2H), 7.23 (d, J = 7.4 Hz, 2H), 6.12 - 5.92 (m, 1H), 5.78 (s, 1H), 5.34 (dd, J = 17.2, 1.3 Hz, 1H), 5.22 (dd, J = 10.4, 1.3 Hz, 1H), 5.18 - 5.01 (m, 3H), 4.62 (d, J = 5.5 Hz, 2H), 4.19 - 4.02 (m, 4H), 4.02 - 3.92 (m, 2H), 2.14 - 1.89 (m, 8H), 1.67 (s, 3H), 1.64 (s, 3H), 1.59 (s, 3H), 1.57 (s, 3H).¹³C-NMR (75 MHz, CDCl₃) δ/ppm: 164.20, 158.45, 140.75, 137.12, 135.50, 133.73, 131.41, 128.36, 125.40, 124.91, 124.38, 123.72, 119.40, 117.47, 103.19, 66.07, 65.54, 39.77, 39.56, 39.50, 26.79, 26.35, 25.80, 17.80, 16.53, 16.11. |
| **16a** | 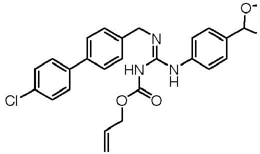 | Used crude | n\a | n\a |

### Example 9: Synthesis of 17a and 18a

Compounds **17a** and **18a** were synthesized and purified according to the above described protocol for **4a** using the corresponding lipid amines. The results for these compounds are summarized in Table 2.

**Table 2: Compounds 17a and 18a**

| | **Structure** | **Yield** | **HR-MS** | **NMR** |
|---|---|---|---|---|
| **17a** | 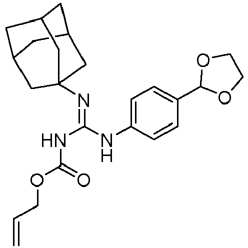 | 91% | *m*/*z* 426.2396 (observed), 426.2393 (calculated for [M+H⁺]) | ¹H-NMR (300 MHz, CDCl₃) δ/ppm: 10.64 (s, 1H), 7.51 (d, J = 8.0 Hz, 2H), 7.21 (d, J = 7.9 Hz, 2H), 6.14 - 5.92 (m, 1H), 5.78 (s, 1H), 5.34 (d, J = 17.3 Hz, 1H), 5.21 (dd, J = 10.4, 1.2 Hz, 1H), 4.80 (s, 1H), 4.65 (d, J = 5.1 Hz, 2H), 4.21 - 3.96 (m, 4H), 2.20 - 1.95 (m, 9H), 1.74 - 1.57 (m, 6H). |
| | | | | ¹³C-NMR (75 MHz, CDCl₃) δ/ppm: 157.73, 137.62, 134.12, 128.37, 125.06, 117.01, 103.26, 66.46, 65.55, 42.25, 36.40, 29.65 |
| **18a** | 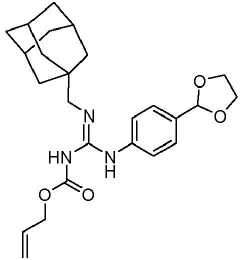 | 91% | *m*/*z* 440.2541 (observed), 4440.2549 (calculated for [M+H⁺]) | ¹H-NMR (300 MHz, CDCl₃) δ/ppm: 10.77 (s, 1H), 7.55 (d, J = 7.4 Hz, 2H), 7.25 (d, J = 7.1 Hz, 2H), 6.13 - 5.90 (m, 1H), 5.80 (s, 1H), 5.34 (dd, J = 17.2, 1.5 Hz, 1H), 5.21 (dd, J = 10.4, 1.3 Hz, 1H), 4.87 (s, 1H), 4.62 (d, J = 5.7 Hz, 2H), 4.21 - 4.00 (m, 4H), 3.10 (d, J = 5.4 Hz, 2H), 2.04 - 1.87 (m, 3H), 1.78 - 1.33 (m, 12H). |
| | | | | ¹³C-NMR (75 MHz, CDCl₃) δ/ppm: 164.39, 159.09, 140.63, 133.82, 128.41, 125.30, 117.43, 103.21, 66.07, 65.51, 52.66, 40.36, 36.95, 33.71, 28.20. |

### Example 10: Synthesis of 4b

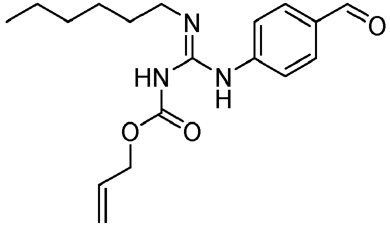

To a solution of **4a** (2.19 g, 5.8 mmol, 1 eq) in THF, 1M aqueous HCl (11.7 mmol, 2 eq) was added and the reaction mixture was stirred at RT for 1h. The reaction was quenched with saturated NaHCO₃, and product was extracted with DCM twice. Organic layers were combined, dried over Na₂SO₄ and filtered. The crude product was concentrated under reduced pressure and purified by silica gel column chromatography (2/1 PE/EtOAc). Yield; 100%. ¹H-NMR (300 MHz, CDCl₃) δ/ppm: 9.86 (s, 1H), 7.82 (d, J = 7.9 Hz, 2H), 7.28 (d, J = 7.9 Hz, 2H), 6.07 - 5.86 (m, 1H), 5.33 (dd, J = 17.2, 1.4 Hz, 1H), 5.23 (dd, J = 10.4, 1.3 Hz, 1H), 4.61 (d, J = 5.7 Hz, 2H), 3.47 - 3.30 (m, 2H), 1.67 - 1.52 (m, 2H), 1.43 - 1.22 (m, 6H), 0.94-0.82 (m, 3H).¹³C-NMR (75 MHz, CDCl₃) δ/ppm: 190.84, 132.51, 131.41, 123.21, 118.14, 66.27, 41.51, 31.35, 29.08, 26.50, 22.45, 13.94. HR-MS: m/z 332.1980 (observed), 332.1974 (calculated for [M+H+]).

### Example 11: Synthesis of 5b-16b

Compounds **5b-16b** were synthesized and purified according to the above described protocol for **4b** using their corresponding precursor (**5a-16a**). The results for these compounds are summarized in Table 3.

**Table 3: Compounds 5b-16b**

| | **Structure** | **Yield** | **HR-MS** | **NMR** | |
|---|---|---|---|---|---|
| **5b** | 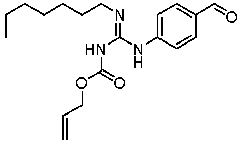 | quantitat ive | *m*/*z* | ¹H-NMR (300 MHz, CDCl₃) | |
| | | | 346.2138 (observed), 346.2130 (calculated for [M+H⁺]) | | δ/ppm: 9.89 (s, 1H), 7.85 (d, J = 8.0 Hz, 2H), 7.26 (d, J = 7.0 Hz, 2H), 6.05 - 5.86 (m, 1H), 5.33 (dd, J = 17.2, 1.4 Hz, 1H), 5.24 (dd, J = 10.4, 1.3 Hz, 1H), 4.61 (d, J = 5.8 Hz, 2H), 3.48 - 3.29 (m, 2H), 1.67 - 1.53 (m, 2H), 1.41 - 1.21 (m, 8H), 0.93 - 0.83 (m, 3H).¹³C-NMR (75 MHz, CDCl₃) δ/ppm: 190.91, 131.59, 123.37, 118.35, 66.41, 41.56, 31.73, 29.23, 28.96, 26.90, 22.61, 14.10. |
| **6b** | 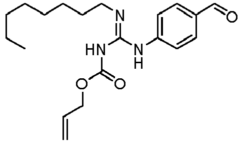 | 98% | *m*/*z* | ¹H-NMR (300 MHz, CDCl₃) | |
| | | | 360.2286 (observed), 360.2287 (calculated for [M+H⁺]) | | δ/ppm: 9.90 (s, 1H), 7.86 (d, J = 8.0 Hz, 2H), 7.25 (d, J = 6.3 Hz, 2H), 6.10 - 5.85 (m, 1H), 5.33 (dd, J = 17.2, 1.4 Hz, 1H), 5.24 (dd, J = 10.4, 1.2 Hz, 1H), 4.61 (d, J = 5.8 Hz, 2H), 3.46 - 3.32 (m, 2H), 1.67 - 1.50 (m, 2H), 1.41 - 1.22 (m, 10H), 0.92 - 0.82 (m, 3H). ¹³C-NMR (75 MHz, CDCl₃) δ/ppm: 190.95, 131.64, 123.43, 118.40, 66.45, 41.60, 31.83, 29.28, 29.22, 26.97, 22.69, 14.16. |
| **7b** | 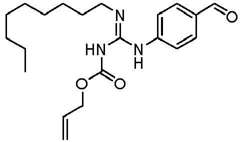 | 71% | *m*/*z* | ¹H-NMR (300 MHz, CDCl₃) | |
| | | | 374.2450 (observed), 374.2443 (calculated for [M+H⁺]) | | δ/ppm: 9.90 (s, 1H), 7.85 (d, J = 7.8 Hz, 2H), 7.25 (d, J = 6.2 Hz, 2H), 6.05 - 5.86 (m, 1H), 5.33 (dd, J = 17.2, 1.4 Hz, 1H), 5.24 (dd, J = 10.4, 1.3 Hz, 1H), 4.61 (d, J = 5.8 Hz, 2H), 3.47 - 3.28 (m, 2H), 1.68 - 1.51 (m, 2H), 1.41 - 1.18 (m, 12H), 0.92 - 0.81 (m, 3H). ¹³C-NMR (75 MHz, CDCl₃) δ/ppm: 190.92, 131.63, 123.41, 118.36, 66.44, 41.57, 31.89, 29.51, 29.32, 29.28, 26.96, 22.71, 14.16. |
| **8b** | 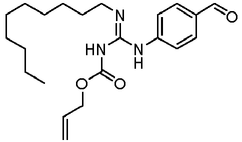 | 95% | *m*/*z* | ¹H-NMR (300 MHz, CDCl₃) | |
| | | | 388.2610 (observed), 388.2600 (calculated for [M+H⁺]) | | δ/ppm: 9.88 (s, 1H), 7.84 (d, J = 8.0 Hz, 2H), 7.26 (d, J = 7.2 Hz, 2H), 6.06 - 5.87 (m, 1H), 5.33 (dd, J = 17.2, 1.4 Hz, 1H), 5.23 (dd, J = 10.4, 1.2 Hz, 1H), 4.61 (d, J = 5.8 Hz, 2H), 3.45 - 3.29 (m, 2H), 1.69 - 1.52 (m, 2H), 1.42 - 1.19 (m, 14H), 0.93 - 0.83 (m, 3H).¹³C-NMR (75 MHz, CDCl₃) δ/ppm: 190.87, 131.56, 123.36, 118.26, 66.39, 41.55, 31.90, 29.53, 29.31, 29.29, 29.21, 26.93, 22.69, 14.14. |
| **9b** | 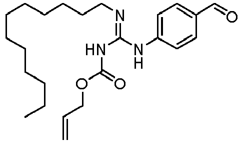 | 98% | *m*/*z* | ¹H-NMR (300 MHz, CDCl₃) | |
| | | | 416.2920 (observed), 416.2913 (calculated for [M+H⁺]) | | δ/ppm: 9.88 (s, 1H), 7.84 (d, J = 8.0 Hz, 2H), 7.26 (d, J = 7.2 Hz, 2H), 6.06 - 5.87 (m, 1H), 5.33 (dd, J = 17.2, 1.4 Hz, 1H), 5.23 (dd, J = 10.4, 1.2 Hz, 1H), 4.61 (d, J = 5.8 Hz, 2H), 3.46 - 3.27 (m, 2H), 1.69 - 1.53 (m, 2H), 1.42 - 1.17 (m, 18H), 0.94 - 0.81 (m, 3H). ¹³C-NMR (75 MHz, CDCl₃) δ/ppm: 190.86, 131.55, 123.34, 118.33, 66.39, 41.55, 31.92, 29.65, 29.59, 29.53, 29.36, 29.29, 29.21, 26.94, 22.70, 14.15. |
| **10b** | 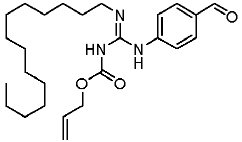 | quantitat ive | *m*/*z* | ¹H-NMR (300 MHz, CDCl₃) | |
| | | | 444.3232 (observed), 444.3226 (calculated for [M+H⁺]) | | δ/ppm: 9.87 (s, 1H), 7.83 (d, J = 8.1 Hz, 2H), 7.26 (d, J = 7.7 Hz, 2H), 6.05 - 5.83 (m, 1H), 5.33 (dd, J = 17.2, 1.5 Hz, 1H), 5.23 (dd, J = 10.4, 1.3 Hz, 1H), 4.61 (d, J = 5.8 Hz, 2H), 3.46 - 3.31 (m, 2H), 1.68 - 1.53 (m, 2H), 1.41 - 1.18 (m, 22H), 0.94 - 0.81 (m, 3H). ¹³C-NMR (75 MHz, CDCl₃) δ/ppm: 190.81, 131.49, 123.29, 118.27, 66.34, 41.54, 31.91, 29.64, 29.57, 29.50, 29.35, 29.27, 29.19, 26.91, 22.68, 14.12. |
| **11b** | 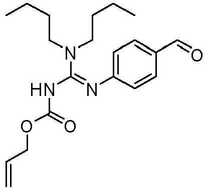 | 94% | *m*/*z* | ¹H-NMR (300 MHz, CDCl₃) | |
| | | | 360.2294 (observed), 360.2287 (calculated for [M+H⁺]) | | δ/ppm: 9.90 (s, 1H), 7.82 (d, J = 8.6 Hz, 2H), 7.07 (d, J = 8.4 Hz, 2H), 6.05 - 5.87 (m, 1H), 5.31 (dd, J = 17.2, 1.5 Hz, 1H), 5.20 (dd, J = 10.4, 1.4 Hz, 1H), 4.56 (dt, J = 5.8, 1.3 Hz, 2H), 3.34 (t, J = 7.5 Hz, 4H), 1.65-1.49 (m, 4H), 1.29 (h, J = 7.4 Hz, 4H), 0.90 (t, J = 7.3 Hz, 6H). ¹³C-NMR (75 MHz, CDCl₃) |
| | | | | | δ/ppm: 190.82, 133.11, 131.64, 131.51, 119.86, 117.86, 66.43, 48.26, 29.82, 20.12, 13.84. |
| **12b** | 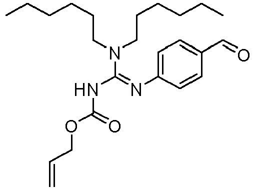 | 88% | *m*/*z* | ¹H-NMR (300 MHz, CDCl₃) | |
| | | | 416.2932 (observed), 416.2913 (calculated for [M+H⁺]) | | δ/ppm: δ 9.90 (s, 1H), 7.82 (d, J = 8.6 Hz, 2H), 7.07 (d, J = 8.4 Hz, 2H), 6.04 - 5.88 (m, 1H), 5.31 (dd, J = 17.2, 1.5 Hz, 1H), 5.20 (dd, J = 10.4, 1.4 Hz, 1H), 4.56 (dt, J = 5.8, 1.3 Hz, 2H), 3.41 - 3.23 (m, 4H), 3.33 (t, J = 7.5 Hz, 4H), 1.35 - 1.18 (m, 12H), 0.94 - 0.79 (m, 6H). ¹³C-NMR (75 MHz, CDCl₃) |
| | | | | | δ/ppm: 190.82, 133.13, 131.69, 131.52, 119.90, 117.86, 66.44, 48.55, 31.51, 27.68, 26.57, 22.59, 14.07. |
| **13b** | 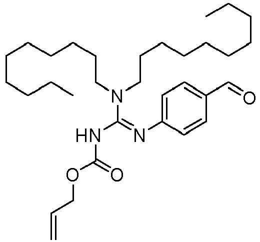 | 80% | *m*/*z* | ¹H-NMR (300 MHz, CDCl₃) | |
| | | | 528.4174 (observed), 528.4165 (calculated for [M+H⁺]) | | δ/ppm: 10.22 (s, 1H), 9.90 (s, 1H), 7.82 (d, J = 8.4 Hz, 2H), 7.07 (d, J = 8.0 Hz, 2H), 6.05 - 5.86 (m, 1H), 5.31 (dd, J = 17.2, 1.0 Hz, 1H), 5.20 (dd, J = 10.4, 0.9 Hz, 1H), 4.56 (d, J = 5.7 Hz, 2H), 3.32 (t, J = 7.4 Hz, 4H), 1.68 - 1.47 (m, 4H), 1.38 - 1.15 (m, 28H), 0.97 - 0.79 (m, 6H). ¹³C-NMR (75 MHz, CDCl₃) |
| | | | | | δ/ppm: 190.76, 133.14, 131.68, 131.50, 119.71, 117.85, 66.44, 48.54, 31.94, 29.58, 29.35, 27.72, 26.91, 22.74, 14.19. |
| **14b** | 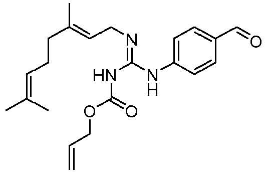 | 77% | *m*/*z* | ¹H-NMR (300 MHz, CDCl₃) | |
| | | | 384.2296 (observed), 384.2287 (calculated for [M+H⁺]) | | δ/ppm: 9.92 (s, 1H), 7.86 (d, J = 8.3 Hz, 2H), 7.28 (d, J = 7.1 Hz, 2H), 6.06 - 5.87 (m, 1H), 5.40 - 5.19 (m, 3H), 5.10 - 5.01 (m, 1H), 4.61 (d, J = 5.8 Hz, 2H), 4.06 - 3.90 (m, 2H), 2.17 - 1.96 (m, 4H), 1.70 (s, 3H), 1.66 (s, 3H), 1.59 (s, 3H). ¹³C-NMR (75 MHz, CDCl₃) δ/ppm: 190.96, 140.97, 132.03, 131.65, 123.68, 123.16, 119.40, 66.45, 39.64, 39.54, 26.36, 25.76, 17.80, 16.52. |
| **15b** | 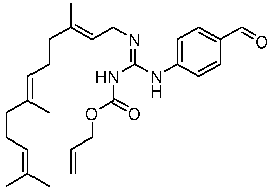 | 40% | *m*/*z* | ¹H-NMR (300 MHz, CDCl₃) | |
| | | | 452.2923 (observed), 452.2913 (calculated for [M+H⁺]) | | δ/ppm: 9.93 (s, 1H), 7.87 (d, J = 8.0 Hz, 2H), 7.35 - 7.19 (m, 2H), 6.05 - 5.87 (m, 1H), 5.39 - 5.20 (m, 3H), 5.14 - 5.02 (m, 2H), 4.61 (d, J = 5.7 Hz, 2H), 4.05 - 3.93 (m, 2H), 2.18 - 1.91 (m, 8H), 1.71 (s, 3H), 1.67 (s, 3H), 1.59 (s, 6H). ¹³C-NMR (75 MHz, CDCl₃) δ/ppm: 190.96, 141.11, 135.70, 131.70, 131.48, 124.37, 123.60, 123.20, 119.39, 66.51, 39.79, 39.68, 39.60, 26.81, 26.36, 25.83, 17.82, 16.60, 16.15. |
| **16b** | 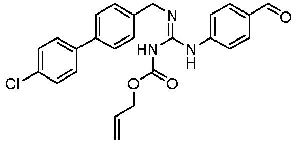 | 65% | *m*/*z* | ¹H-NMR (300 MHz, CDCl₃) | |
| | | (over 2 steps) | 448.1445 (observed), 448.1428 (calculated for [M+H⁺]) | | δ/ppm: 9.88 (s, 1H), 7.84 (d, J = 8.3 Hz, 2H), 7.59 - 7.45 (m, 4H), 7.45 - 7.34 (m, 4H), 7.19 (s, 2H), 6.05 - 5.84 (m, 1H), 5.34 (dd, J = 17.2, 1.2 Hz, 1H), 5.25 (dd, J = 10.4, 1.2 Hz, 1H), 4.68 - 4.58 (m, 4H). ¹³C-NMR (75 MHz, CDCl₃) |
| | | | | | δ/ppm: 190.94, 139.49, 139.12, 137.20, 133.61, 131.75, 129.06, 128.37, 128.33, 127.45, 123.61, 66.70, 45.05. |

### Example 12: Synthesis of 17b and 18b

Compounds **17b and** were synthesized and purified according to the above described protocol for **4b** using their corresponding precursor (**17a** and **18a**). The results for these compounds are summarized in Table 4.

**Table 4: Compounds 17b and 18b**

| | **Structure** | **Yield** | **HR-MS** | **NMR** |
|---|---|---|---|---|
| **17b** | 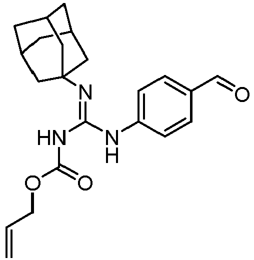 | 62% | *m*/*z* 382.2120 (observed), 382.2131 (calculated for [M+H⁺]) | ¹H-NMR (300 MHz, CDCl₃) δ/ppm: 9.92 (s, 1H), 7.85 (d, J = 8.2 Hz, 2H), 7.11 (s, 2H), 6.05 - 5.79 (m, 1H), 5.32 (dd, J = 17.2, 1.3 Hz, 1H), 5.25 (dd, J = 10.4, 1.2 Hz, 1H), 4.60 (d, J = 5.6 Hz, 2H), 2.17 - 2.05 (m, 9H), 1.75 - 1.62 (m, 6H). |
| | | | | ¹³C-NMR (75 MHz, CDCl₃) δ/ppm: 191.11, 131.84, 123.22, 66.67, 41.97, 36.49, 29.65 |
| **18b** | 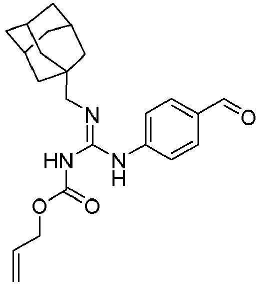 | 86% | *m*/*z* 396.2271 (observed), 396.2287 (calculated for [M+H⁺]) | ¹H-NMR (300 MHz, CDCl₃) δ/ppm: 9.93 (s, 1H), 7.87 (d, J = 8.7 Hz, 2H), 7.24 (s, 2H), 6.09 - 5.80 (m, 1H), 5.34 (dd, J = 17.2, 1.5 Hz, 1H), 5.25 (dd, J = 10.4, 1.2 Hz, 1H), 4.62 (dt, J = 5.8, 1.3 Hz, 2H), 3.21 - 2.97 (m, 2H), 2.09 - 1.91 (m, 3H), 1.81 - 1.39 (m, 12H). |
| | | | | ¹³C-NMR (75 MHz, CDCl₃) δ/ppm: 191.01, 131.73, 123.25, 66.55, 53.23, 40.48, 36.96, 33.66, 28.27 |

### Example 13: Synthesis of 4c

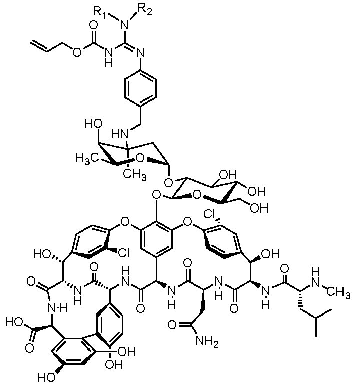

| | | | | | | | | | |
|---|---|---|---|---|---|---|---|---|---|
| R₁ = | C₆H₁₃ | R₂ = | H | **4c** | R₁ = | 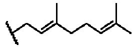 | R₂ = | H | **14c** |
| | C₇H₁₅ | | H | **5c** | | | | | |
| | C₈H₁₇ | | H | **6c** | | 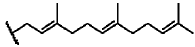 | | | |
| | C₉H₁₉ | | H | **7c** | | | | H | **15c** |
| | C₁₀H₂₁ | | H | **8c** | | | | | |
| | C₁₂H₂₅ | | H | **9c** | | | | | |
| | C₁₄H₂₉ | | H | **10c** | | 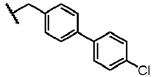 | | H | **16c** |
| | C₄H₉ | | C₄H₉ | **11c** | | | | | |
| | C₆H₁₃ | | C₆H₁₃ | **12c** | | | | | |
| | C₁₀H₂₁ | | C₁₀H₂₁ | **13c** | | | | | |

To a solution of **4b** (178 mg, 538 µmol, 2 eq) in DMF/MeOH (4 mL), vancomycin hydrochloride (400 mg, 269 µmol, 1 eq) and DIPEA (0.23 mL, 1.35 mmol, 5 eq) were added. After the reaction was stirred under reflux conditions at 70 °C for 2h, NaBH₃CN (169 mg, 2.69 mmol, 10 eq) was added and the reaction temperature was reduced to 50 °C. After 5h, an additional 10 eq of NaBH₃CN (169 mg, 2.69 mmol) was added and 18h after that 10 eq NaBH₃CN (169 mg, 2.69 mmol) and 1 eq of **4b** (89 mg, 269 µmol) was added. After an additional 18h, the reaction mixture was quenched by adding water. Solvents were evaporated under reduced pressure, and the residue was redissolved in DMF and precipitated twice in cold diethyl ether. The precipitate was dried and used crude in the next step.

### Example 14: Synthesis of 5c-16c

Compounds **5c-16c** were synthesized according to the above described protocol for **4c** using their corresponding aldehyde precursor (**5b-16b**). Compounds **5c-16c** were all used crude in the next reaction step.

### Example 15: Synthesis of 17c and 18c

Compounds **17c** and **18c** were synthesized according to the above described protocol for **4c** using their corresponding aldehyde precursor (**17b** and **18b**). Compounds **17c** and **18c** were all used crude in the next reaction step.

### Example 16: Synthesis of 4

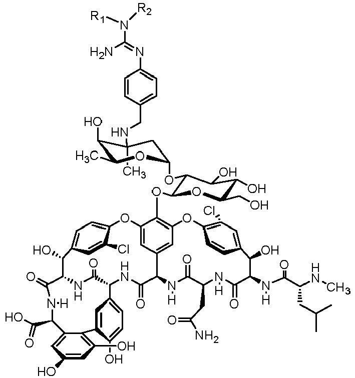

| | | | | | | | | | |
|---|---|---|---|---|---|---|---|---|---|
| R₁ = | C₆H₁₃ | R₂ = | H | **4** | R₁ = | 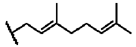 | R₂ = H | | **14** |
| | C₇H₁₅ | | H | **5** | | | | | |
| | C₈H₁₇ | | H | **6** | | | | | |
| | C₉H₁₉ | | H | **7** | | 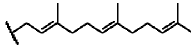 | | H | **15** |
| | C₁₀H₂₁ | | H | **8** | | | | | |
| | C₁₂H₂₅ | | H | **9** | | | | | |
| | C₁₄H₂₉ | | H | **10** | | 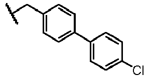 | | H | **16** |
| | C₄H₉ | | C₄H₉ | **11** | | | | | |
| | C₆H₁₃ | | C₆H₁₃ | **12** | | | | | |
| | C₁₀H₂₁ | | C₁₀H₂₁ | **13** | | | | | |

To a solution of crude **4c** (269 µmol, 1 eq) in dry DMF (5 mL) under argon atmosphere, Pd(PPh₃)₄ (78 mg, 67 µmol, 0.25 eq) and phenylsilane (0.83 mL, 6.7 mmol, 25 eq) were added. The reaction mixture was stirred for 1h at RT under argon atmosphere. After complete deprotection, the reaction was quenched with water and the solvents were evaporated under reduced pressure. The residue was redissolved a mixture of buffer A (50 mM ammonium acetate) with 20% buffer B (95% MeCN, 5% water) and centrifuged to remove all solid residue. The supernatant was applied to preparative RP-HPLC and the product was purified using a 20-55% buffer B gradient over 50 minutes. Purity of the fractions was assessed on analytical RP-HPLC using a 0-100% buffer B gradient over 30 minutes. Pure fractions were pooled an lyophilized to obatain a white powder. Purity of the pooled final compound was assesed on analytical RP-HPLC using a 0-100% buffer B gradient over 60 minutes. Yield over 2 steps; 54.4%. HR-MS: *m*/*z* 840.3100 (observed), 840.3097 (calculated for [M+2H⁺]/2). Retention time RP-HPLC analysis; 20.37 min.

### Example 17: Synthesis of 5-16

Compounds **5-16** were synthesized according to the above described protocol for **4** using their corresponding precursor (**5c-16c**). The preparative RP-HPLC purification buffer gradient was adjusted for each compound based on the hydrophobicity of the R group present. Furthermore, percentage of buffer B present in the initial solvent system (from 20% up to 50%) for the preparative RP-HPLC was adjusted based on the hydrophobicity of the R group as well. The results for these compounds are indicated in Table 5.

**Table 5: Compounds 5a-16a**

| | **Yield (over two steps)** | **HR-MS** | **Retention time** |
|---|---|---|---|
| **5** | 43% | *m*/*z* 847.3168 (observed), 847.3175 (calculated for [M+2H⁺]/2) | 22.29 min |
| **6** | 5% | *m*/*z* 854.3256 (observed), 854.3253 (calculated for [M+2H⁺]/2). | 24.19 min |
| **7** | 74% | *m*/*z* 861.3334 (observed), 861.3332 (calculated for [M+2H⁺]/2) | 26.00 min |
| **8** | 13% | *m*/*z* 868.3418 (observed), 868.3410 (calculated for [M+2H⁺]/2) | 27.76 min |
| **9** | 16% | *m*/*z* 882.3573 (observed), 882.3566 (calculated for [M+2H⁺]/2) | 31.20 min |
| **10** | 5% | *m*/*z* 896.3725 (observed), 896.3723 (calculated for [M+2H⁺]/2) | 34.74 min |
| **11** | 33% | *m*/*z* 854.3265 (observed), 854.3254 (calculated for [M+2H⁺]/2) | 21.80 min |
| **12** | 34% | *m*/*z* 882.3561 (observed), 882.3567 (calculated for [M+2H⁺]/2) | 28.89 min |
| **13** | 9% | *m*/*z* 938.4197 (observed), 938.4192 (calculated for [M+2H⁺]/2) | 43.00 min |
| **14** | 34% | *m*/*z* 866.3254 (observed), 866.3253 (calculated for [M+2H⁺]/2) | 24.38 min |
| **15** | 11% | *m*/*z* 900.3564 (observed), 900.3566 (calculated for [M+2H⁺]/2) | 30.56 min |
| **16** | 15% | *m*/*z* 898.2828 (observed), 898.2824 (calculated for [M+2H⁺]/2) | 25.43 min |

### Example 18: Synthesis of 17 and 18

Compounds **17** and **18** were synthesized according to the above described protocol for **4** using their corresponding precursor (**17c** and **18c**). The preparative RP-HPLC purification buffer gradient was adjusted for each compound based on the hydrophobicity of the R group present. Furthermore, percentage of buffer B present in the initial solvent system (from 20% up to 50%) for the preparative RP-HPLC was adjusted based on the hydrophobicity of the R group as well. The results for these compounds are indicated in Table 6.

**Table 6: Compounds 17a and 18a**

| | **Yield (over two steps)** | **HR-MS** | **Retention time** |
|---|---|---|---|
| **17** | 9% | *m*/*z* 1729.6245 (observed), 1729.6272 (calculated for [M+H⁺]) | 21.10 min |
| **18** | 9% | *m*/*z* 1743.6435 (observed), 1743.6429 (calculated for [M+H⁺]) | 22.96 min |

### Example 19: Minimum inhibitory concentration assay

The activity of the compounds was assessed against a panel of Gram-positive bacteria in an MIC assay. In this assay the minimum inhibitory concentration, meaning the lowest tested concentration at which visible growth of bacteria is prevented, is determined and compared to clinically used antibiotics. Compared to the clinically used glycopeptides, almost all compounds have equipotent or superior activity against most of the tested MRSA, MSSA, VRE, VSE, VISA, VRSA, and *S*. *pneumoniae* strains. In some cases (depending on the compound and bacteria tested) there is more than a 1000-fold improvement in activity compared to vancomycin (the data obtained is summarized in Table 7, with the MIC values indicated in units of µg/mL). Further MIC data is provided in Tables 8-12.

**Table 7: MIC results of the clinically available glycopeptide antibiotics and 4-18 against a panel of Gram-positive bacteria.**

| | ***S*. *aureus*** | | ***E. faecium*** | | | ***E.faecalis*** | |
|---|---|---|---|---|---|---|---|
| | ATCC29213 | USA300 | E155 | E980 | E7314 | E1246 | E7406 |
| | *MSSA* | *MRSA* | *VRE (VanA)* | *VSE* | *VRE (VanB)* | *VRE (VanA)* | *VRE (VanB)* |
| **Vancomycin** | 1 | 1 | >128 | 0.5 | 128 | >128 | 16 |
| **Telavancin** | 0.125 | 0.125 | 4 | 0.015625 | ≤0.0078125 | 4 | 0.0625 |
| **Teicoplanin** | 0.5 | 0.5 | >128 | 0.5 | 0.25 | >128 | 0.125 |
| **Oritavancin** | 0.25 | 0.0625 | 0.5 | 0.0625 | 0.125 | 1 | 0.0625 |
| **Dalbavancin** | 0.25 | 0.25 | 128 | 0.0625 | 0.015625 | >128 | 0.03125 |
| **4** | 0.0625 | 0.0625 | 8 | 0.03125 | 0.03125 | 32 | 0.25 |
| **5** | ≤0.0078125 | 0.015625 | 2 | ≤0.0078125 | ≤0.0078125 | 16 | 0.015625 |
| **6** | ≤0.0078125 | ≤0.0078125 | 1 | 0.015625 | ≤0.0078125 | 8 | ≤0.0078125 |
| **7** | 0.015625 | ≤0.0078125 | 0.5 | 0.03125 | ≤0.0078125 | 2 | ≤0.0078125 |
| **8** | 0.015625 | 0.0625 | 0.25 | 0.015625 | ≤0.0078125 | 1 | ≤0.0078125 |
| **9** | 0.125 | 0.5 | 0.125 | 0.125 | 0.03125 | 1 | 0.0625 |
| **10** | 2 | 4 | 0.5 | 0.5 | 0.5 | 2 | 1 |
| **11** | 0.125 | 0.125 | 32 | 0.0625 | 0.5 | >128 | 8 |
| **12** | ≤0.0078125 | 0.0625 | 1 | ≤0.0078125 | ≤0.0078125 | 8 | ≤0.0078125 |
| **13** | 16 | 16 | 8 | 4 | 4 | 32 | 4 |
| **14** | ≤0.0078125 | 0.03125 | 1 | ≤0.0078125 | ≤0.0078125 | 8 | ≤0.0078125 |
| **15** | 0.03125 | 0.0625 | 0.125 | 0.03125 | 0.015625 | 1 | 0.03125 |
| **16** | 0.015625 | 0.03125 | 0.125 | ≤0.0078125 | ≤0.0078125 | 1 | ≤0.0078125 |
| **17** | 0.016 | 0.031 | 8 | ≤0.008 | 0.016 | 16 | 0.125 |
| **18** | ≤0.008 | ≤0.008 | 2 | ≤0.008 | ≤0.008 | 16 | ≤0.008 |

**Table 8: MIC results of the clinically available glycopeptide antibiotics and 4-18 against Vancomycin-intermediate S. aureus LIM-2, NR-45881, Vancomycin-resistant (VanA) S. aureus HIP13419, NR-46413, and S. pneumoniae 153.**

| | ***S*. *aureus*** | | ***S. pneumoniae*** |
|---|---|---|---|
| | LIM-2, NR-25881 | HIP13419, NR-46413 | 153 |
| | *VISA* | *VRSA* | *S. P*. |
| **Vancomycin** | 8 | >128 | 0.5 |
| **Teicoplanin** | 16 | 32 | 0.031 |
| **Telavancin** | 0.25 | 4 | ≤0.008 |
| **Dalbavancin** | 1 | 16 | ≤0.008 |
| **Oritavancin** | 1 | 0.25 | ≤0.008 |
| **4** | 0.25 | 4 | 0.016 |
| **5** | 0.031 | 1 | ≤0.008 |
| **6** | ≤0.008 | 0.5 | ≤0.008 |
| **7** | ≤0.008 | 0.125 | ≤0.008 |
| **8** | 0.016 | 0.063 | ≤0.008 |
| **9** | 0.5 | 0.125 | ≤0.008 |
| **10** | 4 | 0.5 | 0.031 |
| **11** | 0.5 | 16 | 0.063 |
| **12** | ≤0.008 | 0.25 | ≤0.008 |
| **13** | 32 | 8 | 2 |
| **14** | ≤0.008 | 0.063 | ≤0.008 |
| **15** | 0.125 | 0.125 | ≤0.008 |
| **16** | 0.016 | 0.063 | ≤0.008 |
| **17** | 0.125 | 8 | 0.016 |
| **18** | 0.016 | 2 | ≤0.008 |

**Table 9: Further MIC results of the clinically available glycopeptide antibiotics and 4-18 against a panel of Gram-positive bacteria.**

| | ***S*. *aureus*** | | | ***E. faecalis*** | |
|---|---|---|---|---|---|
| | NY-155, NR-46236 | HIP12864, NR-46074 | 880, NR-49120 | E1246 | E7604 |
| | *MRSA* | *VISA* | *VRSA* | *VRE (vanA)* | *VRE (vanB)* |
| **Vancomycin** | 2 | 8 | >128 | >128 | 16 |
| **Teicoplanin** | 0.5 | 4 | 16 | >128 | 0.125 |
| **Telavancin** | 0.031 | 0.25 | 8 | 4 | 0.063 |
| **Dalbavancin** | ≤0.008 | 0.5 | 2 | >128 | 0.031 |
| **Oritavancin** | 0.25 | 4 | 0.25 | 1 | 0.063 |
| **4** | 0.063 | 0.5 | 16 | 32 | 0.25 |
| **5** | ≤0.008 | 0.063 | 4 | 16 | 0.016 |
| **6** | ≤0.008 | ≤0.008 | 2 | 8 | ≤0.008 |
| **7** | ≤0.008 | ≤0.008 | 0.5 | 2 | ≤0.008 |
| **8** | ≤0.008 | 0.062 | 0.25 | 1 | ≤0.008 |
| **9** | 0.5 | 0.25 | 0.25 | 1 | 0.063 |
| **10** | 8 | 2 | 1 | 2 | 1 |
| **11** | 0.063 | 1 | 64 | >128 | 8 |
| **12** | ≤0.008 | 0.063 | 1 | 8 | ≤0.008 |
| **13** | 16 | 16 | 8 | 32 | 4 |
| **14** | ≤0.008 | 0.031 | 1 | 8 | ≤0.008 |
| **15** | 0.125 | 0.125 | 0.5 | 1 | 0.031 |
| **16** | ≤0.008 | 0.031 | 0.5 | 1 | ≤0.008 |
| **17** | 0.031 | ≤0.016 | 8 | 16 | 0.125 |
| **18** | ≤0.008 | ≤0.016 | 2 | 16 | ≤0.008 |

**Table 10: MIC results of the clinically available glycopeptide antibiotics and 5, 6, 7, 12, 14 and 16 against a panel of 31 vancomycin-resistant E. faecium strains.**

| **Strain** | | **Vancomycin** | **Telavancin** | **5** | **6** | **7** | **12** | **14** | **16** |
|---|---|---|---|---|---|---|---|---|---|
| E155 | VanA | >128 | 4 | 2 | 1 | 0.5 | 1 | 1 | 0.125 |
| E0013 | VanA | >128 | 4 | 4 | 1 | 0.5 | 2 | 2 | 0.25 |
| E0072 | VanA | >128 | 2 | 0.25 | 0.5 | 0.125 | 0.25 | 0.5 | ≤0.008 |
| E0300 | VanA | >128 | 8 | 4 | 2 | 0.5 | 1 | 4 | 0.25 |
| E0321 | VanA | >128 | 16 | 8 | 4 | 1 | 2 | 8 | 0.5 |
| E0333 | VanA | >128 | 8 | 8 | 4 | 1 | 2 | 8 | 0.5 |
| E0338 | VanA | >128 | 8 | 4 | 2 | 0.5 | 2 | 4 | 0.125 |
| E0341 | VanA | >128 | 8 | 8 | 4 | 1 | 2 | 4 | 0.25 |
| E0506 | VanA | >128 | 4 | 2 | 2 | 0.5 | 1 | 2 | 0.125 |
| E0745 | VanA | >128 | 4 | 1 | 0.25 | 0.125 | 0.25 | 1 | 0.031 |
| E1130 | VanA | >128 | ≤0.008 | ≤0.008 | ≤0.008 | ≤0.008 | ≤0.008 | ≤0.008 | ≤0.008 |
| E1441 | VanA | >128 | 8 | 4 | 2 | 1 | 2 | 4 | 0.5 |
| E1679 | VanA | >128 | 16 | 16 | 8 | 4 | 8 | 16 | 1 |
| E1763 | VanA | 128 | 1 | 0.25 | 0.031 | ≤0.008 | 0.031 | 0.125 | ≤0.008 |
| E2297 | VanA | >128 | 4 | 2 | 1 | 0.5 | 1 | 2 | 0.125 |
| E2359 | VanB | 128 | ≤0.008 | ≤0.008 | ≤0.008 | ≤0.008 | ≤0.008 | ≤0.008 | ≤0.008 |
| E2365 | VanB | 16 | ≤0.008 | ≤0.008 | ≤0.008 | ≤0.008 | ≤0.008 | ≤0.008 | ≤0.008 |
| E2373 | VanA | >128 | 8 | 4 | 2 | 0.5 | 1 | 4 | 0.25 |
| E6016 | VanA | >128 | 4 | 1 | 0.5 | 0.25 | 0.5 | 2 | 0.063 |
| E7312 | VanA | >128 | 2 | 0.25 | 0.063 | 0.031 | 0.125 | 0.25 | 0.016 |
| E7314 | VanB | 128 | ≤0.008 | ≤0.008 | ≤0.008 | ≤0.008 | ≤0.008 | ≤0.008 | ≤0.008 |
| E7319 | VanA | >128 | 8 | 0.5 | 0.125 | 0.063 | 0.25 | 0.5 | 0.031 |
| E7329 | VanA | 1 | ≤0.008 | ≤0.008 | ≤0.008 | ≤0.008 | ≤0.008 | ≤0.008 | ≤0.008 |
| E7401 | VanB | 16 | ≤0.008 | ≤0.008 | ≤0.008 | ≤0.008 | ≤0.008 | ≤0.008 | ≤0.008 |
| E7403 | VanB | 16 | ≤0.008 | ≤0.008 | ≤0.008 | ≤0.008 | ≤0.008 | ≤0.008 | ≤0.008 |
| E7413 | VanA | >128 | 8 | 8 | 4 | 2 | 2 | 8 | 0.5 |
| E7424 | VanB | 4 | ≤0.008 | ≤0.008 | ≤0.008 | ≤0.008 | ≤0.008 | ≤0.008 | ≤0.008 |
| E7464 | VanB | 16 | ≤0.008 | ≤0.008 | ≤0.008 | ≤0.008 | ≤0.008 | ≤0.008 | ≤0.008 |
| E8218 | VanB | 8 | ≤0.008 | ≤0.008 | ≤0.008 | ≤0.008 | ≤0.008 | ≤0.008 | ≤0.008 |
| E8235 | VanB | 16 | ≤0.008 | ≤0.008 | ≤0.008 | ≤0.008 | ≤0.008 | ≤0.008 | ≤0.008 |
| E8237 | VanA | >128 | 16 | 8 | 2 | 1 | 2 | 4 | 0.25 |
| MIC₅₀ | | 128 | 4 | 1 | 0.5 | 0.125 | 0.25 | 1 | 0.031 |
| MIC₉₀ | | 128 | 8 | 8 | 4 | 1 | 2 | 8 | 0.5 |

**Table 11: MIC results of the clinically available glycopeptide antibiotics and 5, 6, 7, 12, 14 and 16 against Gram-negative bacteria.**

| | ***Escherichia coli*** | | | ***Klebsiella pneumoniae*** | | |
|---|---|---|---|---|---|---|
| | *ATCC 35218* | *ATCC 25922* | *W3110* | *ATCC 13883* | *ATCC 27736* | *JS265* |
| **Vancomycin** | >128 | >128 | >128 | >128 | >128 | >128 |
| **Telavancin** | >128 | >128 | >128 | >128 | >128 | >128 |
| **5** | >128 | >128 | >128 | >128 | >128 | >128 |
| **6** | >128 | >128 | >128 | >128 | >128 | >128 |
| **7** | >128 | >128 | >128 | >128 | >128 | >128 |
| **12** | >128 | >128 | >128 | >128 | >128 | >128 |
| **14** | >128 | >128 | >128 | >128 | >128 | >128 |
| **16** | >128 | >128 | >128 | >128 | >128 | >128 |

**Table 12: MIC results of the clinically available glycopeptide antibiotics and 5, 12 and 16 against a panel of VISA and VRSA strains.**

| | Vancomycin | Telavancin | Oritavancin | **5** | **12** | **16** |
|---|---|---|---|---|---|---|
| **VRS1** | >128 | 8 | 1 | 16 | 4 | 0.5 |
| **VRS2** | 32 | 2 | 0.25 | 0.25 | <0.016 | <0.016 |
| **VRS3a** | 64 | 1 | 0.5 | 0.25 | <0.016 | <0.016 |
| **VRSA3b** | >128 | 4 | 0.25 | 1 | 0.25 | 0.063 |
| **VRS4** | >128 | 4 | 1 | 8 | 2 | 0.25 |
| **VRS5** | >128 | 4 | 0.5 | 4 | 1 | 0.25 |
| **VRS7** | >128 | 4 | 0.5 | 4 | 2 | 0.25 |
| **VRS8** | >128 | 16 | 1 | 16 | 4 | 1 |
| **VRS9** | >128 | 16 | 2 | 16 | 4 | 1 |
| **VRS11a** | >128 | 8 | 1 | 16 | 4 | 1 |
| **VRS11b** | >128 | 8 | 1 | 16 | 4 | 1 |
| **NRS63SH** | >128 | 8 | 1 | 8 | 2 | 0.5 |
| **BR-VRSA** | >128 | 8 | 0.25 | 4 | 1 | 0.5 |
| **NRS17** | 8 | 1 | 4 | 0.25 | 0.5 | 0.25 |
| **NRS18** | 8 | 0.25 | 1 | 0.125 | 0.25 | 0.063 |
| **NRS19** | 4 | 0.25 | 1 | 0.063 | 0.25 | 0.063 |
| **NRS51** | 4 | 0.125 | 0.5 | 0.031 | 0.125 | 0.016 |
| **NRS52** | 4 | 0.25 | 0.5 | 0.031 | 0.031 | 0.016 |

The activity of the compounds was further assessed against MRSA USA300 strain in the presence of sheep serum in another MIC assay. The cultures that grew in the presence of serum used TSB + 0.002% P80 + 50% sheep serum as growth media, as opposed to normal MICs done in only TSB + 0.002% P80.

**Table 13: MIC results of clinically available glycopeptide antibiotics and 4-18 against MRSA USA300 in the presence of sheep serum.**

| | ***TSB* + *0.002% p80*** | ***TSB* +** ***0.002% p80*** + ***50% sheep serum*** |
|---|---|---|
| Vancomycin | 1 | 0.25 |
| Teicoplanin | 0.5 | 2 |
| Telavancin | 0.125 | 1 |
| Dalbavancin | 0.25 | 2 |
| Oritavancin | 0.063 | 0.25 |
| **4** | 0.063 | 0.063 |
| **5** | 0.016 | 0.031 |
| **6** | ≤0.008 | 0.063 |
| **7** | ≤0.008 | 0.063 |
| **8** | 0.063 | 0.125 |
| **9** | 0.5 | 1 |
| **10** | 4 | 16 |
| **11** | 0.125 | 0.063 |
| **12** | 0.063 | 0.063 |
| **13** | 16 | 64 |
| **14** | 0.031 | 0.063 |
| **15** | 0.063 | 1 |
| **16** | 0.031 | 0.063 |
| **17** | 0.031 | 0.125 |
| **18** | ≤0.008 | 0.031 |

### Example 20: Testing of compounds in Hemolysis assay

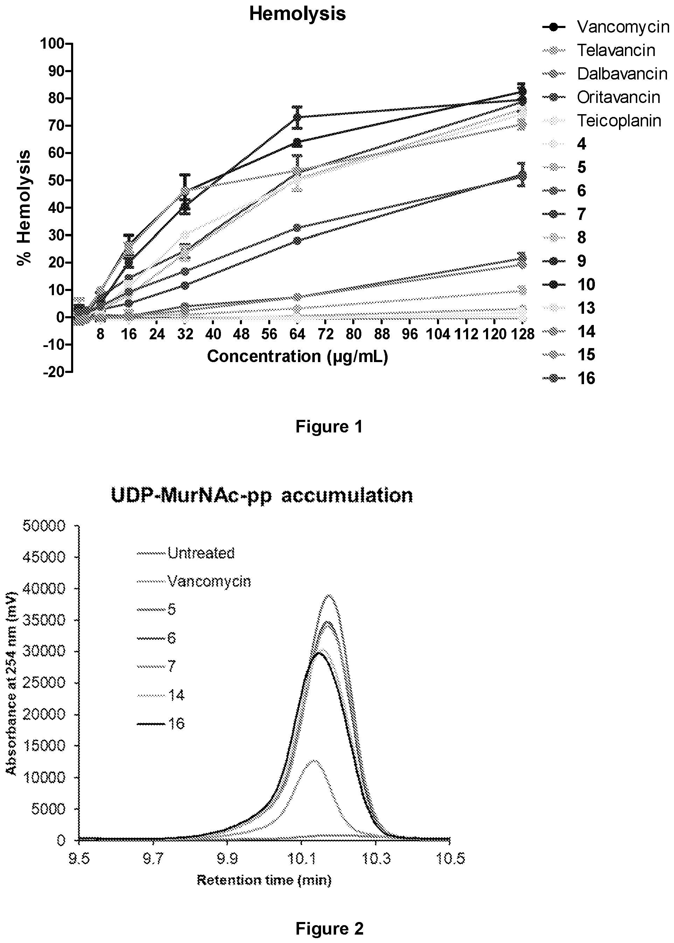

In the hemolysis assay sheep blood was treated with the test compounds for 18 hours to determine whether the compounds are lytic to these blood cells. Overall, when lipid length was increased, the percentage hemolysis appeared to increase as well. However, even at 1000-fold MIC (for some strains), many compounds did not have hemolytic properties, meaning that these compounds are non-hemolytic at relevant concentrations (the data obtained is summarized in Figure 1).

### Example 21: Testing of compounds in UDP-MurNAc-pentapeptide accumulation assay

An UDP-MurNAc-pentapeptide accumulation assay was performed to elucidate part of the mechanism of action of the compounds. UDP-MurNAc-pentapeptide is the last soluble precursor of peptidoglycan cell wall biosynthesis. In this assay, live cells accumulate this last soluble precursor when treated with compounds that interfere with the membrane bound stages of peptidoglycan biosynthesis (Sass, V. et al. Human beta-defensin 3 inhibits cell wall biosynthesis in Staphylococci. Infect. Immun. 78, 2793-2800 (2010)). Five compounds (**5, 6, 7, 14, 16**) were tested in the assay, and all showed accumulation of UDP-MurNAc-pentapeptide (the data obtained is summarized in Figure 2), similar to what was observed for the clinically used glycopeptides (data not shown). This indicates that part of the mechanism of action of the molecules involves interference with the cell wall biosynthesis in Gram-positive bacteria. The assay was performed for *S*. *aureus* ATCC29213 (as illustrated in Figure 2), *E. faecium* E155, *E. faecium* E7314, and *E. faecium* E980 and accumulation of UDP-MurNAc-pentapeptide was visible for all tested compounds in all of these tested strains (data not shown).

### Example 22: Testing of compounds in Lipid II antagonization assay

Lipid II is a known target of vancomycin and other glycopeptides (Ling, L. L. et al. A new antibiotic kills pathogens without detectable resistance. Nature 517, 455 (2015); Breukink, E. & de Kruijff, B. Lipid II as a target for antibiotics. Nat. Rev. Drug Discov. 5, 321-323 (2006)). In the antagonization assay, lipid II is co-incubated with the test antibiotics in the presence of bacterial culture. When lipid II binds the glycopeptides, the binding of these antibiotics to their target in the growing bacterial culture is antagonized. This ultimately results in diminished activity of the antibiotics and bacterial growth which normally would not be visible (at 8xMIC) becomes visible. Therefore, this assay could be used to determine if part of the mechanism of action of our compounds involves binding to lipid II. Bacterial growth was visible after co-incubation with lipid II for all five tested compounds (**5**, **6, 7, 14, 16**), indicating that binding of the compounds to lipid II is part of their mode of action, as is the case for other clinically used glycopeptide antibiotics (the data obtained is summarized in Table 11).

**Table 11. Lipid II antagonization assay. - means no visible growth, + means visible growth.**

| | **without lipid II** | **with lipid II** |
|---|---|---|
| **Vancomycin** | - | + |
| **Telavancin** | - | + |
| **5** | - | + |
| **6** | - | + |
| **7** | - | + |
| **14** | - | + |
| **16** | - | + |

### Example 23: Testing of compounds in Resistance development serial

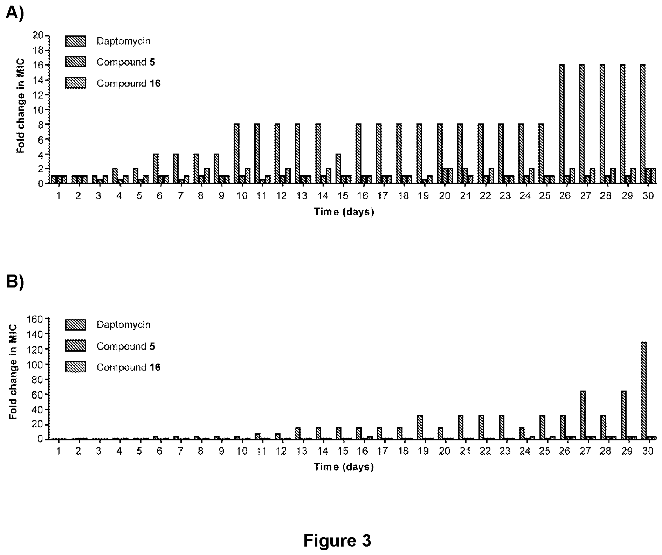

**passage assay**A resistance development serial passage assay was performed as described herein in the "Assays" section. The result obtained in this assay are depicted in Figure 3. As is apparent from the results, when MRSA was serially passaged over 30 days in the presence of sub-lethal antibiotic concentrations, compounds **5** and **16** did not induce resistance, whereas clinically used daptomycin induced significant resistance levels, increasing the MIC 16-fold. In a VanA-type VRE strain this difference was even more pronounced: low levels of resistance induction were seen for compounds **5** and **16,** as opposed to the high resistance induction of a 128-fold MIC increase for daptomycin.

### Example 24: Testing of compounds in time kill assay

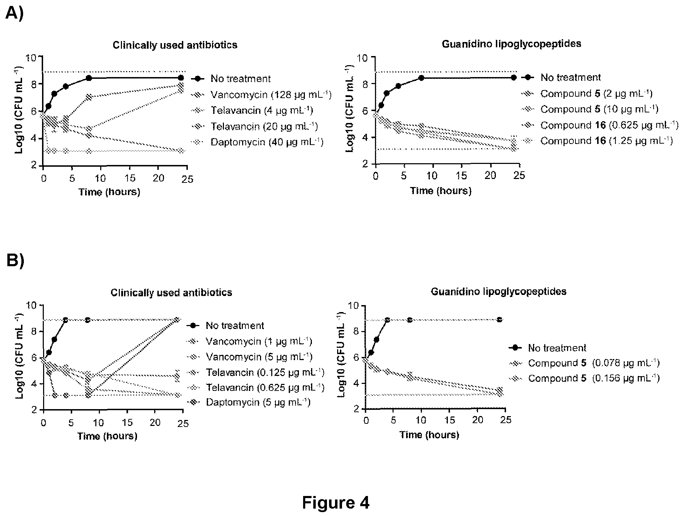

A time kill assay was performed as described herein in the "Assays" section. The result obtained in this assay are depicted in Figure 4.

### Example 25: Mammalian cytotoxicity assay

A mammalian cytotoxicity assay was performed as described herein in the "Assays" section. The results obtained in this assay are depicted in Table 12.

**Table 12: MEC and AC₅₀ of selected glycopeptides after 24h as measured by MTT assay in HepG2 cells grown in the presence of 10% FBS (replicates per concentration n = 3).**

| | Direction of response (↑↓) | MEC (µM) | AC₅₀ (µM) |
|---|---|---|---|
| carbonyl cyanide 3-chlorophenylhydrazone | | NR^{e} | NR^{e} |
| Chropromazine | ↓ | 12.4 | 19.0 |
| Vancomycin | | NR^{e} | NR^{e} |
| Telavancin | | NR^{e} | NR^{e} |
| Oritavancin | ↓ | 76.5 | >100 |
| Compound **5** | | NR^{e} | NR^{e} |
| Compound **12** | | NR^{e} | NR^{e} |
| Compound **16** | ↓ | 76.1 | 89.9 |

### Example 26: In vivo studies

Several in vivo studies were performed as described herein in the "Assays" section. In particular, the tolerability, PK and efficacy of the compounds were assessed.

### Tolerability

100 mg/kg (SC) administration of compound **5** to two mice was well tolerated. No adverse effects and normal gross morphology was observed.

### PK

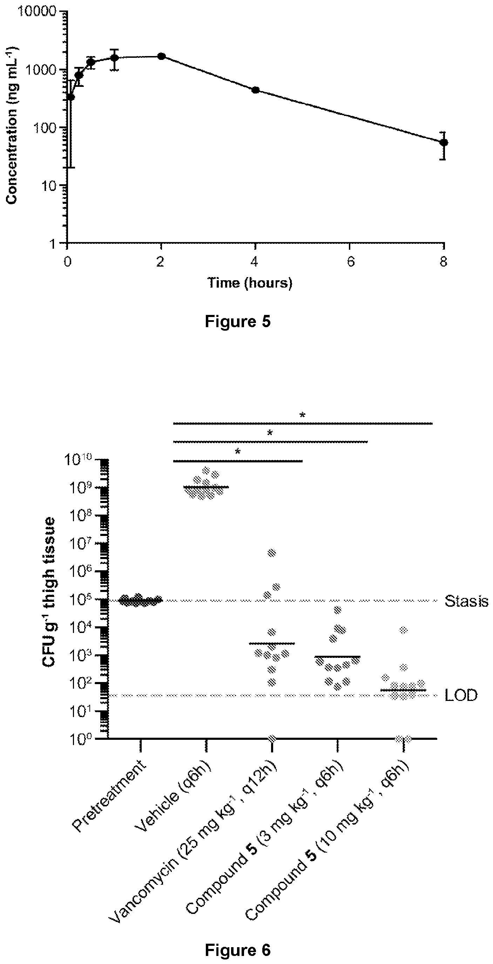

Compound **5** was administered subcutaneous at 3 mg kg⁻¹ in mice, followed by serial sampling. Data are mean ± SD (n=3). The obtained PK data is depicted in Figure 5 and Table 13.

**Table 13: Individual and mean/median mouse pharmacokinetic parameters of compound 5 (3 mg kg⁻¹, subcutaneous).**

| | **Sample 1** | **Sample 2** | **Sample 3** | **Mean / Median** | **SD** |
|---|---|---|---|---|---|
| **C₀** / **Cₘₐₓ (ng mL⁻¹)** | 1989 | 1903 | 1546 | 1813 | 235 |
| **C₀** / **Cₘₐₓ (nM)** | 1174 | 1123 | 912 | 1070 | 139 |
| **Cₗₐₛₜ (ng mL⁻¹)** | 85.3 | 34.4 | 44.2 | 54.6 | 27.0 |
| **tₗₐₛₜ (h)** | 8.00 | 8.00 | 8.00 | 8.00 | |
| **tₘₐₓ (h)** | 1.00 | 1.00 | 2.00 | 1.00 | |
| **t_{1/2} (h)** | 1.43 | 1.05 | 1.17 | 1.22 | 0.196 |
| **CL** / **CL_F (mL min⁻¹ kg^{- 1})** | 9 | 9 | 10 | 9.30 | 0.944 |
| **AUC_{inf} (ng hr mL ⁻¹)** | 5664 | 5759 | 4814 | 5412 | 520 |
| **AUC_{inf} (nM hr)** | 3342 | 3398 | 2841 | 3194 | 307 |
| **AUC₀₋ₜ(ng hr mL⁻¹)** | 5487 | 5707 | 4740 | 5311 | 507 |
| **AUC₀₋ₜ(nM hr)** | 3238 | 3368 | 2797 | 3134 | 299 |
| **Number of Points used for Lambda z** | 3 | 3 | 3 | 3 | |
| **AUC % Extrapolation to infinity** | 3.1 | 0.9 | 1.6 | 1.9 | 1.1 |

### Efficacy

Colony forming units in neutropenic mice infected in each thigh with MRSA followed 1 h later by the first subcutaneous dose of antibiotic at concentrations indicated and subsequently the same dose at indicated intervals, with sacrifice and bacterial load determination in homogenized thighs at 23 h post treatment (24 h post infection). A significant difference was found between vehicle and vancomycin at 25 mg kg⁻¹ (q12h) as well as between vehicle and compound **5** at both 3 mg kg⁻¹ and 10 mg kg⁻¹ (q6h) (all p<0.0001). A significant difference was found between vancomycin at 25 mg kg⁻¹ and compound **5** at 10 mg kg⁻¹ (p<0.0001) as well, whereas no significant difference was found between vancomycin at 25 mg kg⁻¹ and compound **5** at 3 mg kg⁻¹. A significant difference was found between the two different compound **5** doses (p=0.0001). Vancomycin (25 mg kg⁻¹) and compound **5** (3 mg kg⁻¹ and 10 mg kg⁻¹) also showed a significant reduction in thigh burden compared to pretreatment (p=0.0042, p=0.0001 and p<0.0001 respectively). Data are mean ± SEM (n=12, six mice per group, two thighs per mouse). The data obtained in this efficacy study is depicted in Figure 6.

**Table 14: Thigh burdens in colony forming units (CFU) g⁻¹. BLD= below limit of detection.**

| **Pretreatment** | **Vehicle q6h** | **Vancomycin 25 mg kg⁻¹ q12h)** | **Compound 5 3 mg kg⁻¹ q6h** | **Compound 5 10 mg kg⁻¹ q6h** |
|---|---|---|---|---|
| 76075 | 488345865 | 106 | 42132 | 7984 |
| 80434 | 3971232877 | 825 | 7735 | 81 |
| 83111 | 555841584 | 1030 | 3885 | 34 |
| 86078 | 835263158 | 2023 | 113 | 368 |
| 90667 | 987022901 | 1148 | 368 | 35 |
| 100286 | 722195122 | 142564 | 456 | 77 |
| 73102 | 512903226 | 4605085 | 9139 | 96 |
| 85217 | 814594595 | 283390 | 666 | 76 |
| 121720 | 855384615 | 1204 | 350 | 40 |
| 73286 | 1851351351 | 310 | 587 | BLD |
| 89346 | 1471428571 | BLD | 75 | 158 |
| 110505 | 2851282051 | 6736 | 113 | BLD |

### Colony

**Table 15: Kruskal-Wallis statistical comparison of thigh burden. Corrected for multiple comparisons, StatsDirect-Conover-Inman. NS = not significant.**

| | **Vehicle q6h** | **Compound 5 3 mg kg⁻¹ q6h** | **Compound 5 10 mg kg⁻¹ q6h** | **Vancomycin 25 mg kg⁻¹ q12h** |
|---|---|---|---|---|
| **Pretreatment** | p=0.004 | p=0.0001 | p<0.0001 | p=0.0042 |
| **Vehicle q6h** | | p<0.0001 | p<0.0001 | p<0.0001 |
| **Compound 5 (3 mg kg⁻¹ q6h)** | | | | |
| **Compound 5 (10 mg kg⁻¹ q6h)** | | | | |
| **Vancomycin (25 mg kg⁻¹ q12h)** | | | | |

## Claims

1. A compound of formula I: 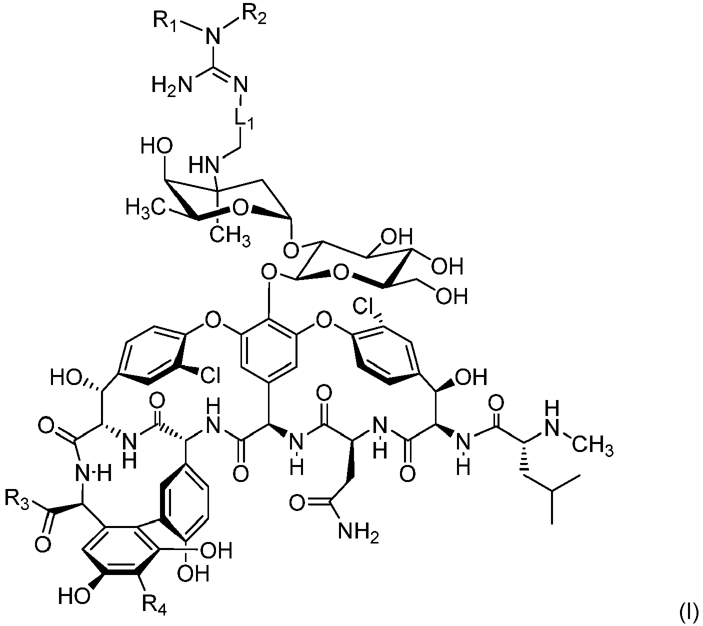 wherein:
R₁ is a lipid;
R₂ is selected from -H or a lipid;
R₃ is selected from -OH, substituted or unsubstituted -C₁-C₂₀ alkyl, substituted or unsubstituted -C₂-C₂₀ alkenyl, substituted or unsubstituted -C₁-C₄ alkylaryl and carbohydrate selected from -(NHCH₂CH₂)ₓ-Glc, -(NHCH₂CH₂)ₓ-Gal, -(NHCH₂CH₂)ₓ-Man, - (NHCH₂CH₂)ₓ₋GlcNAc, -(NHCH₂CH₂)ₓ-MurNAc, -(NHCH₂CH₂)ₓ-ManNAc, -(NHCH₂CH₂)ₓ-GaINAc, -(NHCH₂CH₂)ₓ-cellbiose and -(NHCH₂CH₂)ₓ-maltose, where x is 0 or 1;
R₄ is selected from -H, substituted or unsubstituted -C₁-C₂₀ alkyl, substituted or unsubstituted -C₂-C₂₀ alkenyl, substituted or unsubstituted -C₁-C₄ alkylaryl and carbohydrate selected from -(NHCH₂CH₂)_{y}-Glc, -(NHCH₂CH₂)_{y}-Gal, -(NHCH₂CH₂)_{y}-Man, - (NHCH₂CH₂)_{y}-GlcNAc, -(NHCH₂CH₂)_{y}-MurNAc, -(NHCH₂CH₂)_{y}-ManNac, -(NHCH₂CH₂)_{y}-GaINAc, -(NHCH₂CH₂)_{y}-cellbiose and -(NHCH₂CH₂)_{y}-maltose, where y is 0 or 1; and
L₁ is a linker selected from -C₁-C₂₀ alkylene-, -C₂-C₂₀ alkenylene-, -(C₁-C₄ alkyl)arylene-, -C₂-C₄ alkyl-[O(CH₂)ᵣ]ₛ-O(CH₂)ᵣ-, -C₂-C₁₂ alkyl-S-S-C₁-C₁₂ alkyl-, -C(O)C₁-C₂₀ alkylene-, - C(O)C₂-C₂₀ alkenylene-, -C(O)(C₁-C₄ alkyl)arylene-, -C(O)C₁-C₄ alkyl-[O(CH₂)ᵣ]ₛ-O(CH₂)ᵣ-, - C(O)NHC₁-C₂₀ alkylene-, -C(O)NHC₂-C₂₀ alkenylene-, --C(O)NH(C₁-C₄ alkyl)arylene-, - C(O)NHC₁-C₄ alkyl-[O(CH₂)ᵣ]ₛ-O(CH₂)ᵣ-, -C(S)NHC₁-C₂₀ alkylene-, -C(S)NHC₂-C₂₀ alkenylene-, -C(S)NH(C₁-C₄ alkyl)arylene- and -C(S)NHC₁-C₄ alkyl-[O(CH₂)ᵣ]ₛ-O(CH₂)ᵣ-, where r is selected from 2 and 3 and s is selected from 0 to 20,
or a pharmaceutically acceptable salt, stereoisomer, or solvate thereof,
wherein each lipid is a hydrophobic moiety comprising substituted or unsubstituted alkyl, alkenyl, cycloalkyl, bridged cycloalkyl, (alkyl)cycloalkyl, (alkyl) bridged cycloalkyl, (alkyl)cycloalkenyl, and/or alkylaryl groups,
wherein, when substituted, any of the above groups are substituted by one or more substituents selected from: -OH, -CN, -NH₂, -NH(C₁-C₆ alkyl), -N(C₁-C₄ alkyl)₂, =O, -halo, - C₁-C₆ alkyl, -C₂-C₆ alkenyl, -C₁-C₆ haloalkyl, -C₁-C₆ haloalkoxy and-C₂-C₆ haloalkenyl, and - C₁-C₆ alkylcarboxylic acid.

2. The compound of claim 1, wherein R₁ is selected from -C₄-C₂₀ alkyl, -C₄-C₂₀ alkenyl, -C₄-C₂₀ cycloalkyl, C₁-C₄alkyl-C₄-C₂₀ cycloalkyl, substituted or unsubstituted -C₁-C₄ alkylaryl, -C₁-C₄ alkyl-[O(CH₂)ₘ]ₙ-O(CH₂)ₘ₋₁CH₃, where m is selected from 2 and 3 and n is selected from 0 to 20, -C₂-C₁₂ alkyl-S-S-C₁-C₁₂ alkyl,
optionally wherein R₁ is selected from -C₄-C₂₀ alkyl, -C₄-C₂₀ alkenyl, substituted or unsubstituted -C₁-C₄ alkylaryl, -C₁-C₄ alkyl-[O(CH₂)ₘ]ₙ-O(CH₂)ₘ₋₁CH₃, where m is selected from 2 and 3 and n is selected from 0 to 20, -C₂C₁₂ alkyl-S-S-C₁-C₁₂ alkyl.

3. The compound of any preceding claim, wherein R₁ is selected from -C₆-C₁₄ alkyl, -C₆-C₁₆ alkenyl and substituted or unsubstituted -C₁-C₄ alkylbisphenyl.

4. The compound of any preceding claim, wherein R₁ is selected from -C₆-C₁₄ alkyl, -C₁-C₄ adamantyl, -adamantyl, -geranyl, -farnesyl and -chlorobisphenyl;
optionally wherein R₁ is selected from -C₆-C₁₂ alkyl, -geranyl, -farnesyl and - chlorobisphenyl, or
optionally wherein R₁ is selected from 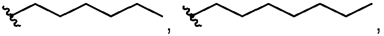 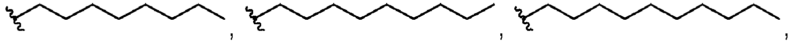 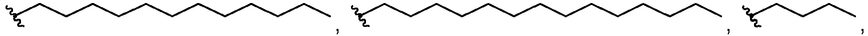 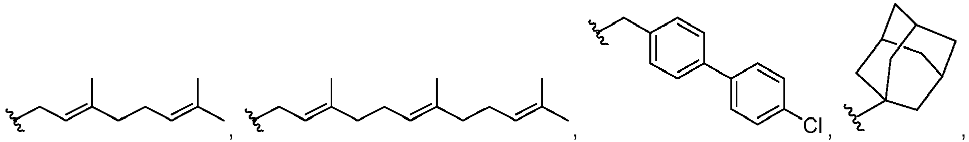 or

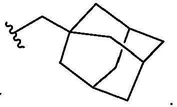

5. The compound of any preceding claim, wherein R₂ is selected from -H, -C₄-C₂₀ alkyl, -C₄-C₂₀ alkenyl, substituted or unsubstituted -C₁-C₄ alkylaryl, -C₁-C₄ alkyl-[O(CH₂)ₚ]_{q}-O(CH₂)ₚ₋₁CH₃, where p is selected from 2 and 3 and q is selected from 0 to 20; and -C₂-C₁₂ alkyl-S-S-C₁-C₁₂ alkyl,
optionally wherein R₂ is selected from -H, -C₄-C₁₂ alkyl, -C₄-C₁₂ alkenyl, substituted or unsubstituted -C₁-C₄ alkylaryl, and -C₁-C₄ alkyl-[O(CH₂)ₚ]_{q}-O(CH₂)ₚ₋₁CH₃, where p is selected from 2 and 3 and q is selected from 0 to 20; further optionally wherein R₂ is selected from -H, -C₄-C₁₂ alkyl, -C₄-C₁₂ alkenyl;
still further optionally wherein R₂ is -H.

6. The compound of claim 1, wherein each of R₁ and R₂ are independently selected from -C₂-C₁₀ alkyl, -C₂-C₁₀ alkenyl and -C₁-C₄ alkyl-[O(CH₂)ₘ]ₙ-O(CH₂)ₘ₋₁CH₃, where m is selected from 2 and 3 and n is selected from 0 to 20,
optionally wherein each of R₁ and R₂ are independently selected from -C₂-C₁₀ alkyl and -C₂-C₁₀ alkenyl.

7. The compound of any preceding claim, wherein R₃ is selected from -OH, substituted or unsubstituted -C₁-C₁₀ alkyl, substituted or unsubstituted -C₂-C₁₀ alkenyl, substituted or unsubstituted -C₁-C₄ alkylaryl and carbohydrate selected from -(NHCH₂CH₂)_{y}-Glc, - (NHCH₂CH₂)_{y}-Gal, -(NHCH₂CH₂)_{y}-Man, -(NHCH₂CH₂)_{y}-GlcNAc, -(NHCH₂CH₂)_{y}-MurNAc, - (NHCH₂CH₂)_{y}-ManNac, -(NHCH₂CH₂)_{y}-GalNAc, -(NHCH₂CH₂)_{y}-cellbiose and - (NHCH₂CH₂)_{y}-maltose, where y is 0 or 1,
optionally wherein R₃ is selected from -OH, -NHCH₂CH₂CH₂N(CH₃)₂, 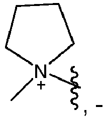 (NHCH₂CH₂)ₓ-Glc, -(NHCH₂CH₂)ₓ-Gal, -(NHCH₂CH₂)ₓ-Man, -(NHCH₂CH₂)ₓ-GlcNAc, - (NHCH₂CH₂)ₓ-MurNAc, -(NHCH₂CH₂)ₓ-ManNAc, -(NHCH₂CH₂)ₓ-GalNAc, -(NHCH₂CH₂)ₓ-cellbiose and -(NHCH₂CH₂)ₓ-maltose, where x is 0 or 1,
further optionally wherein R₃ is -OH.

8. The compound of any preceding claim, wherein R₄ is selected from -H, substituted or unsubstituted -C₁-C₁₀ alkyl, substituted or unsubstituted -C₂-C₁₀ alkenyl, substituted or unsubstituted -C₁-C₄ alkylaryl and carbohydrate selected from -(NHCH₂CH₂)_{y}-Glc, - (NHCH₂CH₂)_{y}-Gal, -(NHCH₂CH₂)_{y}-Man, -(NHCH₂CH₂)_{y}-GlcNAc, -(NHCH₂CH₂)_{y}-MurNAc, - (NHCH₂CH₂)_{y}-ManNac, -(NHCH₂CH₂)_{y}-GalNAc, -(NHCH₂CH₂)_{y}-cellbiose and - (NHCH₂CH₂)_{y}-maltose, where y is 0 or 1,
optionally wherein R₄ is selected from -H, -CH₂NHCH₂P(O)(OH)₂, - CH₂N(CH₂PO₃H₂)₂, -CH₂NHCH₂CH₂CH₂N(CH₃)₂, -CH₂NHCH₂CH₂COOH, - CH₂N(CH₃)CH₂(CH(OH))₄CH₂OH, -CH₂NHCH(COOH)CH₂COOH, -CH₂NH(CH₂CH₂OH)₂, - (NHCH₂CH₂)_{y}-Glc, -(NHCH₂CH₂)_{y}-Gal, -(NHCH₂CH₂)_{y}-Man, -(NHCH₂CH₂)_{y}-GlcNAc, - (NHCH₂CH₂)_{y}-MurNAc, -(NHCH₂CH₂)_{y}-ManNAc, -(NHCH₂CH₂)_{y}-GalNAc, -(NHCH₂CH₂)_{y}-cellbiose and -(NHCH₂CH₂)_{y}-maltose, where y is 0 or 1,
further optionally wherein R₄ is -H.

9. The compound of any preceding claim, wherein L₁ is selected from -C₂-C₂₀ alkylene-, -C₂-C₂₀ alkenylene-, -(C₁-C₄ alkyl)arylene-, -C₂-C₄ alkyl-[O(CH₂)ᵣ]ₛ-O(CH₂)ᵣ-, -C₂-C₁₂ alkyl-S-S-C₁-C₁₂ alkyl-, -C(O)C₁-C₂₀ alkylene-, -C(O)C₂-C₂₀ alkenylene-, -C(O)(C₁-C₄ alkyl)arylene-, - C(O)C₁-C₄ alkyl-[O(CH₂)ᵣ]ₛ-O(CH₂)ᵣ-, -C(O)NHC₁-C₂₀ alkylene-, -C(O)NHC₂-C₂₀ alkenylene-, -C(O)NH(C₁-C₄ alkyl)arylene-, -C(O)NHC₁-C₄ alkyl-[O(CH₂)ᵣ]ₛ-O(CH₂)ᵣ-, -C(S)NHC₁-C₂₀ alkylene-, -C(S)NHC₂-C₂₀ alkenylene-, -C(S)NH(C₁-C₄ alkyl)arylene- and -C(S)NHC₁-C₄ alkyl-[O(CH₂)ᵣ]ₛ-O(CH₂)ᵣ-,
where r is selected from 2 and 3 and s is selected from 0 to 20,
optionally wherein L₁ is selected from -C₂-C₁₀ alkylene- and -(C₁-C₄ alkyl)arylene-, further optionally wherein L₁ is -CH₂Ph-.

10. A compound of any preceding claim, selected from: 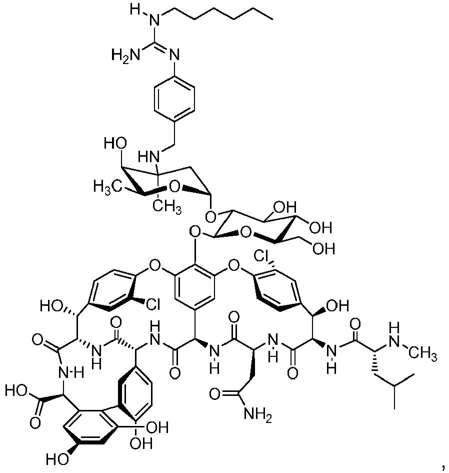 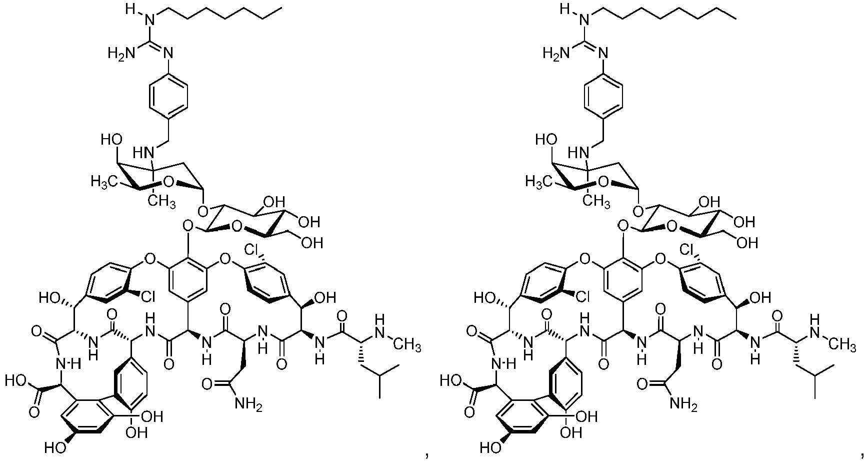 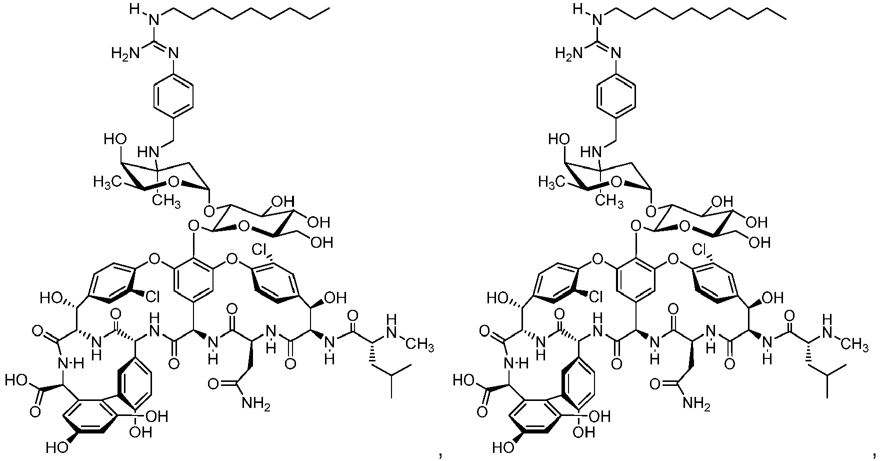 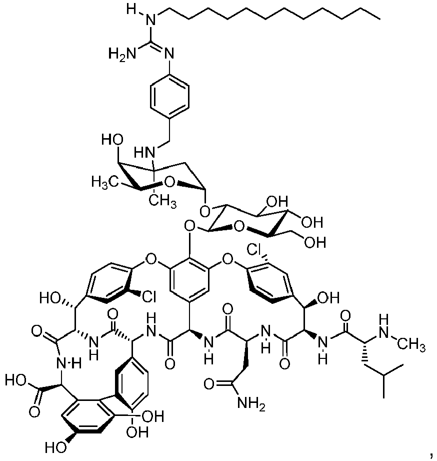 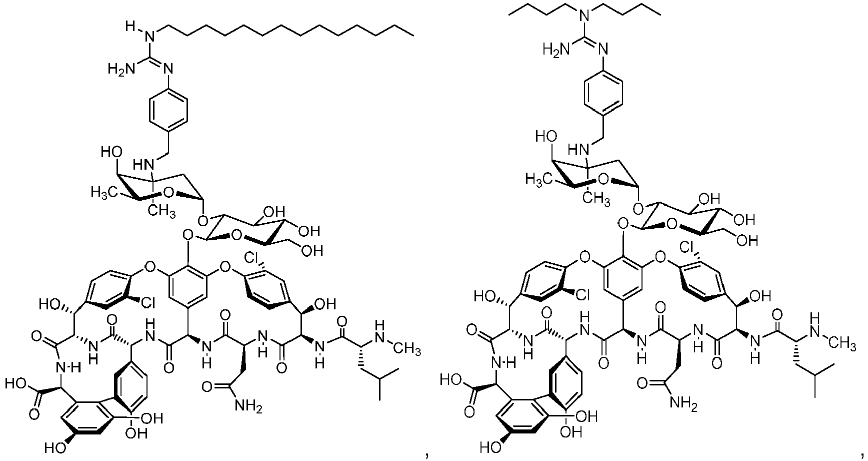 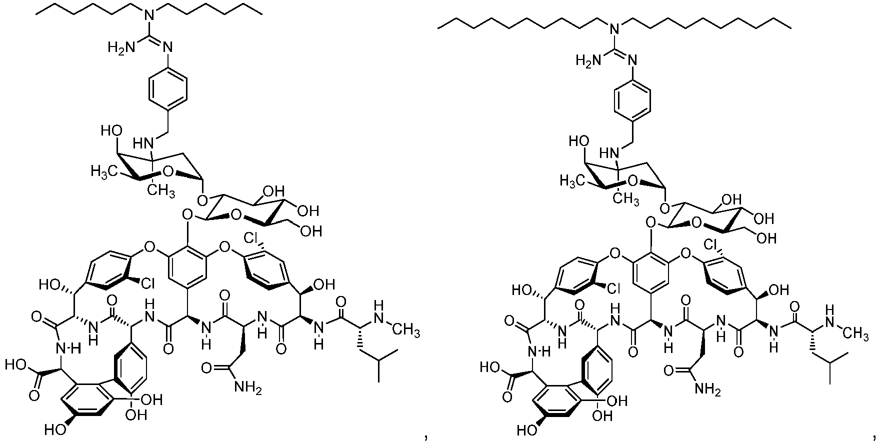 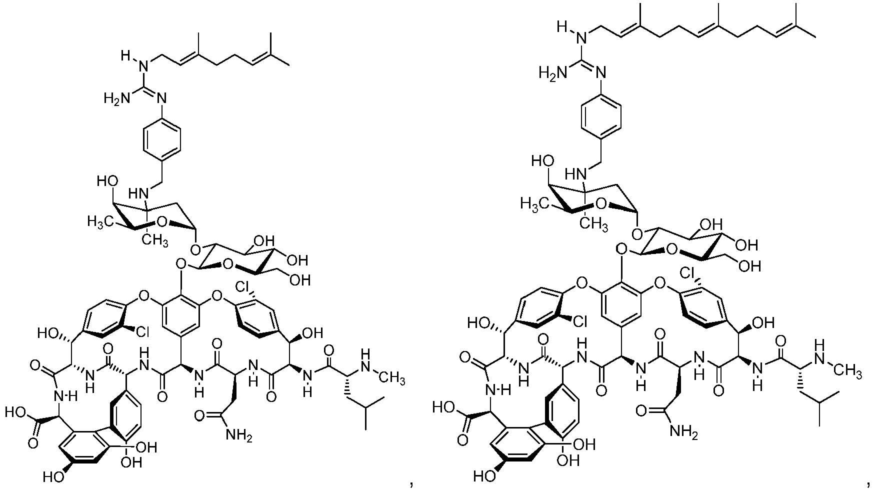 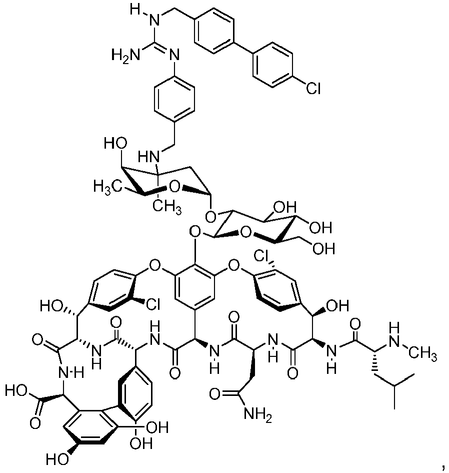 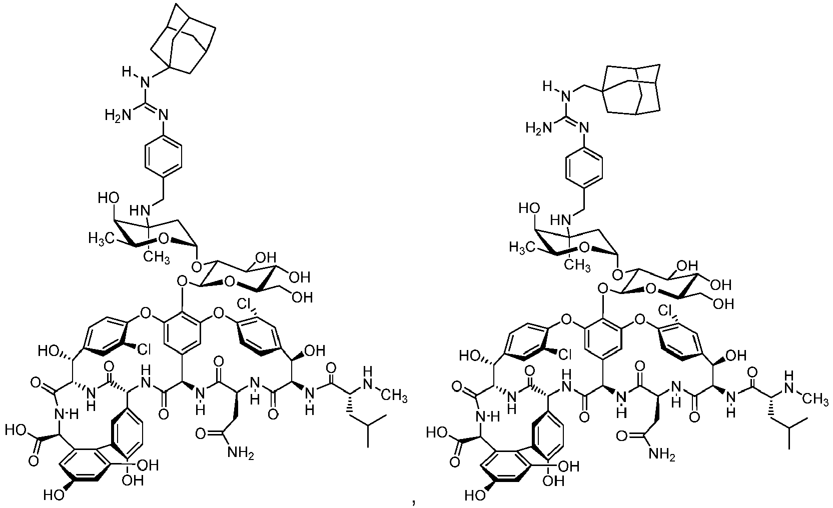 or a pharmaceutically acceptable salt, or solvate thereof.

11. A formulation, comprising the compound of any preceding claim and optionally a pharmaceutically acceptable carrier.

12. The formulation of claim 11, wherein the formulation is a parenteral formulation or an oral formulation,
optionally wherein the formulation is a parenteral formulation, further optionally when the formulation is a formulation for intravenous injection.

13. The compound of any of claims 1 to 10, or formulation of claim 11 or claim 12, for use as a medicament.

14. The compound of any of claims 1 to 10, or formulation of claim 11 or claim 12, for use in the treatment of a bacterial infection.

15. The compound or formulation for use of claim 14, wherein the bacterial infection is an infection by Gram-positive bacteria.

## Patentansprüche

1. Verbindung der Formel I: 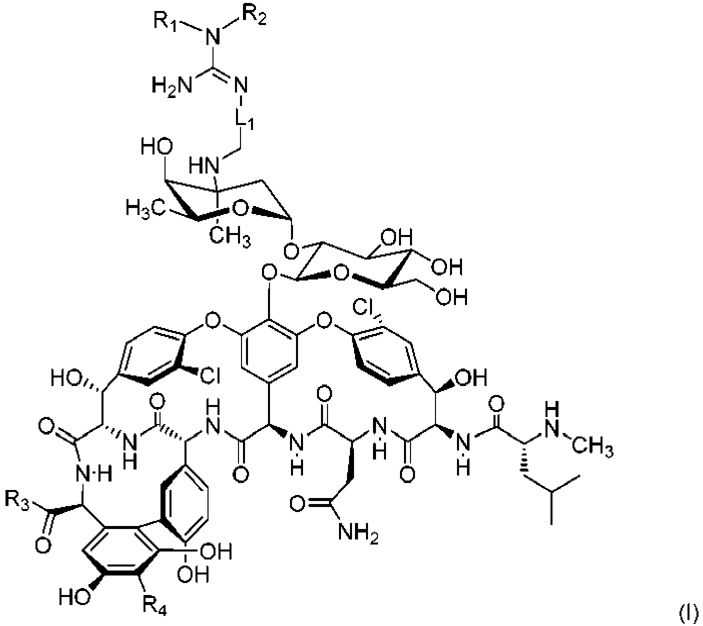 wobei:
R₁ ein Lipid ist;
R₂ ausgewählt ist aus -H oder einem Lipid;
R₃ ausgewählt ist aus -OH, substituiertem oder unsubstituiertem -C₁-C₂₀-Alkyl, substituiertem oder unsubstituiertem -C₂-C₂₀-Alkenyl, substituiertem oder unsubstituiertem -C₁-C₄-Alkylaryl und Kohlenhydrat ausgewählt aus -(NHCH₂CH₂)ₓ-Glc, -(NHCH₂CH₂)ₓ-Gal, - (NHCH₂CH₂)ₓ-Man, -(NHCH₂CH₂)ₓ-GlcNAc, -(NHCH₂CH₂)ₓ-MurNAc, -(NHCH₂CH₂)ₓ-ManNAc, -(NHCH₂CH₂)ₓ-GalNAc, -(NHCH₂CH₂)ₓ-Cellbiose und -(NHCH₂CH₂)ₓ-Maltose, wobei x 0 oder 1 ist;
R₄ ausgewählt ist aus -H, substituiertem oder unsubstituiertem -C₁-C₂₀-Alkyl, substituiertem oder unsubstituiertem -C₂-C₂₀-Alkenyl, substituiertem oder unsubstituiertem -C₁-C₄-Alkylaryl und Kohlenhydrat ausgewählt aus -(NHCH₂CH₂)_{y}-Glc, -(NHCH₂CH₂)_{y}-Gal, - (NHCH₂CH₂)_{y}-Man, -(NHCH₂CH₂)_{y}-GlcNAc, -(NHCH₂CH₂)_{y}-MurNAc, -(NHCH₂CH₂)_{y}-ManNac, -(NHCH₂CH₂)_{y}-GalNAc, -(NHCH₂CH₂)_{y}-Cellbiose und -(NHCH₂CH₂)_{y}-Maltose, wobei y 0 oder 1 ist; und
L₁ ein Linker ausgewählt aus -C₁-C₂₀-Alkylen-, -C₂-C₂₀-Alkenylen-, -(C₁-C₄-Alkyl)arylen-, -C₂-C₄-Alkyl-[O(CH₂)ᵣ]ₛ-O(CH₂)ᵣ-, -C₂-C₁₂-Alkyl-S-S-C₁-C₁₂-alkyl-, -C(O)C₁-C₂₀-Alkylen-, - C(O)C₂-C₂₀-Alkenylen-, -C(O)(C₁-C₄-Alkyl)arylen-, -C(O)C₁-C₄-Alkyl-[O(CH₂)ᵣ]ₛ-O(CH₂)ᵣ-, - C(O)NHC₁-C₂₀-Alkylen-, -C(O)NHC₂-C₂₀-Alkenylen-, --C(O)NH(C₁-C₄-Alkyl)arylen-, - C(O)NHC₁-C₄-Alkyl-[O(CH₂)ᵣ]ₛ-O(CH₂)ᵣ-, -C(S)NHC₁-C₂₀-Alkylen-, -C(S)NHC₂-C₂₀-Alkenylen-, -C(S)NH(C₁-C₄-Alkyl)arylen- und -C(S)NHC₁-C₄-Alkyl-[O(CH₂)ᵣ]_{S}-O(CH₂)ᵣ- ist, wobei r ausgewählt ist aus 2 und 3 und s ausgewählt ist aus 0 bis 20,
oder pharmazeutisch verträgliches Salz, Stereoisomer oder Solvat davon,
wobei jedes Lipid eine hydrophobe Einheit ist, die substituierte oder unsubstituierte Alkyl-, Alkenyl-, Cycloalkyl-, überbrückte Cycloalkyl-, (Alkyl)cycloalkyl-, (Alkyl)überbrückte Cycloalkyl-, (Alkyl)cycloalkenyl- und/oder Alkylarylgruppen umfasst,
wobei, wenn substituiert, beliebige der obigen Gruppen substituiert sind durch einen oder mehrere Substituenten ausgewählt aus: -OH, -CN, -NH₂, -NH(C₁-C₆-Alkyl), -N(C₁-C₄-Alkyl)₂, =O, -Halogen, - C₁-C₆-Alkyl, -C₂-C₆-Alkenyl, -C₁-C₆-Halogenalkyl, -C₁-C₆-Halogenalkoxy und-C₂-C₆-Halogenalkenyl und -C₁-C₆-Alkylcarbonsäure.

2. Verbindung nach Anspruch 1, wobei R₁ ausgewählt ist aus -C₄-C₂₀-Alkyl, -C₄-C₂₀-Alkenyl, -C₄-C₂₀-Cycloalkyl, C₁-C₄-Alkyl-C₄-C₂₀-cycloalkyl, substituiertem oder unsubstituiertem -C₁-C₄-Alkylaryl, -C₁-C₄-Alkyl-[O(CH₂)ₘ]ₙ-O(CH₂)ₘ₋₁CH₃, wobei m ausgewählt ist aus 2 und 3 und n ausgewählt ist aus 0 bis 20, -C₂-C₁₂-Alkyl-S-S-C₁-C₁₂-alkyl,
wobei optional R₁ ausgewählt ist aus -C₄-C₂₀-Alkyl, -C₄-C₂₀-Alkenyl, substituiertem oder unsubstituiertem -C₁-C₄-Alkylaryl, -C₁-C₄-Alkyl-[O(CH₂)ₘ]ₙ-O(CH₂)ₘ₋₁CH₃, wobei m ausgewählt ist aus 2 und 3 und n ausgewählt ist aus 0 bis 20, -C₂C₁₂-Alkyl-S-S-C₁-C₁₂-alkyl.

3. Verbindung nach einem vorhergehenden Anspruch, wobei R₁ ausgewählt ist aus -C₆-C₁₄-Alkyl, -C₆-C₁₆-Alkenyl und substituiertem oder unsubstituiertem -C₁-C₄-Alkylbisphenyl.

4. Verbindung nach einem vorhergehenden Anspruch, wobei R₁ ausgewählt ist aus -C₆-C₁₄-Alkyl, -C₁-C₄-Adamantyl, -Adamantyl, -Geranyl, -Farnesyl und -Chlorbisphenyl;
wobei optional R₁ ausgewählt ist aus -C₆-C₁₂-Alkyl, -Geranyl, -Farnesyl und - Chlorbisphenyl, oder
wobei optional R₁ ausgewählt ist aus 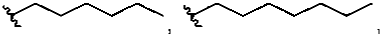 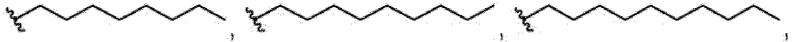 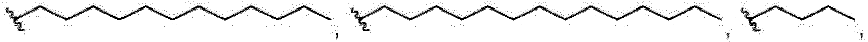 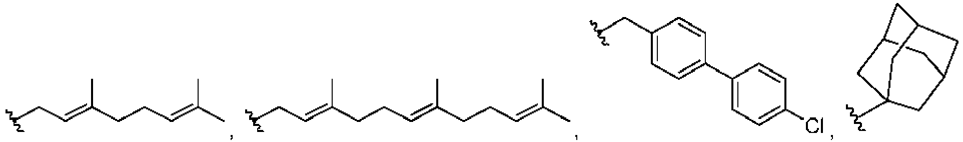 oder

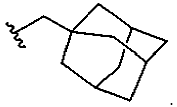

5. Verbindung nach einem vorhergehenden Anspruch, wobei R₂ ausgewählt ist aus -H, -C₄-C₂₀-Alkyl, -C₄-C₂₀-Alkenyl, substituiertem oder unsubstituiertem -C₁-C₄-Alkylaryl, -C₁-C₄-Alkyl-[O(CH₂)ₚ]_{q}-O(CH₂)ₚ₋₁CH₃, wobei p ausgewählt ist aus 2 und 3 und q ausgewählt ist aus 0 bis 20; und -C₂-C₁₂-Alkyl-S-S-C₁-C₁₂-alkyl,
wobei optional R₂ ausgewählt ist aus -H, -C₄-C₁₂-Alkyl, -C₄-C₁₂-Alkenyl, substituiertem oder unsubstituiertem -C₁-C₄-Alkylaryl und -C₁-C₄-Alkyl-[O(CH₂)ₚ]_{q}-O(CH₂)ₚ₋₁CH₃, wobei p ausgewählt ist aus 2 und 3 und q ausgewählt ist aus 0 bis 20; wobei ferner optional R₂ ausgewählt ist aus -H, -C₄-C₁₂-Alkyl, -C₄-C₁₂-Alkenyl;
wobei noch weiter optional R₂ -H ist.

6. Verbindung nach Anspruch 1, wobei jedes von R₁ und R₂ unabhängig ausgewählt ist aus - C₂-C₁₀-Alkyl, -C₂-C₁₀-Alkenyl und -C₁-C₄-Alkyl-[O(CH₂)ₘ]ₙ-O(CH₂)ₘ₋₁CH₃, wobei m ausgewählt ist aus 2 und 3 und n ausgewählt ist aus 0 bis 20,
wobei optional jedes von R₁ und R₂ unabhängig ausgewählt ist aus -C₂-C₁₀-Alkyl und -C₂-C₁₀-Alkenyl.

7. Verbindung nach einem vorhergehenden Anspruch, wobei R₃ ausgewählt ist aus -OH, substituiertem oder unsubstituiertem -C₁-C₁₀-Alkyl, substituiertem oder unsubstituiertem -C₂-C₁₀-Alkenyl, substituiertem oder unsubstituiertem -C₁-C₄-Alkylaryl und Kohlenhydraten ausgewählt aus -(NHCH₂CH₂)_{y}-Glc, -(NHCH₂CH₂)_{y}-Gal, -(NHCH₂CH₂)_{y}-Man, -(NHCH₂CH₂)_{y}-GlcNAc, - (NHCH₂CH₂)_{y}-MurNAc, -(NHCH₂CH₂)_{y}-ManNac, -(NHCH₂CH₂)_{y}-GalNAc, -(NHCH₂CH₂)_{y}-Cellbiose und -(NHCH₂CH₂)_{y}-Maltose, wobei y 0 oder 1 ist,
wobei optional R₃ ausgewählt ist aus -OH, -NHCH₂CH₂CH₂N(CH₃)₂, 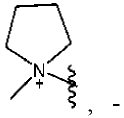 (NHCH₂CH₂)ₓ-Glc, -(NHCH₂CH₂)ₓ-Gal, -(NHCH₂CH₂)ₓ-Man, -(NHCH₂CH₂)ₓ-GlcNAc, - (NHCH₂CH₂)ₓ-MurNAc, -(NHCH₂CH₂)ₓ-ManNAc, -(NHCH₂CH₂)ₓ-GalNAc, -(NHCH₂CH₂)ₓ-Cellbiose und -(NHCH₂CH₂)ₓ-Maltose, wobei x 0 oder 1 ist,
wobei ferner optional R₃ -OH ist.

8. Verbindung nach einem vorhergehenden Anspruch, wobei R₄ ausgewählt ist aus -H, substituiertem oder unsubstituiertem -C₁-C₁₀-Alkyl, substituiertem oder unsubstituiertem -C₂-C₁₀-Alkenyl, substituiertem oder unsubstituiertem -C₁-C₄-Alkylaryl und Kohlenhydraten ausgewählt aus -(NHCH₂CH₂)_{y}-Glc, -(NHCH₂CH₂)_{y}-Gal, -(NHCH₂CH₂)_{y}-Man, -(NHCH₂CH₂)_{y}-GlcNAc, - (NHCH₂CH₂)_{y}-MurNAc, -(NHCH₂CH₂)_{y}-ManNac, -(NHCH₂CH₂)_{y}-GalNAc, -(NHCH₂CH₂)_{y}-Cellbiose und -(NHCH₂CH₂)_{y}-Maltose, wobei y 0 oder 1 ist,
wobei optional R₄ ausgewählt ist aus -H, -CH₂NHCH₂P(O)(OH)₂, -CH₂N(CH₂PO₃H₂)₂, - CH₂NHCH₂CH₂CH₂N(CH₃)₂, -CH₂NHCH₂CH₂COOH, -CH₂N(CH₃)CH₂(CH(OH))₄CH₂OH, - CH₂NHCH(COOH)CH₂COOH, -CH₂NH(CH₂CH₂OH)₂, -(NHCH₂CH₂)_{y}-Glc, -(NHCH₂CH₂)_{y}-Gal, -(NHCH₂CH₂)_{y}-Man, -(NHCH₂CH₂)_{y}-GlcNAc, -(NHCH₂CH₂)_{y}-MurNAc, -(NHCH₂CH₂)_{y}-ManNAc, -(NHCH₂CH₂)_{y}-GalNAc, -(NHCH₂CH₂)_{y}-Cellbiose und -(NHCH₂CH₂)_{y}-Maltose, wobei y 0 oder 1 ist,
wobei ferner optional R₄ -H ist.

9. Verbindung nach einem vorhergehenden Anspruch, wobei L₁ ausgewählt ist aus -C₂-C₂₀-Alkylen-, -C₂-C₂₀-Alkenylen-, -(C₁-C₄-Alkyl)arylen-, -C₂-C₄-Alkyl-[O(CH₂)ᵣ]ₛ-O(CH₂)ᵣ-, -C₂-C₁₂-Alkyl-S-S-C₁-C₁₂-alkyl-, -C(O)C₁-C₂₀-Alkylen-, -C(O)C₂-C₂₀-Alkenylen-, -C(O)(C₁-C₄-Alkyl)arylen-, -C(O)C₁-C₄-Alkyl-[O(CH₂)ᵣ]ₛ-O(CH₂)ᵣ-, -C(O)NHC₁-C₂₀-Alkylen-, -C(O)NHC₂-C₂₀-Alkenylen-, -C(O)NH(C₁-C₄-Alkyl)arylen-, -C(O)NHC₁-C₄-Alkyl-[O(CH₂)ᵣ]ₛ-O(CH₂)ᵣ-, - C(S)NHC₁-C₂₀-Alkylen-, -C(S)NHC₂-C₂₀-Alkenylen-, -C(S)NH(C₁-C₄-Alkyl)arylen- und - C(S)NHC₁-C₄-Alkyl-[O(CH₂)ᵣ]ₛ-O(CH₂)ᵣ-,
wobei r ausgewählt ist aus 2 und 3 und s ausgewählt ist aus 0 bis 20,
wobei optional L₁ ausgewählt ist aus -C₂-C₁₀-Alkylen- und -(C₁-C₄-Alkyl)arylen-,
wobei ferner optional L₁ -CH₂Ph- ist.

10. Verbindung nach einem vorhergehenden Anspruch, ausgewählt aus: 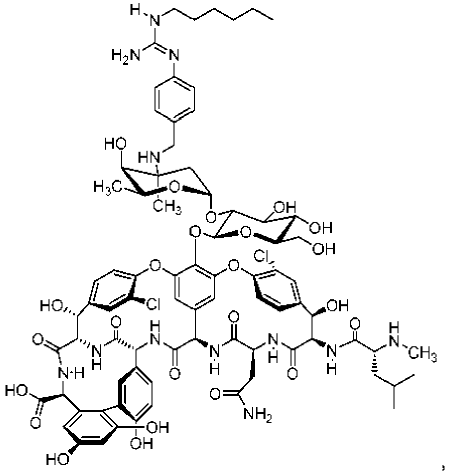 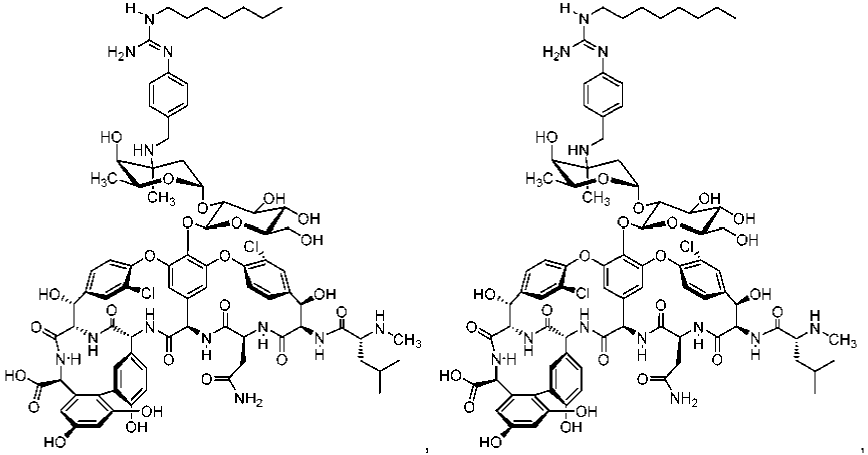 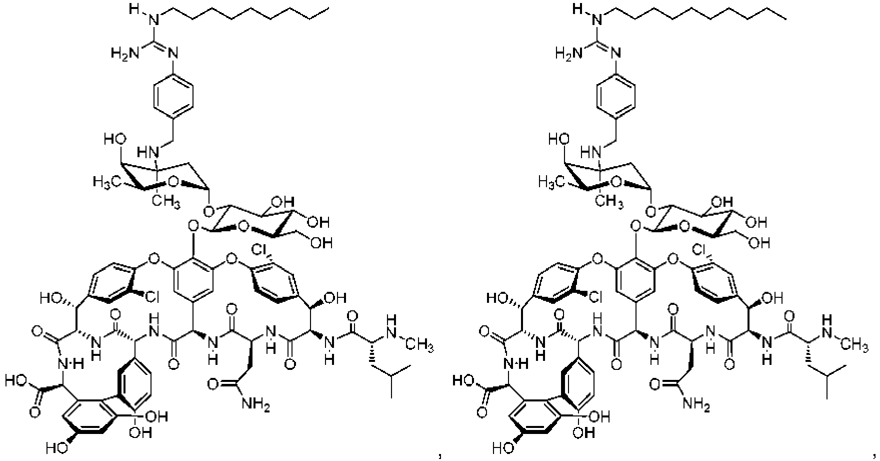 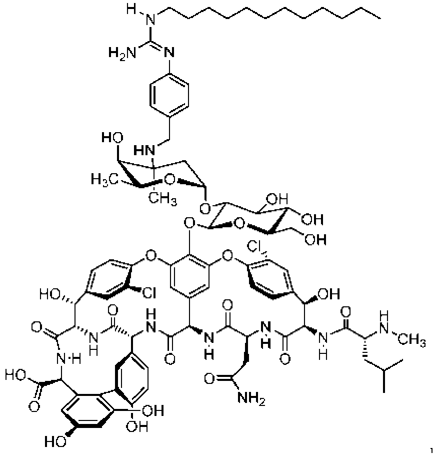 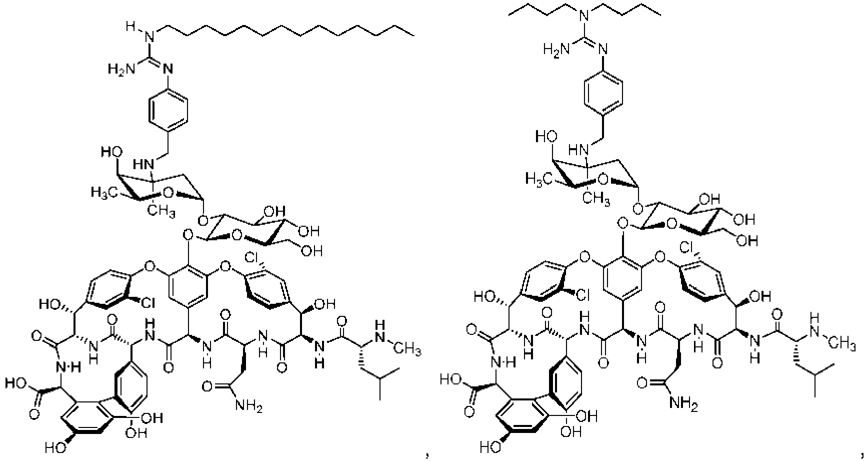 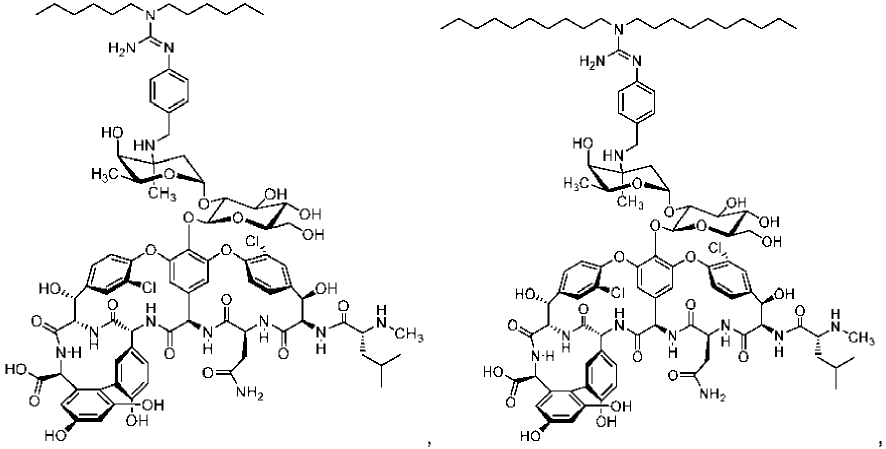 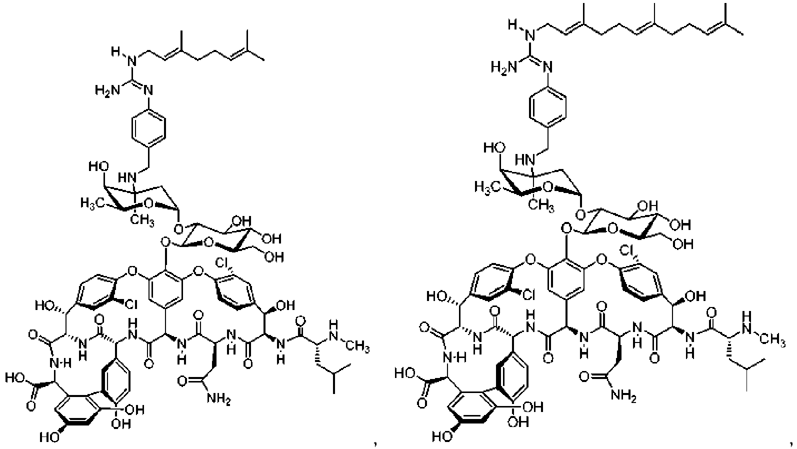 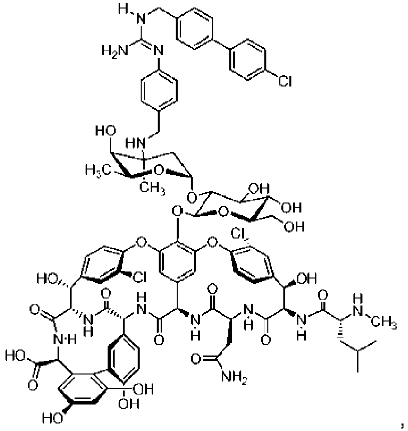 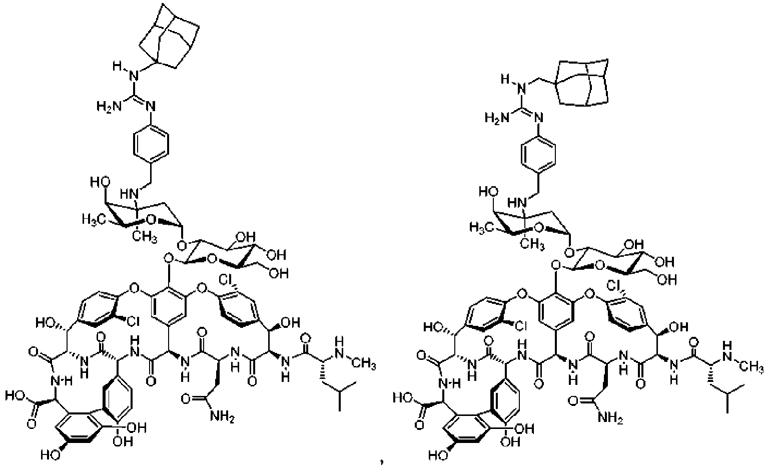 oder pharmazeutisch verträgliches Salz oder Solvat davon.

11. Formulierung, umfassend die Verbindung nach einem vorhergehenden Anspruch und optional einen pharmazeutisch verträglichen Träger.

12. Formulierung nach Anspruch 11, wobei die Formulierung eine parenterale Formulierung oder eine orale Formulierung ist,
wobei optional die Formulierung eine parenterale Formulierung ist, ferner optional wenn die Formulierung eine Formulierung zur intravenösen Injektion ist.

13. Verbindung nach einem der Ansprüche 1 bis 10 oder Formulierung nach Anspruch 11 oder Anspruch 12 zur Verwendung als Medikament.

14. Verbindung nach einem der Ansprüche 1 bis 10 oder Formulierung nach Anspruch 11 oder Anspruch 12 zur Verwendung bei der Behandlung einer bakteriellen Infektion.

15. Verbindung oder Formulierung zur Verwendung nach Anspruch 14, wobei die bakterielle Infektion eine Infektion durch grampositive Bakterien ist.

## Revendications

1. Composé de formule 1 : 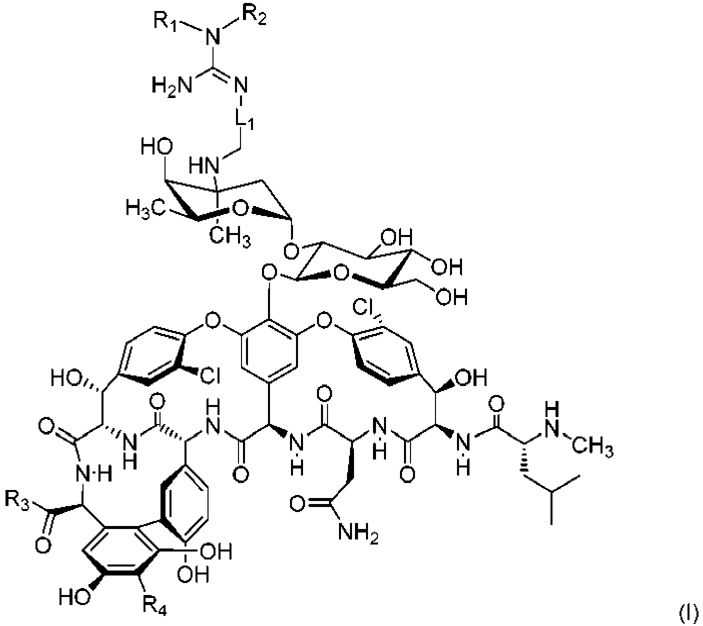 dans laquelle :
R₁ représente un lipide ;
R₂ est choisi parmi un groupe -H ou un lipide ;
R₃ est choisi parmi un groupe -OH, -C₁-C₂₀ alkyle substitué ou non substitué, -C₂-C₂₀ alcényle substitué ou non substitué, -C₁-C₄ alkylaryle substitué ou non substitué et glucide choisi parmi les groupes -(NHCH₂CH₂)ₓ-Glc, -(NHCH₂CH₂)ₓ-Gal, -(NHCH₂CH₂)ₓ-Man, - (NHCH₂CH₂)ₓ-GlcNAc, -(NHCH₂CH₂)ₓ-MurNAc, -(NHCH₂CH₂)ₓ-ManNAc, -(NHCH₂CH₂)ₓ-GalNAc, -(NHCH₂CH₂)ₓ-cellbiose et -(NHCH₂CH₂)ₓ-maltose, où x vaut 0 ou 1 ;
R₄ est choisi parmi un groupe -H, -C₁-C₂₀ alkyle substitué ou non substitué, -C₂-C₂₀ alcényle substitué ou non substitué, -C₁-C₄ alkylaryle substitué ou non substitué et glucide choisi parmi -(NHCH₂CH₂)_{y}-Glc, -(NHCH₂CH₂)_{y}-Gal, -(NHCH₂CH₂)_{y}-Man, -(NHCH₂CH₂)_{y}-GlcNAc, - (NHCH₂CH₂)_{y}-MurNAc, -(NHCH₂CH₂)_{y}-ManNac, -(NHCH₂CH₂)_{y}-GalNAc, -(NHCH₂CH₂)_{y}-cellbiose et -(NHCH₂CH₂)_{y}-maltose, où y vaut 0 ou 1 ; et
L₁ représente un lieur choisi parmi les groupes -C₁-C₂₀ alkylène-, -C₂-C₂₀ alcénylène-, - (C₁-C₄ alkyl)arylène-, -C₂-C₄ alkyl-[O(CH₂)ᵣ]ₛ-O(CH₂)ᵣ-, -C₂-C₁₂ alkyl-S-S-C₁-C₁₂ alkyle-, - C(O)C₁-C₂₀ alkylène-, -C(O)C₂-C₂₀ alcénylène-, -C(O)(C₁-C₄ alkyl)arylène-, -C(O)C₁-C₄ alkyl-[O(CH₂)ᵣ]ₛ-O(CH₂)ᵣ, - C(O)NHC₁-C₂₀ alkylène-, -C(O)NHC₂-C₂₀ alcénylène-, -C(O)NH(C₁-C₄ alkyl)arylène-, -C(O)NHC₁-C₄ alkyl-[O(CH₂)ᵣ]ₛ-O(CH₂)ᵣ-, -C(S)NHC₁-C₂₀ alkylène-, - C(S)NHC₂-C₂₀ alcénylène-, -C(S)NH(C₁-C₄ alkyl)arylène- et -C(S)NHC₁-C₄ alkyl-[O(CH₂)ᵣ]ₛ-O(CH₂)ᵣ-, où r est choisi parmi 2 et 3 et s est choisi parmi 0 à 20,
ou sel, stéréoisomère ou solvate pharmaceutiquement acceptable de celui-ci,
chaque lipide étant un groupement hydrophobe comprenant des groupes alkyle, alcényle, cycloalkyle, cycloalkyle pontés, (alkyl)cycloalkyle, (alkyl) cycloalkyle pontés, (alkyl)cycloalcényle et/ou alkylaryle substitués ou non substitués,
lorsqu'ils sont substitués, tout groupe parmi les groupes précédents est substitué par un ou plusieurs substituants choisis parmi : les groupes -OH, -CN, -NH₂, -NH(C₁-C₆ alkyle), -N(C₁-C₄ alkyle)₂, =O, -halogéno, -C₁-C₆ alkyle, -C₂-C₆ alcényle, -C₁-C₆ halogénoalkyle, -C₁-C₆ halogénoalcoxy et -C₂-C₆ halgénoalcényle et -C₁-C₆ acide alkylcarboxylique.

2. Composé selon la revendication 1, R₁ étant choisi parmi les groupes -C₄-C₂₀ alkyle, -C₄-C₂₀ alcényle, -C₄-C₂₀ cycloalkyle, C₁-C₄ alkyl-C₄-C₂₀ cycloalkyle, -C₁-C₄ alkylaryle substitué ou non substitué, -C₁-C₄ alkyl-[O(CH₂)ₘ]ₙ-O(CH₂)ₘ₋₁CH₃, où m est choisi parmi 2 et 3 et n est choisi parmi 0 à 20, -C₂-C₁₂ alkyl-S-S-C₁-C₁₂ alkyle,
éventuellement R₁ étant choisi parmi les groupes -C₄-C₂₀ alkyle, -C₄-C₂₀ alcényle, -C₁-C₄ alkylaryle substitué ou non substitué, -C₁-C₄ alkyl-[O(CH₂)ₘ]ₙ-O(CH₂)ₘ₋₁CH₃, où m est choisi parmi 2 et 3 et n est choisi parmi 0 à 20, -C₂-C₁₂ alkyl-S-S-C₁-C₁₂ alkyle.

3. Composé selon l'une quelconque des revendications précédentes, R₁ étant choisi parmi les groupes -C₆-C₁₄ alkyle, -C₆-C₁₆ alcényle et -C₁-C₄ alkylbisphényle substitué ou non substitué.

4. Composé selon l'une quelconque des revendications précédentes, R₁ étant choisi parmi les groupes -C₆-C₁₄ alkyle, -C₁-C₄ adamantyle, -adamantyle, -géranyle, -farnésyle et - chlorobisphényle ;
éventuellement R₁ étant choisi parmi les groupes -C₆-C₁₂ alkyle, -géranyle, -farnésyle et - chlorobisphényle, ou
éventuellement R₁ étant choisi parmi 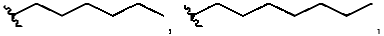 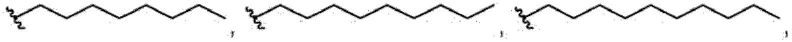 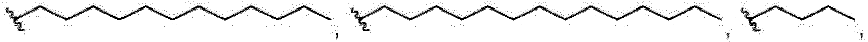 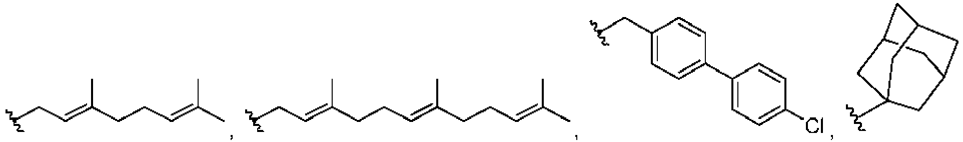 ou

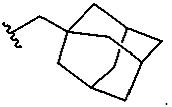

5. Composé selon l'une quelconque des revendications précédentes, R₂ étant choisi parmi les groupes -H, -C₄-C₂₀ alkyle, -C₄-C₂₀ alcényle, -C₁-C₄ alkylaryle substitué ou non substitué, -C₁-C₄ alkyl-[O(CH₂)ₚ]_{q}-O(CH₂)ₚ₋₁CH₃, où p est choisi parmi 2 et 3 et q est choisi parmi 0 à 20 ; et -C₂-C₁₂ alkyl-S-S-C₁-C₁₂ alkyle,
éventuellement R₂ étant choisi parmi les groupes -H, -C₄-C₁₂ alkyle, -C₄-C₁₂ alcényle, -C₁-C₄ alkylaryle substitué ou non substitué, et -C₁-C₄ alkyl-[O(CH₂)ₚ]_{q}-O(CH₂)ₚ₋₁CH₃, où p est choisi parmi 2 et 3 et q est choisi parmi 0 à 20 ; éventuellement en outre R₂ étant choisi parmi les groupes -H, -C₄-C₁₂ alkyle, -C₄-C₁₂ alcényle ;
éventuellement en outre encore R₂ représentant un groupe -H.

6. Composé selon la revendication 1, chacun des groupes R₁ et R₂ étant indépendamment choisi parmi les groupes -C₂-C₁₀ alkyle, -C₂-C₁₀ alcényle et -C₁-C₄ alkyl-[O(CH₂)ₘ]ₙ-O(CH₂)ₘ₋₁CH₃, où m est choisi parmi 2 et 3 et n est choisi parmi 0 à 20,
éventuellement chacun des groupes R₁ et R₂ étant indépendamment choisi parmi les groupes -C₂-C₁₀ alkyle et -C₂-C₁₀ alcényle.

7. Composé selon l'une quelconque des revendications précédentes, R₃ étant choisi parmi les groupes -OH, -C₁-C₁₀ alkyle substitué ou non substitué, -C₂-C₁₀ alcényle substitué ou non substitué, -C₁-C₄ alkylaryle substitué ou non substitué et glucide choisi parmi les groupes - (NHCH₂CH₂)_{y}-Glc, -(NHCH₂CH₂)_{y}-Gal, -(NHCH₂CH₂)_{y}-Man, -(NHCH₂CH₂)_{y}-GlcNAc, - (NHCH₂CH₂)_{y}-MurNAc, -(NHCH₂CH₂)_{y}-ManNac, -(NHCH₂CH₂)_{y}-GalNAc, -(NHCH₂CH₂)_{y}-cellbiose et -(NHCH₂CH₂)_{y}-maltose, où y vaut 0 ou 1,
éventuellement R₃ étant choisi parmi les groupes -OH, -NHCH₂CH₂CH₂N(CH₃)₂, 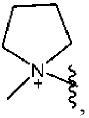 -(NHCH₂CH₂)ₓ-Glc, -(NHCH₂CH₂)ₓ-Gal, -(NHCH₂CH₂)ₓ-Man, -(NHCH₂CH₂)ₓ-GlcNAc, - (NHCH₂CH₂)ₓ-MurNAc, -(NHCH₂CH₂)ₓ-ManNAc, -(NHCH₂CH₂)ₓ-GalNAc, -(NHCH₂CH₂)ₓ-cellbiose et -(NHCH₂CH₂)ₓ-maltose, où x vaut 0 ou 1,
éventuellement en outre R₃ représentant un groupe -OH.

8. Composé selon l'une quelconque des revendications précédentes, R₄ étant choisi parmi les groupes -H, -C₁-C₁₀ alkyle substitué ou non substitué, -C₂-C₁₀ alcényle substitué ou non substitué, -C₁-C₄ alkylaryle substitué ou non substitué et glucide choisi parmi les groupes -(NHCH₂CH₂)_{y}-Glc, -(NHCH₂CH₂)_{y}-Gal, -(NHCH₂CH₂)_{y}-Man, -(NHCH₂CH₂)_{y}-GlcNAc, -(NHCH₂CH₂)_{y}-MurNAc, - (NHCH₂CH₂)_{y}-ManNac, -(NHCH₂CH₂)_{y}-GalNAc, -(NHCH₂CH₂)_{y}-cellbiose et - (NHCH₂CH₂)_{y}-maltose, où y vaut 0 ou 1,
éventuellement R₄ étant choisi parmi les groupes -H, -CH₂NHCH₂P(O)(OH)₂, - CH₂N(CH₂PO₃H₂)₂, -CH₂NHCH₂CH₂CH₂N(CH₃)₂, -CH₂NHCH₂CH₂COOH, - CH₂N(CH₃)CH₂(CH(OH))₄CH₂OH, -CH₂NHCH(COOH)CH₂COOH, -CH₂NH(CH₂CH₂OH)₂, - (NHCH₂CH₂)_{y}-Glc, -(NHCH₂CH₂)_{y}-Gal, -(NHCH₂CH₂)_{y}-Man, -(NHCH₂CH₂)_{y}-GlcNAc, - (NHCH₂CH₂)_{y}-MurNAc, -(NHCH₂CH₂)_{y}-ManNAc, -(NHCH₂CH₂)_{y}-GalNAc, -(NHCH₂CH₂)_{y}-cellbiose et -(NHCH₂CH₂)_{y}-maltose, où y vaut 0 ou 1,
éventuellement en outre R₄ représentant un groupe -H.

9. Composé selon l'une quelconque des revendications précédentes, L₁ étant choisi parmi les groupes -C₂-C₂₀ alkylène-, -C₂-C₂₀ alcénylène-, -(C₁-C₄ alkyl)arylène-, -C₂-C₄ alkyl-[O(CH₂)ᵣ]ₛ-O(CH₂)ᵣ-, -C₂-C₁₂ alkyl-S-S-C₁-C₁₂ alkyle-, -C(O)C₁-C₂₀ alkylène-, -C(O)C₂-C₂₀ alcénylène-, - C(O)(C₁-C₄ alkyl)arylène-, -C(O)C₁-C₄ alkyl-[O(CH₂)ᵣ]ₛ-O(CH₂)ᵣ-, -C(O)NHC₁-C₂₀ alkylène-, - C(O)NHC₂-C₂₀ alcénylène-, -C(O)NH(C₁-C₄ alkyl)arylène-, -C(O)NHC₁-C₄ alkyl-[O(CH₂)ᵣ]ₛ-O(CH₂)ᵣ, -C(S)NHC₁-C₂₀ alkylène-, -C(S)NHC₂-C₂₀ alcénylène-, -C(S)NH(C₁-C₄ alkyl)arylène-et -C(S)NHC₁-C₄ alkyl-[O(CH₂)ᵣ]ₛ-O(CH₂)ᵣ, où r est choisi parmi 2 et 3 et s est choisi parmi 0 à 20,
éventuellement L₁ étant choisi parmi les groupes -C₂-C₁₀ alkylène- et -(C₁-C₄ alkyl)arylène-,
éventuellement en outre L₁ représentant un groupe -CH₂Ph-,

10. Composé selon l'une quelconque des revendications précédentes, choisi parmi : 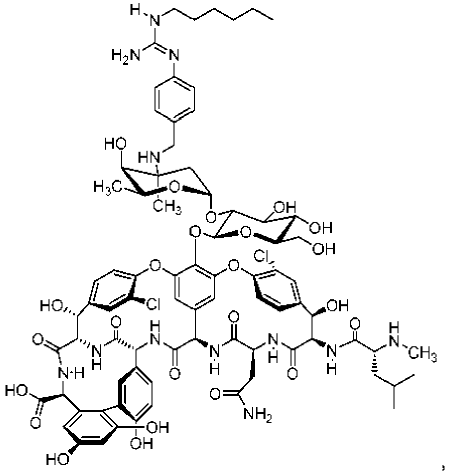 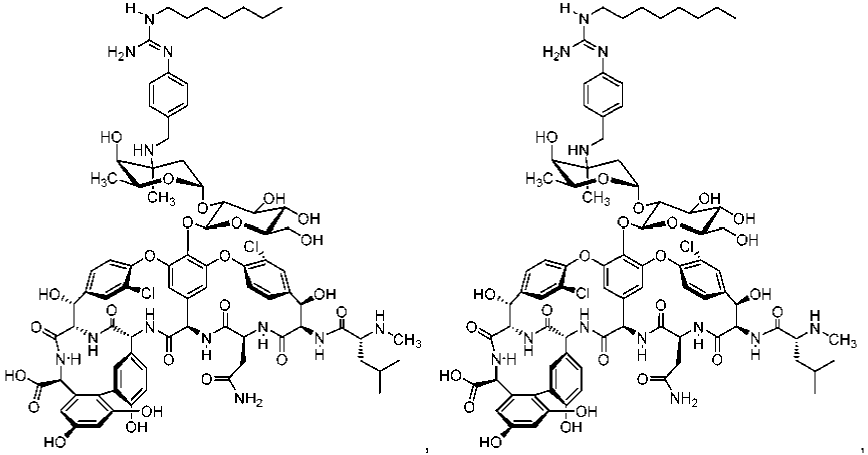 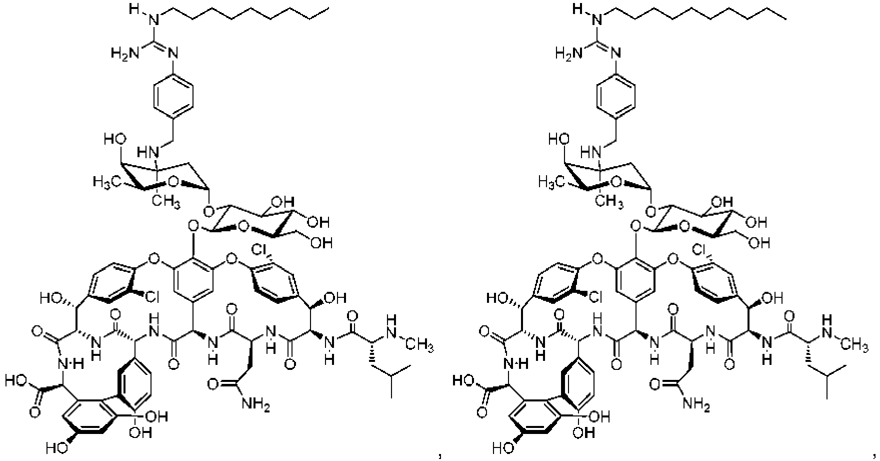 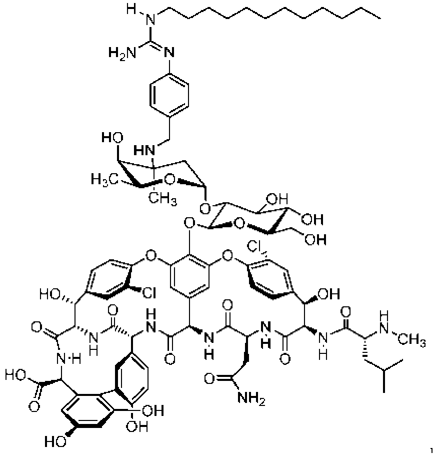 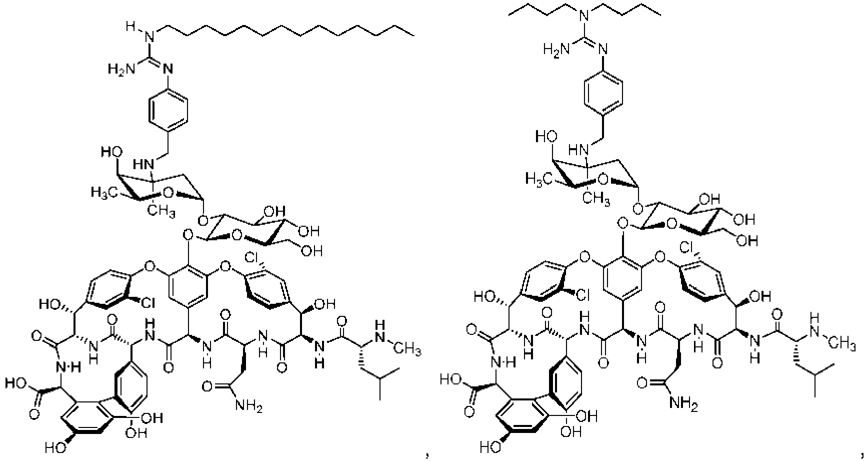 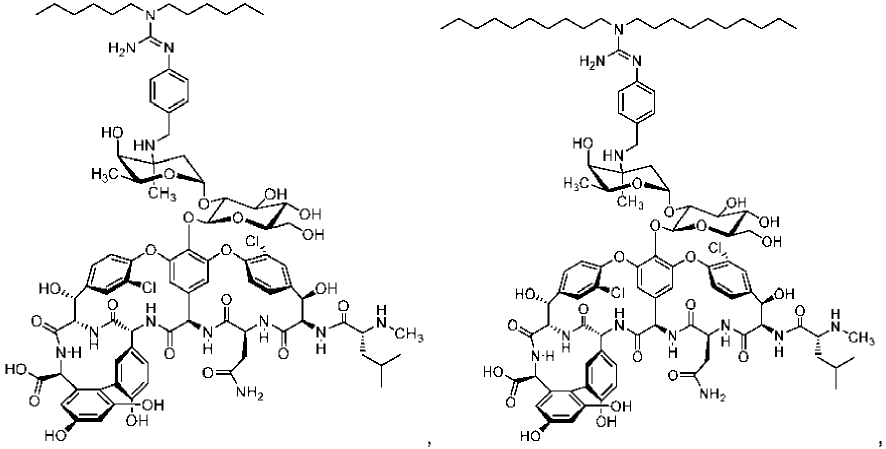 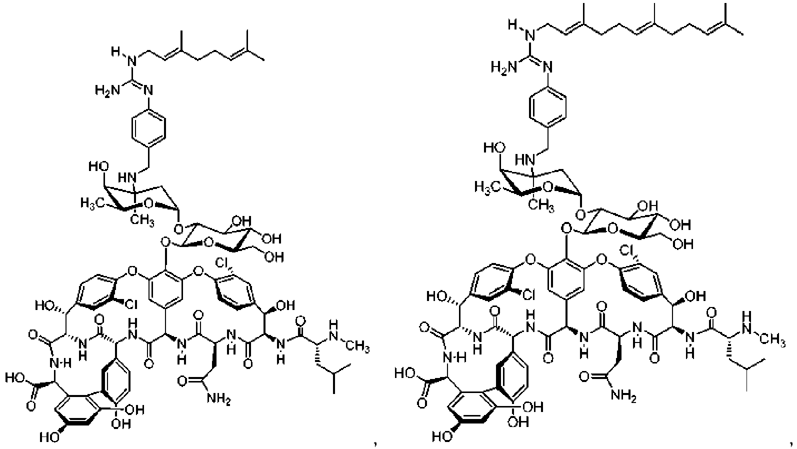 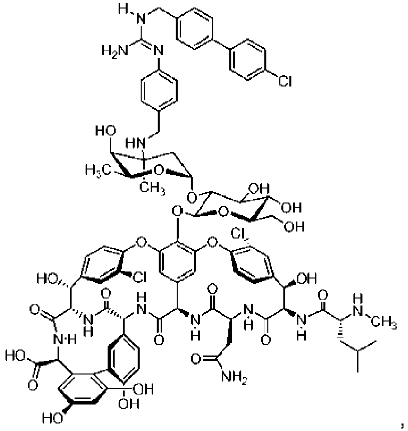 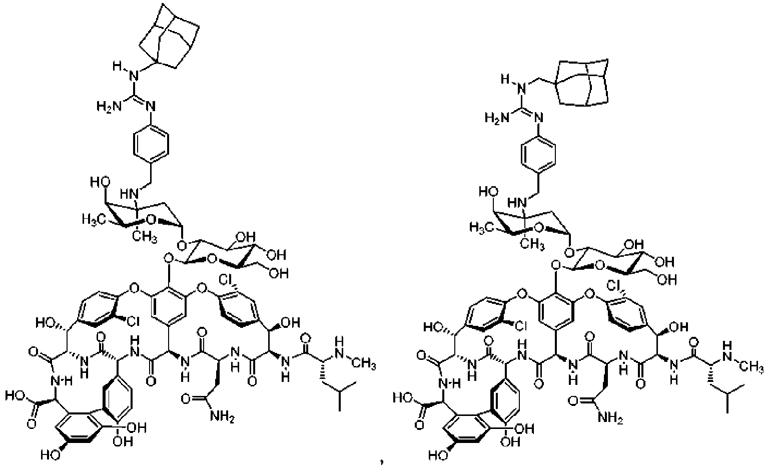 ou sel ou solvate pharmaceutiquement acceptable de celui-ci.

11. Formulation comprenant le composé selon l'une quelconque des revendications précédentes et éventuellement un vecteur pharmaceutiquement acceptable.

12. Formulation selon la revendication 11, ladite formulation étant une formulation parentérale ou une formulation orale,
éventuellement ladite formulation étant une formulation parentérale, éventuellement en outre lorsque la formulation est une formulation pour injection intraveineuse.

13. Composé selon l'une quelconque des revendications 1 à 10, ou formulation selon la revendication 11 ou la revendication 12, destiné à être utilisé comme médicament.

14. Composé selon l'une quelconque des revendications 1 à 10, ou formulation selon la revendication 11 ou la revendication 12, destiné à être utilisé dans le traitement d'une infection bactérienne.

15. Composé ou formulation destiné à être utilisé selon la revendication 14, ladite infection bactérienne étant une infection par bactérie à Gram positif.
